(19) Europäisches Patentamt
European Patent Office
Office européen des brevets

(11) **EP 4 729 943 A2**

(12) **EUROPEAN PATENT APPLICATION**

(43) Date of publication:
22.04.2026   Bulletin 2026/17

(21) Application number: 25216677.2

(22) Date of filing: 02.10.2017

(51) International Patent Classification (IPC):
*G01N 33/573* (2006.01)

(52) Cooperative Patent Classification (CPC):
**G01N 33/573;** G01N 2333/916; G01N 2333/948;
G01N 2333/978; G01N 2800/2871

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 03.10.2016   US 201662403293 P
06.02.2017   US 201762455269 P

(62) Document number(s) of the earlier application(s) in
accordance with Art. 76 EPC:
**17791224.3 / 3 519 825**

(71) Applicant: **Abbott Laboratories**
**Abbott Park, IL 60064 (US)**

(72) Inventors:
• **DATWYLER, Saul**
**Abbott Park, IL 60064 (US)**
• **MCQUISTON, Beth**
**Abbott Park, IL 60064 (US)**

• **BRATE, Elaine**
**Abbott Park, IL 60064 (US)**
• **RAMP, John**
**Abbott Park, IL 60064 (US)**
• **PACENTI, David**
**Abbott Park, IL 60064 (US)**

(74) Representative: **Carpmaels & Ransford LLP**
**One Southampton Row**
**London WC1B 5HA (GB)**

Remarks:
•This application was filed on 18.11.2025 as a
divisional application to the application mentioned
under INID code 62.
•Claims filed after the date of filing of the application
/after the date of receipt of the divisional application
(Rule 68(4) EPC).

(54) **IMPROVED METHODS OF ASSESSING GFAP STATUS IN PATIENT SAMPLES**

(57)   Disclosed herein are improved methods of assessing Glial fibrillary acidic protein (GFAP) status in a subject (such as for examples, as a measure of traumatic brain injury or for other clinical reasons).

FIG. 1

EP 4 729 943 A2

**Description**

**CROSS REFERENCE TO RELATED APPLICATIONS**

This application claims priority to U.S. Provisional Application No. 62/403,293, filed October 3, 2016, and U.S. Provisional Application No. 62/455,269, filed February 6, 2017, all of which are incorporated herein by reference in their entirety.

**INCORPORATION-BY-REFERENCE OF MATERIAL SUBMITTED ELECTRONICALLY**

The instant application contains a Sequence Listing which has been submitted in ASCII format via EFS-Web and is hereby incorporated by reference in its entirety. Said ASCII copy, created on October 2, 2017, is named 2017_10_02_12850WOO1-SEQ-LIST.txt, and is 4,317 bytes in size.

**TECHNICAL FIELD**

The present disclosure relates to improved methods of assessing Glial fibrillary acidic protein (GFAP) status, e.g., as a measure of traumatic brain injury or for other clinical reasons.

**BACKGROUND**

There are many scenarios in which trauma-induced brain, spinal cord, and other neurologic injuries are observed. For example, military field care providers reported severe pain experienced by casualties with spine and head injuries when subjected to bumpy and high vibration ground and air transport. Repeated shock and vibration experienced by patients during medical transport may affect medical outcomes. Casualties with spinal cord injury (SCI), traumatic brain injury (TBI), and/or other severe neurologic injuries are the most vulnerable to vehicle repeated shock and vibration. Fluid markers of neuronal, axonal and astroglial damage would be valuable to aid in the diagnosis of concussion in patients as well as assess their need for imaging with head trauma, to predict short- and long-term clinical outcome and to tell when the brain has recovered from the TBI. Current biomarker candidates are limited by being insufficient in their sensitivity in serum detection, specificity to point to the brain, and lack of assay standardization. There is a lack of an acute marker for a field assay to evaluate the spectrum of injury of TBI from hyperacute to acute. Furthermore, there is currently no way of identifying mild TBI (mTBI) with lasting brain damage after a concussion that can cause post-traumatic stress disorder (PTSD) or chronic neurodegeneration (Chronic traumatic encephalophathy, CTE, "punch drunk").

Mild TBI or concussion is much harder to objectively detect and presents an everyday challenge in emergency care units, in the military field, emergency rooms, inpatient hospitals, and outpatient clinics, sports fields and arenas, globally. Concussion usually causes no gross pathology, such as hemorrhage, and no abnormalities on conventional computed tomography scans of the brain, but rather rapid-onset neuronal dysfunction that resolves in a spontaneous manner over a few days to a few weeks. Approximately 15% of mild TBI patients suffer persisting cognitive dysfunction.

There is an unmet need for tools for assessing mild TBI victims on scene, in emergency rooms, inpatient hospitals, and clinics, in the sports area and in military activity (e.g., combat).

**SUMMARY**

The present disclosure is directed to a method of assessing a subject's glial fibrillary acid protein (GFAP) status. The present disclosure is also directed to a method of assessing a subject's glial fibrillary acid protein (GFAP) status as a measure of traumatic brain injury wherein said subject is known to have sustained an injury to the head. The method includes the steps of:

a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein either the first or second specific binding member comprises a detectable label; and

b) assessing a signal from the one or more first complexes, wherein the amount of detectable signal from the detectable label indicates the amount of GFAP present in the sample, such that the amount of detectable signal from the detectable label can be employed to assess said subject's GFAP status as a measure of traumatic brain injury,

wherein the method (i) can be used to determine levels of up to 50,000 pg/mL of GFAP, (ii) does not require dilution of the biological sample, and (iii) is conducted using a point-of-care device.

The present disclosure is directed to a method of measuring GFAP in a biological sample from a subject that may have sustained an injury to the head. The present disclosure is also directed to a method of measuring GFAP in a biological sample from a subject that is known to have sustained an injury to the head. The method comprises (a) obtaining a biological sample from said subject, (b) contacting the biological sample with, either simultaneously or sequentially, in any order:

(1) a capture antibody, which binds to13 an epitope on GFAP or GFAP fragment to form a capture antibody-GFAP antigen complex, and (2) a detection antibody which includes a detectable label and binds to an epitope on GFAP that is not bound by the capture antibody, to form a GFAP antigen-detection antibody complex, such that a capture antibody-GFAP antigen-detection antibody complex is formed, and

(c) determining the amount or concentration of GFAP in the biological sample based on the signal generated by the detectable label in the capture antibody-GFAP antigen-detection antibody complex, wherein the method can be used to determine levels of GFAP in an amount of less than or equal to 50,000 pg/mL, and wherein said method has a dynamic range of 5 log, and is linear over said dynamic range.

The present disclosure is directed to a method of assessing a subject's glial fibrillary acid protein (GFAP) status as a measure of traumatic brain injury wherein said subject may have sustained an injury to the head. The present disclosure is also directed to a method of assessing a subject's glial fibrillary acid protein (GFAP) status as a measure of traumatic brain injury wherein said subject is known to have sustained an injury to the head. The method comprises the steps of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

The present disclosure is directed to a method of assessing glial fibrillary acid protein (GFAP) status as a measure of traumatic brain injury in a subject that may have sustained an injury to the head. The present disclosure is also directed to a method of assessing glial fibrillary acid protein (GFAP) status as a measure of traumatic brain injury in a subject that is known to have sustained an injury to the head. The method comprises the steps of:

a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; and

b) assessing a signal from the one or more first complexes, wherein the presence of a detectable signal from the detectable label indicates that GFAP is present in the sample, and the presence of detectable signal from the detectable label can be employed to assess said subject's GFAP status as a measure of traumatic brain injury,

wherein the method can be used to determine levels of GFAP in an amount of less than or equal to 50,000 pg/mL, and wherein said method has a dynamic range of 5 log, and is linear over said dynamic range.

The present disclosure is directed to a method of measuring glial fibrillary acid protein (GFAP) status as a measure of traumatic brain injury in a subject that may have sustained an injury to the head. The present disclosure is also directed to a method of measuring glial fibrillary acid protein (GFAP) status as a measure of traumatic brain injury in a subject that is known to have sustained an injury to the head. The method comprises the steps of:

a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label;

b) detecting a signal from the one or more first complexes, wherein the presence of a detectable signal from the detectable label indicates that GFAP is present in the sample, and

c) measuring the amount of detectable signal from the detectable label indicates the amount of GFAP present in the sample, such that the amount of detectable signal from the detectable label can be employed to assess said subject's GFAP status as a measure of traumatic brain injury,

wherein said assay is capable of determining an amount of GFAP less than or equal to 50,000 pg/mL in a volume of less than 20 microliters of test sample, wherein said assay has a dynamic range of 5 log, and is linear over said dynamic range.

Each of the above described methods can provide an expanded window of detection. The expanded window of detection is a broad window of detection, such as a window of detection that is broader than state of the art assays. Specifically, the above described methods can be carried out on any subject without regard to the subject's clinical condition, laboratory values, clinical condition and laboratory values, classification as suffering from mild, moderate or severe TBI, exhibition of low or high levels of GFAP, and/or without regard to the timing of any event wherein a subject may have sustained an injury to the head. The above described methods can also be carried out on any subject without regard to the subject's clinical condition, laboratory values, clinical condition and laboratory values, classification as suffering from mild, moderate or severe TBI, exhibition of low or high levels of GFAP, and/or without regard to the timing of any event wherein a subject is known to have sustained an injury to the head. In addition to, or alternatively, the above methods can have a lower end limit of detection (LoD) of about 10 pg/mL.

Additionally, each of the above methods can be done using a volume of less than 20 microliters of said biological sample.

Moreover, each of the above methods can be used to determine levels of GFAP across a range selected from the group consisting of from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL.

Additionally, in the above described methods, either the first specific binding member or second specific binder member, whichever does not comprise the detectable label, can be immobilized on a solid support.

Also, in the above described methods, GFAP can be assessed along with one or more other biomarker.

Also, in the above described methods, the biological sample does not require dilution. For example, in the above described methods, the biological sample can be selected from the group consisting of a whole blood sample, a serum sample, a cerebrospinal fluid sample and a plasma sample. In the above described methods, the biological sample is from about 1 to about 25 microliters.

Furthermore, in the above described methods, the method can be performed in from about 5 to about 20 minutes. Alternatively, the method is performed in about 15 minutes. Alternatively, the method is performed in less than about 30 minutes, such as in less than about 25 minutes, in less than about 20 minutes, or in less than about 15 minutes.

Additionally, in the above described methods, the time between when the biological sample is obtained and when the subject may have sustained an injury to the head may not be known. Alternatively, the time between when the biological sample is obtained and when the subject may have sustained an injury to the head may be selected from the group consisting of from zero to about 12 hours, from about 12 to about 24 hours, from about 24 to about 36 hours, from about 36 to about 48 hours, from about 48 to about 72 hours, from about 72 to about 96 hours, from about 96 to about 120 hours, from about 120 hours to about 7 days, from about 7 days to about 1 month, from about 1 month to about 3 months, from about 3 months to about 6 months, from about 6 months to about 1 year, from about 1 year to about 3 years, from about 3 years to about 6 years, from about 6 years to about 12 years, from about 12 years to about 20 years, from about 20 years to about 30 years, and from about 30 years to about 50 years. Alternatively, the time between when the biological sample is obtained and when the subject may have sustained an injury to the head may be selected from the group consisting of less than 50 years, less than 30 years, less than 20 years, less than 12 years, less than 6 years, less than 3 years, less than 1 year, less than about 6 months, less than about 3 months, less than about 1 month, less than about 7 days, less than about 120 hours, less than about 96 hours, less than about 72 hours, less than about 48 hours, less than about 36 hours, less than about 24 hours, or less than about 12 hours.

In the above methods, the biological sample can be obtained after the subject may have sustained an injury to the head caused by physical shaking, blunt impact by an external mechanical or other force that results in a closed or open head trauma, one or more falls, explosions or blasts or other types of blunt force trauma.

In the above methods, the biological sample can be obtained after the subject has ingested or been exposed to a chemical, toxin or combination of a chemical and toxin.

In the above methods, the chemical or toxin can be fire, mold, asbestos, a pesticide, an insecticide, an organic solvent, a paint, a glue, a gas, an organic metal, a drug of abuse or one or more combinations thereof.

In the above methods, the biological sample can be obtained from a subject that suffers from a disease, such as an autoimmune disease, a metabolic disorder, a brain tumor, hypoxia, a virus, meningitis, hydrocephalus or combinations thereof. In the above methods, the disease can also be vascular injury.

In the above methods, the method can be done to confirm the occurrence of traumatic brain injury or the absence of traumatic brain injury. For example, the method can be used to aid in the diagnosis of, determine the risk, confirm, evaluate, and/or prognose traumatic brain injury or the absence of traumatic brain injury in a subject. In the above methods, the method can be used to evaluate head injury and/or concussion, to predict a need for imaging, to predict severity of the traumatic brain injury, such as mild TBI, and to prognosticate traumatic brain injury.

In the above methods, the traumatic brain injury can be mild traumatic brain injury. In the above methods, the contacting can be done simultaneously. Alternatively, the contacting can be done sequentially.

In the above methods, the status can be assessed by measuring the level or amount of GFAP at a single point in time. Alternatively, in the above methods, the GFAP status of the subject can be assessed by measuring the level or amount of GFAP at multiple time points.

In any of the above methods, the first specific binding member and the second specific binding member bind human GFAP.

In any of the above methods, the first specific binding member and the second specific binding member may be an antibody or antibody fragment. For example, in any of the above methods the first specific binding member and the second specific binding member may each be a monospecific antibody, such as a monoclonal antibody that binds human GFAP.

The present disclosure is directed to a method of assessing a subject's glial fibrillary acid protein (GFAP) status. The method comprises the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein either the first or second specific binding member comprises a detectable label; and b) assessing a signal from the one or more first complexes, wherein the amount of detectable signal from the detectable label indicates the amount of GFAP present in the sample, wherein the method (i) can be used to determine levels of up to 50,000 pg/mL of GFAP, (ii) does not require dilution of the biological sample, and (iii) is conducted using a point-of-care device.

The present disclosure is directed to a method of measuring GFAP in a biological sample from a subject. The method comprises (a) obtaining a biological sample from said subject, (b) contacting the biological sample with, either simultaneously or sequentially, in any order: (1) a capture antibody, which binds to an epitope on GFAP or GFAP fragment to form a capture antibody-GFAP antigen complex, and (2) a detection antibody which includes a detectable label and binds to an epitope on GFAP that is not bound by the capture antibody, to form a GFAP antigen-detection antibody complex, such that a capture antibody-GFAP antigen-detection antibody complex is formed, and (c) determining the amount or concentration of GFAP in the biological sample based on the signal generated by the detectable label in the capture antibody-GFAP antigen-detection antibody complex, wherein the method can be used to determine levels of GFAP in an amount of less than or equal to 50,000 pg/mL, and wherein said method has a dynamic range of 5 log, and is linear over said dynamic range.

The present disclosure is directed to a method of assessing a subject's glial fibrillary acid protein (GFAP) status. The method comprises the step of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

The present disclosure is directed to a method of assessing glial fibrillary acid protein (GFAP) status. The method comprises the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; and b) assessing a signal from the one or more first complexes, wherein the presence of a detectable signal from the detectable label indicates that GFAP is present in the sample, wherein the method can be used to determine levels of GFAP in an amount of less than or equal to 50,000 pg/mL, and wherein said method has a dynamic range of 5 log, and is linear over said dynamic range.

The present disclosure is directed to a method of measuring glial fibrillary acid protein (GFAP) status. The method comprises the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, wherein said assay is capable of determining an amount of GFAP less than or equal to 50,000 pg/mL in a volume of less than 20 microliters of test sample, wherein said assay has a dynamic range of 5 log, and is linear over said dynamic range.

The present disclosure is directed to a method of assessing a subject's glial fibrillary acid protein (GFAP) status as a measure of traumatic brain injury in a biological sample obtained from a human subject, wherein said subject may have sustained an injury to the head or is known to have sustained an injury to the head, the method comprising the steps of: (a) contacting a biological sample obtained from a human subject, either simultaneously or sequentially, in any order, with: (1) a capture antibody which is immobilized on a solid support and which binds to an epitope on human GFAP to form a capture antibody-GFAP antigen complex, and (2) a detection antibody which includes a detectable label and which binds to an epitope on human GFAP that is not bound by the capture antibody, to form a GFAP antigen-detection antibody complex, such that a capture antibody-GFAP antigen-detection antibody complex is formed, wherein the capture antibody and detection antibody are monoclonal antibodies, (b) determining the level of GFAP in the biological sample based on the signal generated by the detectable label in the capture antibody-GFAP antigen-detection antibody complex, wherein the method is capable of quantitating the level of GFAP across a dynamic range from 5 pg/mL to 50,000 pg/mL with a precision of ≤10 % CV and with less than 10% deviation from linearity (DL) over the dynamic range.

The present disclosure is directed to a method of measuring glial fibrillary acid protein (GFAP) status as a measure of traumatic brain injury in a subject that may have sustained an injury to the head or is known to have sustained an injury to the head, the method comprising the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label, wherein the first specific binding member is immobilized on a solid support; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, such that the amount of detectable signal from the detectable label can be employed to assess said subject's GFAP status as a measure of traumatic brain injury, wherein said assay is capable of determining the level of GFAP across a dynamic range from 5 pg/mL to 50,000 pg/mL, such as from about 10 pg/mL to about 50,000 pg/mL or from about 20 pg/mL to about 50,000 pg/mL, with a precision of ≤10 % CV and with less than 10% deviation from linearity (DL) is achieved over the dynamic range using a volume of less than 20 microliters of said biological sample.

The present disclosure is directed to a method of assessing a subject's glial fibrillary acid protein (GFAP) status. The method includes the steps of: a) contacting a biological sample from said subject, either simultaneously or sequentially, in any order, with at least one first specific binding member and at least one second specific binding member, wherein the first specific binding member and the second specific binding member each specifically bind to GFAP thereby producing one or more first complexes comprising the at least one first specific binding member-GFAP-at least one second specific binding member, wherein either at least one of the first specific binding member or the at least one second specific binding member comprise a detectable label; and b) assessing a signal from the one or more first complexes, wherein the amount of detectable signal from the detectable label indicate the amount of GFAP present in the sample, wherein the method (i) can be used to determine levels of up to 50,000 pg/mL of GFAP, (ii) does not require dilution of the biological sample, and (iii) is conducted using a point-of-care device.

The present disclosure is directed to a method of measuring GFAP in a biological sample from a subject. The method includes: (a) obtaining a biological sample from said subject, (b) contacting the biological sample with, either simultaneously or sequentially, in any order: (1) at least one capture antibody, which binds to an epitope on GFAP or GFAP fragment to form at least one capture antibody-GFAP antigen complex, and (2) at least one detection antibody which includes a detectable label and binds to an epitope on GFAP that is not bound by the at least one capture antibody, to form an at least one capture GFAP antigen-at least one detection antibody complex, and (c) determining the amount or concentration of GFAP in the biological sample based on the signal generated by the detectable label in the at least one capture antibody-GFAP antigen-at least one detection antibody complex, wherein the method can be used to determine levels of GFAP in an amount of less than or equal to 50,000 pg/mL, and wherein said method has a dynamic range of 5 log, and is linear over said dynamic range.

The present disclosure is directed to a method of assessing a subject's glial fibrillary acid protein (GFAP) status. The method includes the step of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

The present disclosure is directed to a method of assessing glial fibrillary acid protein (GFAP) status in a subject. The method includes the steps of: a) contacting a biological sample from said subject, either simultaneously or sequentially, in any order, with at least one first specific binding member and at least one second specific binding member, wherein the at least one first specific binding member and the at least one second specific binding member each specifically bind to GFAP thereby producing one or more first complexes comprising at least one first specific binding member-GFAP-at least one second specific binding member, wherein the at least one second specific binding member comprises a detectable label; and b) assessing a signal from the one or more first complexes, wherein the presence of a detectable signal from the detectable label indicates that GFAP is present in the sample, wherein the method can be used to determine levels of GFAP in an amount of less than or equal to 50,000 pg/mL, and wherein said method has a dynamic range of 5 log, and is linear over said dynamic range.

The present disclosure is directed to a method of measuring glial fibrillary acid protein (GFAP) status. The method includes the steps of: a) contacting a biological sample from said subject, either simultaneously or sequentially, in any order, with at least one first specific binding member and at least one second specific binding member, wherein the at least one first specific binding member and the at least one second specific binding member each specifically bind to GFAP thereby producing one or more first complexes comprising at least one first specific binding member-GFAP- at least one second specific binding member, wherein the at least one second specific binding member comprises a detectable label; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, such that the amount of detectable signal from the detectable label can be employed to assess said subject's GFAP status, wherein said assay is capable of determining an amount of GFAP less than or equal to 50,000 pg/mL in a volume of less than 20 microliters of test sample, wherein said assay has a dynamic range of 5 log, and is linear over said dynamic range.

The present disclosure is directed to a method of assessing a subject's glial fibrillary acid protein (GFAP) status. The method includes the steps of: (a) contacting a biological sample obtained from a human subject, either simultaneously or sequentially, in any order, with: (1) at least one capture antibody which is immobilized on a solid support and which binds to an epitope on human GFAP to form at least one capture antibody-GFAP antigen complex, and (2) at least one detection antibody which includes a detectable label and which binds to an epitope on human GFAP that is not bound by the capture antibody, to form at least one capture antibody-GFAP antigen-at least one detection antibody complex, wherein the at least one capture antibody and at least one detection antibody are monospecific antibodies, and optionally, are monoclonal antibodies, (b) detecting a signal generated by the detectable label in the at least one capture antibody-GFAP antigen-at least one detection antibody complex, wherein the presence of a detectable signal from the detectable label indicate that GFAP is present in the sample, and (c) measuring the amount of detectable signal from the detectable label indicates the amount of GFAP present in the sample, wherein the method is capable of quantitating the level of GFAP across a dynamic range from about 5 pg/mL to about 50,000 pg/mL with a precision of less than 10 % CV and with less than 10% deviation from linearity (DL) is achieved over the dynamic range.

The present disclosure is directed to a method of measuring glial fibrillary acid protein (GFAP) status. The method includes the steps of: a) contacting a biological sample from said subject, either simultaneously or sequentially, in any order, with at least one first specific binding member and at least one second specific binding member, wherein the at least one first specific binding member and the at least one second specific binding member each specifically bind to GFAP thereby producing one or more first complexes comprising the at least one first specific binding member-GFAP-at least one second specific binding member, wherein the at least one second specific binding member comprises a detectable label, wherein the at least one first specific binding member is immobilized on a solid support; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, wherein said assay is capable of determining the level of GFAP across a dynamic range from about 20 pg/mL to about 50,000 pg/mL with a precision of less than 10 % CV and with less than 10% deviation from linearity (DL) is achieved over the dynamic range in a volume of less than 20 microliters of test sample.

The present disclosure is directed to a method of assessing a subject's glial fibrillary acid protein (GFAP) status. The method includes the step of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

The present disclosure is directed to a method of assessing a subject's glial fibrillary acid protein (GFAP) status. The method includes the steps of: a) contacting a biological sample from said subject, either simultaneously or sequentially, in any order, with at least one first specific binding member and at least one second specific binding member, wherein the first specific binding member and the second specific binding member each specifically bind to GFAP thereby producing one or more first complexes comprising the first specific binding member-GFAP-second specific binding member; and b) detecting GFAP in the one or more first complexes present in the sample, wherein the method: (i) can be used to determine levels less than or equal to 50,000 pg/mL of GFAP and does not require dilution of the biological sample; or (ii) can be used to determine levels of GFAP in an amount of less than or equal to 50,000 pg/mL, and wherein said method has a dynamic range of 5 log, and is linear over said dynamic range, or (iii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

The present disclosure is directed to a method of assessing a subject's glial fibrillary acid protein (GFAP) status. The method includes the steps of: a) contacting a biological sample from said subject, either simultaneously or sequentially, in any order, with at least one first specific binding member and at least one second specific binding member, wherein the first specific binding member and the second specific binding member each specifically bind to GFAP thereby producing one or more first complexes comprising the first specific binding member-GFAP-second specific binding member, wherein either the first specific binding member or second specific binding member, comprise a detectable label; and b) assessing a signal from the one or more first complexes, wherein the amount of detectable signal from the detectable label indicates the amount of GFAP present in the sample, wherein the method: (i) can be used to determine levels of up to 50,000 pg/mL of GFAP and does not require dilution of the biological sample; or (ii) can be used to determine levels of GFAP in an amount of less than or equal to 50,000 pg/mL, and wherein said method has a dynamic range of 5 log, and is linear over said dynamic range, or (iii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

The present disclosure is directed to a method of measuring GFAP in a biological sample from a subject. The method includes (a) obtaining a biological sample from said subject; (b) contacting the biological sample with, either simultaneously or sequentially, in any order: (1) at least one capture antibody, which binds to an epitope on GFAP or GFAP fragment to form a capture antibody-GFAP antigen complex, and (2) at least one first detection antibody which includes a detectable label and binds to an epitope on GFAP that is not bound by the capture antibody, to form at least one capture antibody-GFAP antigen-at least one first detection antibody-complex, and (c) determining the amount or concentration of GFAP in the biological sample based on the signal generated by the detectable label in the at least one capture antibody-GFAP antigen-at least one first detection antibody complex, wherein the method: (i)can be used to determine levels of GFAP in an amount of less than or equal to 50,000 pg/mL, and wherein said method has a dynamic range of 5 log, and is linear over said dynamic range; or (ii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

Each of the above described methods can be conducted on a point-of-care device. In each of the above described methods, the UCH-L1 can be detected by an immunoassay or a single molecule detection assay.

## BRIEF DESCRIPTION OF THE DRAWINGS

FIG. 1 shows a GFAP calibration curve.
FIG. 2 shows the GFAP sample distribution in normal (i.e., apparently healthy) donors.
FIG. 3 shows the GFAP biomarker profiles. Timepoints are as described in Example 3.
FIG. 4 shows box plots that show a wide distribution of GFAP results across the patient population. Sample timepoints are as in FIG. 3.
FIG. 5 shows the expected versus observed concentrations of GFAP in Dilution 1 (as described in Example 1).
FIG. 6 shows the expected versus observed concentrations of GFAP in Dilution 2 (as described in Example 1).

## DETAILED DESCRIPTION

The present disclosure relates to improved assays for aiding in the detection, analyzing, or detecting and analyzing the levels of GFAP in a biological or test sample. The improved assays as described herein can be any type of assay known in the art. One preferred type of assay is an immunoassay. The improved assays can be employed to detect, analyze, or detect and analyze or evaluate GFAP for any purpose. In one embodiment, the improved assays can be used to detect, analyze, detect and analyze or evaluate the levels of GFAP in a biological or test sample to rapidly aid in the diagnosis of a traumatic brain injury (TBI), monitor progression, and/or predict outcome in subjects who are either suspected of sustaining an injury to the head or that have sustained an actual injury to the head.

The improved immunoassays surprisingly can be used to measure or assess GFAP at low levels in a biological sample over a wide range of concentrations and thus provide a more versatile and sensitive assay, e.g., for aid in the diagnosing and distinguishing TBI in a patient. In particular, the increased range of concentration of the disclosed immunoassays provides a more accurate and sensitive assay and can optionally be used for aiding in the diagnosing and distinguishing TBI in a patient, for evaluating head injury and/or concussion, for predicting a need for imaging, for predicting severity of the traumatic brain injury, and for prognosticating traumatic brain injury. Thus, the disclosed immunoassays may be used to determine increased or decreased GFAP concentrations at low or higher levels of GFAP in a diluted or undiluted sample compared to a control or calibrator sample, and thus optionally can be used to identify TBI in a patient. In addition, the disclosed immunoassays are linear over the dynamic range of the assay. Moreover, the disclosed immunoassays have a dynamic range of equal to or less than five logs (namely, 5 - 50,000). The use of the GFAP immunoassay may, for example, provide an aid in the accurate diagnosis of and subsequent treatment of patients, e.g., patients with TBI.

State of the art assays, such as immunoassays, used to determine GFAP levels in a biological or test sample may not be able to detect GFAP levels that are outside of the dynamic range of the improved assays described herein. When this occurs, the GFAP levels either have to be re-measured after dilution (e.g., in the case where the sample concentration exceeds the upper detection limit), or using higher sample volumes (e.g., in the case where the GFAP concentrations measured are below the limit of detection (LoD)). Such re-measurements are problematic due to the additional expense incurred as well as loss of time, both of which are problematic particularly when the assay is used to aid in the diagnosis of, diagnose, monitor progression or predict outcome in subjects suspected of or who have sustained an actual TBI (or other GFAP-associated critical disease, disorder or condition), where fast, accurate, cost-effective detection is critical. Therefore, improved assays that increase or expand the dynamic range of the assays known in the art would reduce the number of reruns and allow for the rapid, accurate and cost-effective aid in the diagnosis of patients, including those with traumatic brain injury, in subjects in need thereof.

The assays of the present disclosure exhibit a number of improvements over the assays known in the art. Specifically, the assays of the present disclosure exhibit increased dynamic range and sensitivity. In one aspect, the assays of the present disclosure exhibit lower limit of detection (LoD) of about 1 pg/mL, 5 pg/mL, about 10 pg/mL, about 15 pg/mL, about 20 pg/mL, about 25 pg/mL or about 30 pg/mL. Additionally, the assays of the present disclosure exhibit a dynamic range of equal to or less than five logs (namely, 5 pg/mL - 50,000 pg/mL). One example of an improved assay of the present disclosure is an immunoassay having a LoD of about 10 pg/mL or about 20 pg/mL. Another example of an improved assay is an immunoassay having a dynamic range of equal to or less than five logs. The improved low end sensitivity of the assays of the present disclosure avoids the problem of re-measurement of samples discussed previously herein. Moreover, the biological or test samples used in the assays of the present disclosure may be diluted or undiluted - there should be no requirement to dilute.

Additionally, the improved assays of the present disclosure can be performed or conducted quickly, and provide results in less than about 5 minutes, less than about 6 minutes, less than about 7 minutes, less than about 8 minutes, less than about 9 minutes, less than about 10 minutes, less than about 11 minutes, less than about 12 minutes, less than about 13 minutes, less than about 14 minutes, less than about 15 minutes, less than about 16 minutes, less than about 17 minutes, less than about 18 minutes, less than about 19 minutes and less than about 20 minutes from when the assay is started or commenced. One example of an improved assay of the present disclosure is an immunoassay that provides a result in less than about 10 minutes after it is started or commenced. Another example of an improved assay of the present disclosure is an immunoassay having a LoD of about 10 pg/mL and that provides a result in less than 10 minutes. Still another example of an improved assay of the present disclosure is an immunoassay having a LoD of about 20 pg/mL and that provides a result in less than 10 minutes.

Because the assays of the present disclosure are performed and provide results quickly, the amount of signal produced by the assay is controlled and oversaturation of the signal is reduced. Because such oversaturation of the signal is reduced when compared to the assays known in the art, the assays of the present disclosure exhibit less or reduced hook effect compared to other assays known in the art.

The improved assays of the present disclosure when used to measure or assess GFAP at low levels in a biological sample over a wide range of concentrations provide a more versatile and sensitive assay for assessing traumatic brain injury over the assays currently known in the art. As a result, the increased range of concentration of the disclosed assays provide a more accurate and sensitive assay for aiding in the diagnosing of and distinguishing traumatic brain injury in a patient. Thus, the improved assays of the present disclosure may be used to determine increased or decreased GFAP concentrations at low or higher levels of GFAP in a diluted or undiluted sample compared to a control or calibrator sample, and thus can be used to identify TBI in a patient.

Without being bound by theory, it is believed that there are a number of reasons that contribute to and result in the surprising improved assays of the present disclosure. A key reason appears to be the reduction in assay time, thereby reducing the likelihood of hook effect. Hook effect (or prozone phenomena) can also be avoided by other means known in the art (e.g., increasing conjugate concentration), some of which can destroy low end sensitivity of an assay and potentially cause saturation of signal at the high end. However, in this case care was taken to maintain the low end sensitivity of the assay, e.g., by optimization of the concentration of the reagents used in the assay. Furthermore, care was taken in the screening and selection of antibodies having different binding specificities for GFAP, allowing the antibodies to bind to different sites and thus be employed for either capture or detection. Such optimization can be done using routine techniques in the art.

Also, it has been found that using at least two antibodies that bind non-overlapping epitopes within GFAP breakdown products (BDP), such as the 38 kDa BDP defined by amino acids 60-383 of the GFAP protein sequence (SEQ ID NO: 1), may assist with maintaining the dynamic range and low end sensitivity of the immunoassays. In one aspect, at least two antibodies bind non-overlapping epitopes near the N-terminus of the 38 kDa BDP. In another aspect, at least two antibodies bind non-overlapping epitopes between amino acids 60-383 of SEQ ID NO: 1. In another aspect, at least one first antibody (such as a capture antibody) binds to an epitope near the N-terminus of the 38 kDa BDP and at least one second antibody (such as a detection antibody) binds to an epitope near the middle of the 38 kDa BDP that does not overlap with the first antibody. In another aspect, at least one first antibody (such as a capture antibody) binds to an epitope between amino acids 60-383 of SEQ ID NO:1 and at least one second antibody binds to an epitope between amino acids 60-383 of SEQ ID NO:1 that do not overlap with the first antibody. The epitope bound by first antibody may be 10 amino acids, 11 amino acids, 12 amino acids, 13 amino acids, 14 amino acids or 15 amino acids in length. The epitope bound by the second antibody may be 10 amino acids, 11 amino acids, 12 amino acids, 13 amino acids, 14 amino acids or 15 amino acids in length. One skilled in the art could readily determine antibodies binding to non-overlapping epitopes within the 38 kDa BDP defined by amino acids 60-383 of SEQ ID NO:1 using routine techniques known in the art.

Likewise it is possible that other antibodies can be selected which similarly may assist with maintaining the dynamic range and low end sensitivity of the immunoassays. For example, it may be useful to select at least one first antibody (such as a capture antibody) that binds to an epitope near the N-terminus of the 38 kDa BDP and at least one second antibody (such as a detection antibody) that binds to an epitope near the middle of the 38 kDa BDP, e.g., near the middle of the 38 kDa BDP, and that does not overlap with the first antibody. Other variations are possible and could be readily tested by one of ordinary skill (e.g., using the methods set forth herein in Example 1). E.g., by confirming antibodies bind to different epitopes by examining binding to short peptides, and then screening antibody pairs using low calibrator concentration. Moreover, selecting antibodies of differing affinity for GFAP also can assist with maintaining or increasing the dynamic range of the assay. GFAP antibodies have been described in the literature and are commercially available (e.g., section 4.e herein).

Section headings as used in this section and the entire disclosure herein are merely for organizational purposes and are not intended to be limiting.

## 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present disclosure. All publications, patent applications, patents and other references mentioned herein are incorporated by reference in their entirety. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

"Affinity matured antibody" is used herein to refer to an antibody with one or more alterations in one or more CDRs, which result in an improvement in the affinity (i.e. $K_D$, $k_d$ or $k_a$) of the antibody for a target antigen compared to a parent antibody, which does not possess the alteration(s). Exemplary affinity matured antibodies will have nanomolar or even picomolar affinities for the target antigen. A variety of procedures for producing affinity matured antibodies is known in the art, including the screening of a combinatory antibody library that has been prepared using bio-display. For example, Marks et al., BioTechnology, 10: 779-783 (1992) describes affinity maturation by VH and VL domain shuffling. Random mutagenesis of CDR and/or framework residues is described by Barbas et al., Proc. Nat. Acad. Sci. USA, 91: 3809-3813 (1994); Schier et al., Gene, 169: 147-155 (1995); Yelton et al., J. Immunol, 155: 1994-2004 (1995); Jackson et al., J. Immunol., 154(7): 3310-3319 (1995); and Hawkins et al, J. Mol. Biol., 226: 889-896 (1992). Selective mutation at selective mutagenesis positions and at contact or hypermutation positions with an activity-enhancing amino acid residue is described in U.S. Patent No. 6,914,128 B1.

"Antibody" and "antibodies" as used herein refers to monoclonal antibodies, monospecific antibodies (e.g., which can either be monoclonal, or may also be produced by other means than producing them from a common germ cell), multispecific antibodies, human antibodies, humanized antibodies (fully or partially humanized), animal antibodies such as, but not limited to, a bird (for example, a duck or a goose), a shark, a whale, and a mammal, including a non-primate (for example, a cow, a pig, a camel, a llama, a horse, a goat, a rabbit, a sheep, a hamster, a guinea pig, a cat, a dog, a rat, a mouse, etc.) or a non-human primate (for example, a monkey, a chimpanzee, etc.), recombinant antibodies, chimeric antibodies, single-chain Fvs ("scFv"), single chain antibodies, single domain antibodies, Fab fragments, F(ab') fragments, F(ab')$_2$ fragments, disulfide-linked Fvs ("sdFv"), and anti-idiotypic ("anti-Id") antibodies, dual-domain antibodies, dual variable domain (DVD) or triple variable domain (TVD) antibodies (dual-variable domain immunoglobulins and methods for making them are described in Wu, C., et al., Nature Biotechnology, 25(11):1290-1297 (2007) and PCT International Application WO 2001/058956, the contents of each of which are herein incorporated by reference), or domain antibodies (dAbs) (e.g., such as described in Holt et al. (2014) Trends in Biotechnology 21:484-490), and including single domain antibodies sdAbs that are naturally occurring, e.g., as in cartilaginous fishes and camelid, or which are synthetic, e.g., nanobodies, VHH, or other domain structure), and functionally active epitope-binding fragments of any of the above. In particular, antibodies include immunoglobulin molecules and immunologically active fragments of immunoglobulin molecules, namely, molecules that contain an analyte-binding site. Immunoglobulin molecules can be of any type (for example, IgG, IgE, IgM, IgD, IgA, and IgY), class (for example, IgG1, IgG2, IgG3, IgG4, IgA1, and IgA2), or subclass. For simplicity sake, an antibody against an analyte is frequently referred to herein as being either an "anti-analyte antibody" or merely an "analyte antibody" (e.g., an anti-GFAP antibody or a GFAP antibody).

"Antibody fragment" as used herein refers to a portion of an intact antibody comprising the antigen-binding site or variable region. The portion does not include the constant heavy chain domains (i.e. CH2, CH3, or CH4, depending on the antibody isotype) of the Fc region of the intact antibody. Examples of antibody fragments include, but are not limited to, Fab fragments, Fab' fragments, Fab'-SH fragments, $F(ab')_2$ fragments, Fd fragments, Fv fragments, diabodies, single-chain Fv (scFv) molecules, single-chain polypeptides containing only one light chain variable domain, single-chain polypeptides containing the three CDRs of the light-chain variable domain, single-chain polypeptides containing only one heavy chain variable region, and single-chain polypeptides containing the three CDRs of the heavy chain variable region.

The "area under curve" or "AUC" refers to area under a ROC curve. AUC under a ROC curve is a measure of accuracy. An AUC of 1 represents a perfect test, whereas an AUC of 0.5 represents an insignificant test. A preferred AUC may be at least approximately 0.700, at least approximately 0.750, at least approximately 0.800, at least approximately 0.850, at least approximately 0.900, at least approximately 0.910, at least approximately 0.920, at least approximately 0.930, at least approximately 0.940, at least approximately 0.950, at least approximately 0.960, at least approximately 0.970, at least approximately 0.980, at least approximately 0.990, or at least approximately 0.995.

"Bead" and "particle" are used herein interchangeably and refer to a substantially spherical solid support. One example of a bead or particle is a microparticle. Microparticles that can be used herein can be any type known in the art. For example, the bead or particle can be a magnetic bead or magnetic particle. Magnetic beads/particles may be ferromagnetic, ferrimagnetic, paramagnetic, superparamagnetic or ferrofluidic. Exemplary ferromagnetic materials include Fe, Co, Ni, Gd, Dy, $CrO_2$, MnAs, MnBi, EuO, and NiO/Fe. Examples of ferrimagnetic materials include $NiFe_2O_4$, $CoFe_2O_4$, $Fe_3O_4$ (or $FeO\cdot Fe_2O_3$). Beads can have a solid core portion that is magnetic and is surrounded by one or more non-magnetic layers. Alternately, the magnetic portion can be a layer around a non-magnetic core. The microparticles can be of any size that would work in the methods described herein, e.g., from about 0.75 to about 5 nm, or from about 1 to about 5 nm, or from about 1 to about 3 nm.

"Binding protein" is used herein to refer to a monomeric or multimeric protein that binds to and forms a complex with a binding partner, such as, for example, a polypeptide, an antigen, a chemical compound or other molecule, or a substrate of any kind. A binding protein specifically binds a binding partner. Binding proteins include antibodies, as well as antigen-binding fragments thereof and other various forms and derivatives thereof as are known in the art and described herein below, and other molecules comprising one or more antigen-binding domains that bind to an antigen molecule or a particular site (epitope) on the antigen molecule. Accordingly, a binding protein includes, but is not limited to, an antibody a tetrameric immunoglobulin, an IgG molecule, an IgG1 molecule, a monoclonal antibody, a chimeric antibody, a CDR-grafted antibody, a humanized antibody, an affinity matured antibody, and fragments of any such antibodies that retain the ability to bind to an antigen.

"Bispecific antibody" is used herein to refer to a full-length antibody that is generated by quadroma technology (see Milstein et al., Nature, 305(5934): 537-540 (1983)), by chemical conjugation of two different monoclonal antibodies (see, Staerz et al., Nature, 314(6012): 628-631 (1985)), or by knob-into-hole or similar approaches, which introduce mutations in the Fc region (see Holliger et al., Proc. Natl. Acad. Sci. USA, 90(14): 6444-6448 (1993)), resulting in multiple different immunoglobulin species of which only one is the functional bispecific antibody. A bispecific antibody binds one antigen (or epitope) on one of its two binding arms (one pair of HC/LC), and binds a different antigen (or epitope) on its second arm (a different pair of HC/LC). By this definition, a bispecific antibody has two distinct antigen-binding arms (in both specificity and CDR sequences), and is monovalent for each antigen to which it binds to.

"CDR" is used herein to refer to the "complementarity determining region" within an antibody variable sequence. There are three CDRs in each of the variable regions of the heavy chain and the light chain. Proceeding from the N-terminus of a heavy or light chain, these regions are denoted "CDR1", "CDR2", and "CDR3", for each of the variable regions. The term "CDR set" as used herein refers to a group of three CDRs that occur in a single variable region that binds the antigen. An antigen-binding site, therefore, may include six CDRs, comprising the CDR set from each of a heavy and a light chain variable region. A polypeptide comprising a single CDR, (e.g., a CDR1, CDR2, or CDR3) may be referred to as a "molecular recognition unit." Crystallographic analyses of antigen-antibody complexes have demonstrated that the amino acid residues of CDRs form extensive contact with bound antigen, wherein the most extensive antigen contact is with the heavy chain CDR3. Thus, the molecular recognition units may be primarily responsible for the specificity of an antigen-binding site. In general, the CDR residues are directly and most substantially involved in influencing antigen binding.

The exact boundaries of these CDRs have been defined differently according to different systems. The system described by Kabat (Kabat et al., Sequences of Proteins of Immunological Interest (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as "Kabat CDRs". Chothia and coworkers (Chothia and Lesk, J. Mol. Biol., 196: 901-917 (1987); and Chothia et al., Nature, 342: 877-883 (1989)) found that certain sub-portions within Kabat CDRs adopt nearly identical peptide backbone conformations, despite having great diversity at the level of amino acid sequence. These sub-portions were designated as "L1", "L2", and "L3", or "H1", "H2", and "H3", where the "L" and the "H" designate the light chain and the heavy chain regions, respectively. These regions may be referred to as "Chothia CDRs", which have boundaries that overlap with Kabat CDRs. Other boundaries defining CDRs overlapping with the Kabat CDRs have been described by Padlan, FASEB J., 9: 133-139 (1995), and MacCallum, J. Mol. Biol., 262(5): 732-745 (1996). Still other CDR boundary definitions may not strictly follow one of the herein systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although certain embodiments use Kabat- or Chothia-defined CDRs.

"Coefficient of variation" (CV), also known as "relative variability," is equal to the standard deviation of a distribution divided by its mean.

"Component," "components," or "at least one component," refer generally to a capture antibody, a detection or conjugate a calibrator, a control, a sensitivity panel, a container, a buffer, a diluent, a salt, an enzyme, a co-factor for an enzyme, a detection reagent, a pretreatment reagent/solution, a substrate (e.g., as a solution), a stop solution, and the like that can be included in a kit for assay of a test sample, such as a patient urine, whole blood, serum or plasma sample, in accordance with the methods described herein and other methods known in the art. Some components can be in solution or lyophilized for reconstitution for use in an assay.

"CT scan" as used herein refers to a computerized tomography (CT) scan. A CT scan combines a series of X-ray images taken from different angles and uses computer processing to create cross-sectional images, or slices, of the bones, blood vessels and soft tissues inside your body. The CT scan may use X-ray CT, positron emission tomography (PET), single-photon emission computed tomography (SPECT), computed axial tomography (CAT scan), or computer aided tomography. The CT scan may be a conventional CT scan or a spiral/helical CT scan. In a conventional CT scan, the scan is taken slice by slice and after each slice the scan stops and moves down to the next slice, e.g., from the top of the abdomen down to the pelvis. The conventional CT scan requires patients to hold their breath to avoid movement artefact. The spiral/helical CT scan is a continuous scan which is taken in a spiral fashion and is a much quicker process where the scanned images are contiguous.

"Derivative" of an antibody as used herein may refer to an antibody having one or more modifications to its amino acid sequence when compared to a genuine or parent antibody and exhibit a modified domain structure. The derivative may still be able to adopt the typical domain configuration found in native antibodies, as well as an amino acid sequence, which is able to bind to targets (antigens) with specificity. Typical examples of antibody derivatives are antibodies coupled to other polypeptides, rearranged antibody domains, or fragments of antibodies. The derivative may also comprise at least one further compound, e.g. a protein domain, said protein domain being linked by covalent or non-covalent bonds. The linkage can be based on genetic fusion according to the methods known in the art. The additional domain present in the fusion protein comprising the antibody may preferably be linked by a flexible linker, advantageously a peptide linker, wherein said peptide linker comprises plural, hydrophilic, peptide-bonded amino acids of a length sufficient to span the distance between the C-terminal end of the further protein domain and the N-terminal end of the antibody or vice versa. The antibody may be linked to an effector molecule having a conformation suitable for biological activity or selective binding to a solid support, a biologically active substance (e.g. a cytokine or growth hormone), a chemical agent, a peptide, a protein, or a drug, for example.

"Drugs of abuse" is used herein to refer to one or more addictive substances (such as a drug) taken for non-medical reasons (such as for, example, recreational and/or mind-altering effects). Excessive overindulgence, use or dependence of such drugs of abuse is often referred to as "substance abuse." Examples of drugs of abuse include alcohol, barbiturates, benzodiazepines, cannabis, cocaine, hallucinogens (such as ketamine, mescaline (peyote), PCP, psilocybin, DMT and/or LSD), methaqualone, opioids, amphetamines (including methamphetamines), anabolic steroids, inhalants (namely, substances which contain volatile substances that contain psychoactive properties such as, for example, nitrites, spray paints, cleaning fluids, markers, glues, etc.) and combinations thereof.

"Dual-specific antibody" is used herein to refer to a full-length antibody that can bind two different antigens (or epitopes) in each of its two binding arms (a pair of HC/LC) (see PCT publication WO 02/02773). Accordingly, a dual-specific binding protein has two identical antigen binding arms, with identical specificity and identical CDR sequences, and is bivalent for each antigen to which it binds.

"Dual variable domain" is used herein to refer to two or more antigen binding sites on a binding protein, which may be divalent (two antigen binding sites), tetravalent (four antigen binding sites), or multivalent binding proteins. DVDs may be monospecific, i.e., capable of binding one antigen (or one specific epitope), or multispecific, i.e., capable of binding two or more antigens (i.e., two or more epitopes of the same target antigen molecule or two or more epitopes of different target antigens). A preferred DVD binding protein comprises two heavy chain DVD polypeptides and two light chain DVD polypeptides and is referred to as a "DVD immunoglobulin" or "DVD-Ig." Such a DVD-Ig binding protein is thus tetrameric and reminiscent of an IgG molecule, but provides more antigen binding sites than an IgG molecule. Thus, each half of a tetrameric DVD-Ig molecule is reminiscent of one half of an IgG molecule and comprises a heavy chain DVD polypeptide and a light chain DVD polypeptide, but unlike a pair of heavy and light chains of an IgG molecule that provides a single antigen binding domain, a pair of heavy and light chains of a DVD-Ig provide two or more antigen binding sites.

Each antigen binding site of a DVD-Ig binding protein may be derived from a donor ("parental") monoclonal antibody and thus comprises a heavy chain variable domain (VH) and a light chain variable domain (VL) with a total of six CDRs involved in antigen binding per antigen binding site. Accordingly, a DVD-Ig binding protein that binds two different epitopes (i.e., two different epitopes of two different antigen molecules or two different epitopes of the same antigen molecule) comprises an antigen binding site derived from a first parental monoclonal antibody and an antigen binding site of a second parental monoclonal antibody.

A description of the design, expression, and characterization of DVD-Ig binding molecules is provided in PCT Publication No. WO 2007/024715, U.S. Patent No. 7,612,181, and Wu et al., Nature Biotech., 25: 1290-1297 (2007). A preferred example of such DVD-Ig molecules comprises a heavy chain that comprises the structural formula VD1-(X1)n-VD2-C-(X2)n, wherein VD1 is a first heavy chain variable domain, VD2 is a second heavy chain variable domain, C is a heavy chain constant domain, X1 is a linker with the proviso that it is not CH1, X2 is an Fc region, and n is 0 or 1, but preferably 1; and a light chain that comprises the structural formula VD1-(X1)n-VD2-C-(X2)n, wherein VD1 is a first light chain variable domain, VD2 is a second light chain variable domain, C is a light chain constant domain, X1 is a linker with the proviso that it is not CH1, and X2 does not comprise an Fc region; and n is 0 or 1, but preferably 1. Such a DVD-Ig may comprise two such heavy chains and two such light chains, wherein each chain comprises variable domains linked in tandem without an intervening constant region between variable regions, wherein a heavy chain and a light chain associate to form tandem functional antigen binding sites, and a pair of heavy and light chains may associate with another pair of heavy and light chains to form a tetrameric binding protein with four functional antigen binding sites. In another example, a DVD-Ig molecule may comprise heavy and light chains that each comprise three variable domains (VD1, VD2, VD3) linked in tandem without an intervening constant region between variable domains, wherein a pair of heavy and light chains may associate to form three antigen binding sites, and wherein a pair of heavy and light chains may associate with another pair of heavy and light chains to form a tetrameric binding protein with six antigen binding sites.

In a preferred embodiment, a DVD-Ig binding protein not only binds the same target molecules bound by its parental monoclonal antibodies, but also possesses one or more desirable properties of one or more of its parental monoclonal antibodies. Preferably, such an additional property is an antibody parameter of one or more of the parental monoclonal antibodies. Antibody parameters that may be contributed to a DVD-Ig binding protein from one or more of its parental monoclonal antibodies include, but are not limited to, antigen specificity, antigen affinity, potency, biological function, epitope recognition, protein stability, protein solubility, production efficiency, immunogenicity, pharmacokinetics, bioavailability, tissue cross reactivity, and orthologous antigen binding.

A DVD-Ig binding protein binds at least one epitope of GFAP. Non-limiting examples of a DVD-Ig binding protein include a DVD-Ig binding protein that binds one or more epitopes of GFAP, a DVD-Ig binding protein that binds an epitope of a human GFAP and an epitope of GFAP of another species (for example, mouse), and a DVD-Ig binding protein that binds an epitope of a human GFAP and an epitope of another target molecule.

"Drugs of abuse" is used herein to refer to one or more addictive substances (such as a drug) taken for non-medical reasons (such as for, example, recreational and/or mind-altering effects). Excessive overindulgence, use or dependence of such drugs of abuse is often referred to as "substance abuse." Examples of drugs of abuse include alcohol, barbiturates, benzodiazepines, cannabis, cocaine, hallucinogens (such as ketamine, mescaline (peyote), PCP, psilocybin, DMT and/or LSD), methaqualone, opioids, amphetamines (including methamphetamines), anabolic steroids, inhalants (namely, substances which contain volatile substances that contain psychoactive properties such as, for example, nitrites, spray paints, cleaning fluids, markers, glues, etc.) and combinations thereof.

"Dynamic range" as used herein refers to range over which an assay readout is proportional to the amount of target molecule or analyte in the sample being analyzed. The dynamic range can be the range of linearity of the standard curve.

"Epitope," or "epitopes," or "epitopes of interest" refer to a site(s) on any molecule that is recognized and can bind to a complementary site(s) on its specific binding partner. The molecule and specific binding partner are part of a specific binding pair. For example, an epitope can be on a polypeptide, a protein, a hapten, a carbohydrate antigen (such as, but not limited to, glycolipids, glycoproteins or lipopolysaccharides), or a polysaccharide. Its specific binding partner can be, but is not limited to, an antibody.

"Expanded window of detection" as used herein refers to the fact that the described and/or claimed improved methods can be carried out in or under a variety of clinical scenarios when compared to other GFAP assays. For example, the methods of the present disclosure can be carried out on any subject without regard to factors selected from the group consisting of the subject's clinical condition (e.g., whether or not there are comorbid conditions in addition to the reason for checking on GFAP, or whether some clinical situation other than TBI is being assessed), the subject's laboratory values (e.g., laboratory values other than GFAP levels, including but not limited to values on standard laboratory tests that are run to assess a patient's overall health, and values on more particularized tests that are run when a subject is suspected of having been in an accident or exposed to some sort of trauma including but not limited to those that may result in head injury), the subject's classification as suffering from mild, moderate or severe TBI, the subject's exhibition (e.g., demonstration or possession) of low or high levels of GFAP, and the timing of any event (e.g., relative to testing) where the subject may have sustained an injury to the head. The expanded window of detection of the claimed methods differ from other methods known in the prior art which may or require dilution, or alternately, may lack one or more of the benefits of the improved assays as described herein (e.g., measure up to 50,000 pg/mL, dynamic range of 5 log, assay linearity over the dynamic range, measure of GFAP in a volume less than 20 microliters of sample, expanded window of detection, etc.).

"Fragment antigen-binding fragment" or "Fab fragment" as used herein refers to a fragment of an antibody that binds to antigens and that contains one antigen-binding site, one complete light chain, and part of one heavy chain. Fab is a monovalent fragment consisting of the VL, VH, CL and CH1 domains. Fab is composed of one constant and one variable domain of each of the heavy and the light chain. The variable domain contains the paratope (the antigen-binding site), comprising a set of complementarity determining regions, at the amino terminal end of the monomer. Each arm of the Y thus binds an epitope on the antigen. Fab fragments can be generated such as has been described in the art, e.g., using the enzyme papain, which can be used to cleave an immunoglobulin monomer into two Fab fragments and an Fc fragment, or can be produced by recombinant means.

"F(ab')$_2$ fragment" as used herein refers to antibodies generated by pepsin digestion of whole IgG antibodies to remove most of the Fc region while leaving intact some of the hinge region. F(ab')$_2$ fragments have two antigen-binding F(ab) portions linked together by disulfide bonds, and therefore are divalent with a molecular weight of about 110 kDa. Divalent antibody fragments (F(ab')$_2$ fragments) are smaller than whole IgG molecules and enable a better penetration into tissue thus facilitating better antigen recognition in immunohistochemistry. The use of F(ab')$_2$ fragments also avoids unspecific binding to Fc receptor on live cells or to Protein A/G. F(ab')$_2$ fragments can both bind and precipitate antigens.

"Framework" (FR) or "Framework sequence" as used herein may mean the remaining sequences of a variable region minus the CDRs. Because the exact definition of a CDR sequence can be determined by different systems (for example, see above), the meaning of a framework sequence is subject to correspondingly different interpretations. The six CDRs (CDR-L1, -L2, and -L3 of light chain and CDR-H1, -H2, and -H3 of heavy chain) also divide the framework regions on the light chain and the heavy chain into four sub-regions (FR1, FR2, FR3, and FR4) on each chain, in which CDR1 is positioned between FR1 and FR2, CDR2 between FR2 and FR3, and CDR3 between FR3 and FR4. Without specifying the particular sub-regions as FR1, FR2, FR3, or FR4, a framework region, as referred by others, represents the combined FRs within the variable region of a single, naturally occurring immunoglobulin chain. As used herein, a FR represents one of the four sub-regions, and FRs represents two or more of the four sub-regions constituting a framework region.

Human heavy chain and light chain FR sequences are known in the art that can be used as heavy chain and light chain "acceptor" framework sequences (or simply, "acceptor" sequences) to humanize a non-human antibody using techniques known in the art. In one embodiment, human heavy chain and light chain acceptor sequences are selected from the framework sequences listed in publicly available databases such as V-base (hypertext transfer protocol://vbase.mrc-cpe.cam.ac.uk/) or in the international ImMunoGeneTics® (IMGT®) information system (hypertext transfer protocol://imgt.cines.fr/texts/IMGTrepertoire/LocusGenes/).

"Functional antigen binding site" as used herein may mean a site on a binding protein (e.g. an antibody) that is capable of binding a target antigen. The antigen binding affinity of the antigen binding site may not be as strong as the parent binding protein, e.g., parent antibody, from which the antigen binding site is derived, but the ability to bind antigen must be measurable using any one of a variety of methods known for evaluating protein, e.g., antibody, binding to an antigen. Moreover, the antigen binding affinity of each of the antigen binding sites of a multivalent protein, e.g., multivalent antibody, herein need not be quantitatively the same.

"GFAP" is used herein to describe glial fibrillary acidic protein. GFAP is a protein that is encoded by the *GFAP* gene in humans, and which can be produced (e.g., by recombinant means), in other species.

"GFAP status" can mean either the level or amount of GFAP at a point in time (such as with a single measure of GFAP), the level or amount of GFAP associated with monitoring (such as with a repeat test on a subject to identify an increase or decrease in GFAP amount), the level or amount of GFAP associated with treatment for traumatic brain injury (whether a primary brain injury and/or a secondary brain injury) or combinations thereof.

"Glasgow Coma Scale" or "GCS" as used herein refers to a 15 point scale for estimating and categorizing the outcomes of brain injury on the basis of overall social capability or dependence on others. The test measures the motor response, verbal response and eye opening response with these values: I. Motor Response (6 - Obeys commands fully; 5 - Localizes to noxious stimuli; 4 - Withdraws from noxious stimuli; 3 - Abnormal flexion, i.e. decorticate posturing; 2 - Extensor response, i.e. decerebrate posturing; and 1 - No response); II. Verbal Response (5 - Alert and Oriented; 4 - Confused, yet coherent, speech; 3 - Inappropriate words and jumbled phrases consisting of words; 2 - Incomprehensible sounds; and 1 - No sounds); and III. Eye Opening (4 - Spontaneous eye opening; 3 - Eyes open to speech; 2 - Eyes open to pain; and 1 - No eye opening). The final score is determined by adding the values of I+II+III. The final score can be categorized into four possible levels for survival, with a lower number indicating a more severe injury and a poorer prognosis: Mild (13-15); Moderate Disability (9-12) (Loss of consciousness greater than 30 minutes; Physical or cognitive impairments which may or may resolve: and Benefit from Rehabilitation); Severe Disability (3-8) (Coma: unconscious state. No meaningful response, no voluntary activities); and Vegetative State (Less Than 3) (Sleep wake cycles; Arousal, but no interaction with environment; No localized response to pain). Moderate brain injury is defined as a brain injury resulting in a loss of consciousness from 20 minutes to 6 hours and a Glasgow Coma Scale of 9 to 12. Severe brain injury is defined as a brain injury resulting in a loss of consciousness of greater than 6 hours and a Glasgow Coma Scale of 3 to 8.

"Glasgow Outcome Scale" as used herein refers to a global scale for functional outcome that rates patient status into one of five categories: Dead, Vegetative State, Severe Disability, Moderate Disability or Good Recovery.

"Extended Glasgow Outcome Scale" or "GOSE" as used interchangeably herein provides more detailed categorization into eight categories by subdividing the categories of severe disability, moderate disability and good recovery into a lower and upper category as shown in Table 1.

**Table 1**

| 1 | Death | D | |
|---|---|---|---|
| 2 | Vegetative state | VX | Condition of unawareness with only reflex responses but with periods of spontaneous eye opening |
| 3 | Lower severe disability | SD - | Patient who is dependent for daily support for mental or physical disability, usually a combination of both. If the patient can be left alone for more than 8 hours at home it is upper level of SD, if not then it is low level of SD. |
| 4 | Upper severe disability | SD + | |
| 5 | Lower moderate disability | MD - | Patients have some disability such as aphasia, hemiparesis or epilepsy and/or deficits of memory or personality but are able to look after themselves. They are independent at home but dependent outside. If they are able to return to work even with special arrangement it is upper level of MD, if not then it is low level of MD. |
| 6 | Upper moderate disability | MD + | |
| 7 | Lower good recovery | GR - | Resumption of normal life with the capacity to work even if pre-injury status has not been achieved. Some patients have minor neurological or psychological deficits. If these deficits are not disabling then it is upper level of GR, if disabling then it is lower level of GR. |
| 8 | Upper good recovery | GR + | |

"Humanized antibody" is used herein to describe an antibody that comprises heavy and light chain variable region sequences from a non-human species (e.g. a mouse) but in which at least a portion of the VH and/or VL sequence has been altered to be more "human-like," i.e., more similar to human germline variable sequences. A "humanized antibody" is an antibody or a variant, derivative, analog, or fragment thereof, which immunospecifically binds to an antigen of interest and which comprises a framework (FR) region having substantially the amino acid sequence of a human antibody and a complementary determining region (CDR) having substantially the amino acid sequence of a non-human antibody. As used herein, the term "substantially" in the context of a CDR refers to a CDR having an amino acid sequence at least 80%, at least 85%, at least 90%, at least 95%, at least 98%, or at least 99% identical to the amino acid sequence of a non-human antibody CDR. A humanized antibody comprises substantially all of at least one, and typically two, variable domains (Fab, Fab', F(ab')$_2$, FabC, Fv) in which all or substantially all of the CDR regions correspond to those of a non-human immunoglobulin (i.e., donor antibody) and all or substantially all of the framework regions are those of a human immunoglobulin consensus sequence. In an embodiment, a humanized antibody also comprises at least a portion of an immunoglobulin constant region (Fc), typically that of a human immunoglobulin. In some embodiments, a humanized antibody contains the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or humanized heavy chain.

A humanized antibody can be selected from any class of immunoglobulins, including IgM, IgG, IgD, IgA, and IgE, and any isotype, including without limitation IgG1, IgG2, IgG3, and IgG4. A humanized antibody may comprise sequences from more than one class or isotype, and particular constant domains may be selected to optimize desired effector functions using techniques well-known in the art.

The framework regions and CDRs of a humanized antibody need not correspond precisely to the parental sequences, e.g., the donor antibody CDR or the consensus framework may be mutagenized by substitution, insertion, and/or deletion of at least one amino acid residue so that the CDR or framework residue at that site does not correspond to either the donor antibody or the consensus framework. In a preferred embodiment, such mutations, however, will not be extensive. Usually, at least 80%, preferably at least 85%, more preferably at least 90%, and most preferably at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (see, e.g., Winnaker, From Genes to Clones (Verlagsgesellschaft, Weinheim, 1987)). A "consensus immunoglobulin sequence" may thus comprise a "consensus framework region(s)" and/or a "consensus CDR(s)". In a family of immunoglobulins, each position in the consensus sequence is occupied by the amino acid occurring most frequently at that position in the family. If two amino acids occur equally frequently, either can be included in the consensus sequence. "Identical" or "identity," as used herein in the context of two or more polypeptide or polynucleotide sequences, can mean that the sequences have a specified percentage of residues that are the same over a specified region. The percentage can be calculated by optimally aligning the two sequences, comparing the two sequences over the specified region, determining the number of positions at which the identical residue occurs in both sequences to yield the number of matched positions, dividing the number of matched positions by the total number of positions in the specified region, and multiplying the result by 100 to yield the percentage of sequence identity. In cases where the two sequences are of different lengths or the alignment produces one or more staggered ends and the specified region of comparison includes only a single sequence, the residues of the single sequence are included in the denominator but not the numerator of the calculation.

"Injury to the head" or "head injury" as used interchangeably herein, refers to any trauma to the scalp, skull, or brain. Such injuries may include only a minor bump on the skull or may be a serious brain injury. Such injuries include primary injuries to the brain and/or secondary injuries to the brain. Primary brain injuries occur during the initial insult and result from displacement of the physical structures of the brain. More specifically, a primary brain injury is the physical damage to parenchyma (tissue, vessels) that occurs during the traumatic event, resulting in shearing and compression of the surrounding brain tissue. Secondary brain injuries occur subsequent to the primary injury and may involve an array of cellular processes. More specifically, a secondary brain injury refers to the changes that evolve over a period of time (from hours to days) after the primary brain injury. It includes an entire cascade of cellular, chemical, tissue, or blood vessel changes in the brain that contribute to further destruction of brain tissue.

An injury to the head can be either closed or open (penetrating). A closed head injury refers to a trauma to the scalp, skull or brain where there is no penetration of the skull by a striking object. An open head injury refers a trauma to the scalp, skull or brain where there is penetration of the skull by a striking object. An injury to the head may be caused by physical shaking of a person, by blunt impact by an external mechanical or other force that results in a closed or open head trauma (e.g., vehicle accident such as with an automobile, plane, train, etc.; blow to the head such as with a baseball bat, or from a firearm), a cerebral vascular accident (e.g., stroke), one or more falls (e.g., as in sports or other activities), explosions or blasts (collectively, "blast injuries") and by other types of blunt force trauma. Alternatively, an injury to the head may be caused by the ingestion and/or exposure to a chemical, toxin or a combination of a chemical and toxin. Examples of such chemicals and/or toxins include fires, molds, asbestos, pesticides and insecticides, organic solvents, paints, glues, gases (such as carbon monoxide, hydrogen sulfide, and cyanide), organic metals (such as methyl mercury, tetraethyl lead and organic tin) and/or one or more drugs of abuse. Alternatively, an injury to the head may be caused as a result of a subject suffering from an autoimmune disease, a metabolic disorder, a brain tumor, one or more viruses, meningitis, hydrocephalus, hypoxia or any combinations thereof. In some cases, it is not possible to be certain whether any such event or injury has occurred or taken place. For example, there may be no history on a patient or subject, the subject may be unable to speak, the subject may not be aware of or have full information on what events they were exposed to, etc. Such circumstances are described herein as the subject "may have sustained an injury to the head." In certain embodiments herein, the closed head injury does not include and specifically excludes a cerebral vascular accident, such as stroke.

"Isolated polynucleotide" as used herein may mean a polynucleotide (e.g. of genomic, cDNA, or synthetic origin, or a combination thereof) that, by virtue of its origin, the isolated polynucleotide is not associated with all or a portion of a polynucleotide with which the "isolated polynucleotide" is found in nature; is operably linked to a polynucleotide that it is not linked to in nature; or does not occur in nature as part of a larger sequence.

"Label" and "detectable label" as used herein refer to a moiety attached to an antibody or an analyte to render the reaction between the antibody and the analyte detectable, and the antibody or analyte so labeled is referred to as "detectably labeled." A label can produce a signal that is detectable by visual or instrumental means. Various labels include signal-producing substances, such as chromagens, fluorescent compounds, chemiluminescent compounds, radioactive compounds, and the like. Representative examples of labels include moieties that produce light, e.g., acridinium compounds, and moieties that produce fluorescence, e.g., fluorescein. Other labels are described herein. In this regard, the moiety, itself, may not be detectable but may become detectable upon reaction with yet another moiety. Use of the term "detectably labeled" is intended to encompass such labeling.

Any suitable detectable label as is known in the art can be used. For example, the detectable label can be a radioactive label (such as $^3H$, $^{14}C$, $^{32}P$, $^{33}P$, $^{35}S$, $^{90}Y$, $^{99}Tc$, $^{111}In$, $^{125}I$, $^{131}I$, $^{177}Lu$, $^{166}Ho$, and $^{153}Sm$), an enzymatic label (such as horseradish peroxidase, alkaline peroxidase, glucose 6-phosphate dehydrogenase, and the like), a chemiluminescent label (such as acridinium esters, thioesters, or sulfonamides; luminol, isoluminol, phenanthridinium esters, and the like), a fluorescent label (such as fluorescein (e.g., 5-fluorescein, 6-carboxyfluorescein, 3'6-carboxyfluorescein, 5(6)-carboxyfluorescein, 6-hexachloro-fluorescein, 6-tetrachlorofluorescein, fluorescein isothiocyanate, and the like)), rhodamine, phycobiliproteins, R-phycoerythrin, quantum dots (e.g., zinc sulfide-capped cadmium selenide), a thermometric label, or an immuno-polymerase chain reaction label. An introduction to labels, labeling procedures and detection of labels is found in Polak and Van Noorden, Introduction to Immunocytochemistry, 2nd ed., Springer Verlag, N.Y. (1997), and in Haugland, Handbook of Fluorescent Probes and Research Chemicals (1996), which is a combined handbook and catalogue published by Molecular Probes, Inc., Eugene, Oregon. A fluorescent label can be used in FPIA (see, e.g., U.S. Patent Nos. 5,593,896, 5,573,904, 5,496,925, 5,359,093, and 5,352,803, which are hereby incorporated by reference in their entireties). An acridinium compound can be used as a detectable label in a homogeneous chemiluminescent assay (see, e.g., Adamczyk et al., Bioorg. Med. Chem. Lett. 16: 1324-1328 (2006); Adamczyk et al., Bioorg. Med. Chem. Lett. 4: 2313-2317 (2004); Adamczyk et al., Biorg. Med. Chem. Lett. 14: 3917-3921 (2004); and Adamczyk et al., Org. Lett. 5: 3779-3782 (2003)).

In one aspect, the acridinium compound is an acridinium-9-carboxamide. Methods for preparing acridinium 9-carboxamides are described in Mattingly, J. Biolumin. Chemilumin. 6: 107-114 (1991); Adamczyk et al., J. Org. Chem. 63: 5636-5639 (1998); Adamczyk et al., Tetrahedron 55: 10899-10914 (1999); Adamczyk et al., Org. Lett. 1: 779-781 (1999); Adamczyk et al., Bioconjugate Chem. 11: 714-724 (2000); Mattingly et al., In Luminescence Biotechnology: Instruments and Applications; Dyke, K. V. Ed.; CRC Press: Boca Raton, pp. 77-105 (2002); Adamczyk et al., Org. Lett. 5: 3779-3782 (2003); and U.S. Patent Nos. 5,468,646, 5,543,524 and 5,783,699 (each of which is incorporated herein by reference in its entirety for its teachings regarding same).

Another example of an acridinium compound is an acridinium-9-carboxylate aryl ester. An example of an acridinium-9-carboxylate aryl ester of formula II is 10-methyl-9-(phenoxycarbonyl)acridinium fluorosulfonate (available from Cayman Chemical, Ann Arbor, MI). Methods for preparing acridinium 9-carboxylate aryl esters are described in McCapra et al., Photochem. Photobiol. 4: 1111-21 (1965); Razavi et al., Luminescence 15: 245-249 (2000); Razavi et al., Luminescence 15: 239-244 (2000); and U.S. Patent No. 5,241,070 (each of which is incorporated herein by reference in its entirety for its teachings regarding same). Such acridinium-9-carboxylate aryl esters are efficient chemiluminescent indicators for hydrogen peroxide produced in the oxidation of an analyte by at least one oxidase in terms of the intensity of the signal and/or the rapidity of the signal. The course of the chemiluminescent emission for the acridinium-9-carboxylate aryl ester is completed rapidly, i.e., in under 1 second, while the acridinium-9-carboxamide chemiluminescent emission extends over 2 seconds. Acridinium-9-carboxylate aryl ester, however, loses its chemiluminescent properties in the presence of protein. Therefore, its use requires the absence of protein during signal generation and detection. Methods for separating or removing proteins in the sample are well-known to those skilled in the art and include, but are not limited to, ultrafiltration, extraction, precipitation, dialysis, chromatography, and/or digestion (see, e.g., Wells, High Throughput Bioanalytical Sample Preparation. Methods and Automation Strategies, Elsevier (2003)). The amount of protein removed or separated from the test sample can be about 40%, about 45%, about 50%, about 55%, about 60%, about 65%, about 70%, about 75%, about 80%, about 85%, about 90%, or about 95%. Further details regarding acridinium-9-carboxylate aryl ester and its use are set forth in U.S. Patent App. No. 11/697,835, filed April 9, 2007. Acridinium-9-carboxylate aryl esters can be dissolved in any suitable solvent, such as degassed anhydrous N,N-dimethylformamide (DMF) or aqueous sodium cholate.

"Limit of Blank (LoB)" as used herein refers to the highest apparent analyte concentration expected to be found when replicates of a blank sample containing no analyte are tested.

"Limit of Detection (LoD)" as used herein refers to the lowest concentration of the measurand (i.e., a quantity intended to be measured) that can be detected at a specified level of confidence. The level of confidence is typically 95%, with a 5% likelihood of a false negative measurement. LoD is the lowest analyte concentration likely to be reliably distinguished from the LoB and at which detection is feasible. LoD can be determined by utilizing both the measured LoB and test replicates of a sample known to contain a low concentration of analyte. The LoD term used herein is based on the definition from Clinical and Laboratory Standards Institute (CLSI) protocol EP17-A2 ("Protocols for Determination of Limits of Detection and Limits of Quantitation; Approved Guideline - Second Edition," EP17A2E, by James F. Pierson-Perry et al., Clinical and Laboratory Standards Institute, June 1, 2012).

"Limit of Quantitation (LoQ)" as used herein refers to the lowest concentration at which the analyte can not only be reliably detected but at which some predefined goals for bias and imprecision are met. The LoQ may be equivalent to the LoD or it could be at a much higher concentration.

"Linearity" refers to how well the method or assay's actual performance across a specified operating range approximates a straight line. Linearity can be measured in terms of a deviation, or non-linearity, from an ideal straight line. "Deviations from linearity" can be expressed in terms of percent of full scale. In some of the methods disclosed herein, less than 10% deviation from linearity (DL) is achieved over the dynamic range of the assay. "Linear" means that there is less than or equal to about 20%, about 19%, about 18%, about 17%, about 16%, about 15%, about 14%, about 13%, about 12%, about 11%, about 10%, about 9%, or about 8% variation for or over an exemplary range or value recited.

"Linking sequence" or "linking peptide sequence" refers to a natural or artificial polypeptide sequence that is connected to one or more polypeptide sequences of interest (e.g., full-length, fragments, etc.). The term "connected" refers to the joining of the linking sequence to the polypeptide sequence of interest. Such polypeptide sequences are preferably joined by one or more peptide bonds. Linking sequences can have a length of from about 4 to about 50 amino acids. Preferably, the length of the linking sequence is from about 6 to about 30 amino acids. Natural linking sequences can be modified by amino acid substitutions, additions, or deletions to create artificial linking sequences. Linking sequences can be used for many purposes, including in recombinant Fabs. Exemplary linking sequences include, but are not limited to: (i) Histidine (His) tags, such as a 6X His tag, which has an amino acid sequence of HHHHHH (SEQ ID NO:2), are useful as linking sequences to facilitate the isolation and purification of polypeptides and antibodies of interest; (ii) Enterokinase cleavage sites, like His tags, are used in the isolation and purification of proteins and antibodies of interest. Often, enterokinase cleavage sites are used together with His tags in the isolation and purification of proteins and antibodies of interest. Various enterokinase cleavage sites are known in the art. Examples of enterokinase cleavage sites include, but are not limited to, the amino acid sequence of DDDDK (SEQ ID NO:3) and derivatives thereof (e.g., ADDDDK (SEQ ID NO:4), etc.); (iii) Miscellaneous sequences can be used to link or connect the light and/or heavy chain variable regions of single chain variable region fragments. Examples of other linking sequences can be found in Bird et al., Science 242: 423-426 (1988); Huston et al., PNAS USA 85: 5879-5883 (1988); and McCafferty et al., Nature 348: 552-554 (1990). Linking sequences also can be modified for additional functions, such as attachment of drugs or attachment to solid supports. In the context of the present disclosure, the monoclonal antibody, for example, can contain a linking sequence, such as a His tag, an enterokinase cleavage site, or both.

"Monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological.

"Magnetic resonance imaging" or "MRI" as used interchangeably herein refers to a medical imaging technique used in radiology to form pictures of the anatomy and the physiological processes of the body in both health and disease. MRI is a form of medical imaging that measures the response of the atomic nuclei of body tissues to high-frequency radio waves when placed in a strong magnetic field, and that produces images of the internal organs. MRI scanners, which is based on the science of nuclear magnetic resonance (NMR), use strong magnetic fields, radio waves, and field gradients to generate images of the inside of the body.

"Multivalent binding protein" is used herein to refer to a binding protein comprising two or more antigen binding sites (also referred to herein as "antigen binding domains"). A multivalent binding protein is preferably engineered to have three or more antigen binding sites, and is generally not a naturally occurring antibody. The term "multispecific binding protein" refers to a binding protein that can bind two or more related or unrelated targets, including a binding protein capable of binding two or more different epitopes of the same target molecule.

"Predetermined cutoff" and "predetermined level" as used herein refer to an assay cutoff value that is used to assess diagnostic, prognostic, or therapeutic efficacy results by comparing the assay results against the predetermined cutoff/level, where the predetermined cutoff/level already has been linked or associated with various clinical parameters (e.g., presence of disease, stage of disease, severity of disease, progression, non-progression, or improvement of disease, etc.). The disclosure provides exemplary predetermined levels. However, it is well-known that cutoff values may vary depending on the nature of the immunoassay (e.g., antibodies employed, reaction conditions, sample purity, etc.). It further is well within the ordinary skill of one in the art to adapt the disclosure herein for other immunoassays to obtain immunoassay-specific cutoff values for those other immunoassays based on the description provided by this disclosure. Whereas the precise value of the predetermined cutoff/level may vary between assays, the correlations as described herein should be generally applicable.

"Point-of-care device" refers to a device used to provide medical diagnostic testing at or near the point-of-care (namely, outside of a laboratory), at the time and place of patient care (such as in a hospital, physician's office, urgent or other medical care facility, a patient's home, a nursing home and/or a long term care and/or hospice facility). Examples of point-of-care devices include those produced by Abbott Laboratories (Abbott Park, IL) (*e.g.,* i-STAT and i-STAT Alinity), Universal Biosensors (Rowville, Australia) (see US 2006/0134713), Axis-Shield PoC AS (Oslo, Norway) and Clinical Lab Products (Los Angeles, USA).

"Quality control reagents" in the context of immunoassays and kits described herein, include, but are not limited to, calibrators, controls, and sensitivity panels. A "calibrator" or "standard" typically is used (e.g., one or more, such as a plurality) in order to establish calibration (standard) curves for interpolation of the concentration of an analyte, such as an antibody or an analyte. Alternatively, a single calibrator, which is near a predetermined positive/negative cutoff, reference level or control level (*e.g.,* "low," "medium," or "high" levels), can be used. Multiple calibrators (i.e., more than one calibrator or a varying amount of calibrator(s)) can be used in conjunction to comprise a "sensitivity panel."

A "receiver operating characteristic" curve or "ROC" curve refers to a graphical plot that illustrates the performance of a binary classifier system as its discrimination threshold is varied. For example, an ROC curve can be a plot of the true positive rate against the false positive rate for the different possible cutoff points of a diagnostic test. It is created by plotting the fraction of true positives out of the positives (TPR = true positive rate) vs. the fraction of false positives out of the negatives (FPR = false positive rate), at various threshold settings. TPR is also known as sensitivity, and FPR is one minus the specificity or true negative rate. The ROC curve demonstrates the tradeoff between sensitivity and specificity (any increase in sensitivity will be accompanied by a decrease in specificity); the closer the curve follows the left-hand border and then the top border of the ROC space, the more accurate the test, the closer the curve comes to the 45-degree diagonal of the ROC space, the less accurate the test, the slope of the tangent line at a cutoff point gives the likelihood ratio (LR) for that value of the test; and the area under the curve is a measure of test accuracy.

"Recombinant antibody" and "recombinant antibodies" refer to antibodies prepared by one or more steps, including cloning nucleic acid sequences encoding all or a part of one or more monoclonal antibodies into an appropriate expression vector by recombinant techniques and subsequently expressing the antibody in an appropriate host cell. The terms include, but are not limited to, recombinantly produced monoclonal antibodies, chimeric antibodies, humanized antibodies (fully or partially humanized), multi-specific or multi-valent structures formed from antibody fragments, bifunctional antibodies, heteroconjugate Abs, DVD-Ig®s, and other antibodies as described in (i) herein. (Dual-variable domain immunoglobulins and methods for making them are described in Wu, C., et al., Nature Biotechnology, 25:1290-1297 (2007)). The term "bifunctional antibody," as used herein, refers to an antibody that comprises a first arm having a specificity for one antigenic site and a second arm having a specificity for a different antigenic site, i.e., the bifunctional antibodies have a dual specificity.

"Reference level" as used herein refers to an assay cutoff value that is used to assess diagnostic, prognostic, or therapeutic efficacy and that has been linked or is associated herein with various clinical parameters (*e.g.,* presence of disease, stage of disease, severity of disease, progression, non-progression, or improvement of disease, etc.) This disclosure provides exemplary reference levels. However, it is well-known that reference levels may vary depending on the nature of the immunoassay (*e.g.*, antibodies employed, reaction conditions, sample purity, etc.) and that assays can be compared and standardized. It further is well within the ordinary skill of one in the art to adapt the disclosure herein for other immunoassays to obtain immunoassay-specific reference levels for those other immunoassays based on the description provided by this disclosure. Whereas the precise value of the reference level may vary between assays, the findings as described herein should be generally applicable and capable of being extrapolated to other assays.

"Risk assessment," "risk classification," "risk identification," or "risk stratification" of subjects (e.g., patients) as used herein refers to the evaluation of factors including biomarkers, to predict the risk of occurrence of future events including disease onset or disease progression, so that treatment decisions regarding the subject may be made on a more informed basis.

"Sample," "test sample," "specimen," "sample from a subject," and "patient sample" as used herein may be used interchangeable and may be a sample of blood, such as whole blood, tissue, urine, serum, plasma, amniotic fluid, cerebrospinal fluid, placental cells or tissue, endothelial cells, leukocytes, or monocytes. The sample can be used directly as obtained from a patient or can be pre-treated, such as by filtration, distillation, extraction, concentration, centrifugation, inactivation of interfering components, addition of reagents, and the like, to modify the character of the sample in some manner as discussed herein or otherwise as is known in the art.

A variety of cell types, tissue, or bodily fluid may be utilized to obtain a sample. Such cell types, tissues, and fluid may include sections of tissues such as biopsy and autopsy samples, frozen sections taken for histologic purposes, blood (such as whole blood), plasma, serum, red blood cells, platelets, interstitial fluid, cerebral spinal fluid, etc. Cell types and tissues may also include lymph fluid, cerebrospinal fluid, a fluid collected by A tissue or cell type may be provided by removing a sample of cells from a human and a non-human animal, but can also be accomplished by using previously isolated cells (e.g., isolated by another person, at another time, and/or for another purpose). Archival tissues, such as those having treatment or outcome history, may also be used. Protein or nucleotide isolation and/or purification may not be necessary.

"Sensitivity" of an assay as used herein refers to the proportion of subjects for whom the outcome is positive that are correctly identified as positive.

"Specificity" of an assay as used herein refers to the proportion of subjects for whom the outcome is negative that are correctly identified as negative.

"Series of calibrating compositions" refers to a plurality of compositions comprising a known concentration of GFAP, wherein each of the compositions differs from the other compositions in the series by the concentration of GFAP.

"Solid phase" or "solid support" as used interchangeably herein, refers to any material that can be used to attach and/or attract and immobilize (1) one or more capture agents or capture specific binding partners, or (2) one or more detection agents or detection specific binding partners. The solid phase can be chosen for its intrinsic ability to attract and immobilize a capture agent. Alternatively, the solid phase can have affixed thereto a linking agent that has the ability to attract and immobilize the (1) capture agent or capture specific binding partner, or (2) detection agent or detection specific binding partner. For example, the linking agent can include a charged substance that is oppositely charged with respect to the capture agent (e.g., capture specific binding partner) or detection agent (e.g., detection specific binding partner) itself or to a charged substance conjugated to the (1) capture agent or capture specific binding partner or (2) detection agent or detection specific binding partner. In general, the linking agent can be any binding partner (preferably specific) that is immobilized on (attached to) the solid phase and that has the ability to immobilize the (1) capture agent or capture specific binding partner, or (2) detection agent or detection specific binding partner through a binding reaction. The linking agent enables the indirect binding of the capture agent to a solid phase material before the performance of the assay or during the performance of the assay. For examples, the solid phase can be plastic, derivatized plastic, magnetic, or non-magnetic metal, glass or silicon, including, for example, a test tube, microtiter well, sheet, bead, microparticle, chip, and other configurations known to those of ordinary skill in the art.

"Specific binding" or "specifically binding" as used herein may refer to the interaction of an antibody, a protein, or a peptide with a second chemical species, wherein the interaction is dependent upon the presence of a particular structure (e.g., an antigenic determinant or epitope) on the chemical species; for example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody.

"Specific binding partner" is a member of a specific binding pair. A specific binding pair comprises two different molecules, which specifically bind to each other through chemical or physical means. Therefore, in addition to antigen and antibody specific binding pairs of common immunoassays, other specific binding pairs can include biotin and avidin (or streptavidin), carbohydrates and lectins, complementary nucleotide sequences, effector and receptor molecules, co-factors and enzymes, enzymes and enzyme inhibitors, and the like. Furthermore, specific binding pairs can include members that are analogs of the original specific binding members, for example, an analyte-analog. Immunoreactive specific binding members include antigens, antigen fragments, and antibodies, including monoclonal and polyclonal antibodies as well as complexes and fragments thereof, whether isolated or recombinantly produced.

"Subject" and "patient" as used herein interchangeably refers to any vertebrate, including, but not limited to, a mammal (e.g., cow, pig, camel, llama, horse, goat, rabbit, sheep, hamsters, guinea pig, cat, dog, rat, and mouse, a non-human primate (for example, a monkey, such as a cynomolgous or rhesus monkey, chimpanzee, etc.) and a human). In some embodiments, the subject may be a human or a non-human. The subject or patient may be undergoing other forms of treatment. In some embodiments, when the subject is a human, the subject does not include any humans who have suffered a cerebrovascular accident (*e.g.,* a stroke). In some embodiments, the subject is suspected to have sustained an injury to the head. In some embodiments, the subject is known to have sustained an injury to the head. In some embodiments, the subject is suspected to be suffering from mild, moderate or severe TBI. In some embodiments, the subject is suspected to be suffering from mild TBI. In some embodiments, the subject is suspected to be suffering from moderate TBI. In some embodiments, the subject is suspected to be suffering from severe TBI.

"Treat," "treating" or "treatment" are each used interchangeably herein to describe reversing, alleviating, or inhibiting the progress of a disease and/or injury, or one or more symptoms of such disease, to which such term applies. Depending on the condition of the subject, the term also refers to preventing a disease, and includes preventing the onset of a disease, or preventing the symptoms associated with a disease. A treatment may be either performed in an acute or chronic way. The term also refers to reducing the severity of a disease or symptoms associated with such disease prior to affliction with the disease. Such prevention or reduction of the severity of a disease prior to affliction refers to administration of a pharmaceutical composition to a subject that is not at the time of administration afflicted with the disease. "Preventing" also refers to preventing the recurrence of a disease or of one or more symptoms associated with such disease. "Treatment" and "therapeutically," refer to the act of treating, as "treating" is defined above.

As used herein the term "single molecule detection" refers to the detection and/or measurement of a single molecule of an analyte in a test sample at very low levels of concentration (such as pg/mL or femtogram/mL levels). A number of different single molecule analyzers or devices are known in the art and include nanopore and nanowell devices. Examples of nanopore devices are described in International Patent Publication No. WO 2016/161402, which is hereby incorporated by reference in its entirety. Examples of nanowell device are described in International Patent Publication No. WO 2016/161400, which is hereby incorporated by reference in its entirety.

"Traumatic Brain Injury" or "TBI" as used interchangeably herein refers to a complex injury with a broad spectrum of symptoms and disabilities. TBI is most often an acute event similar to other injuries. TBI can be classified as "mild," "moderate," or "severe." The causes of TBI are diverse and include, for example, physical shaking by a person, a car accident, injuries from firearms, cerebral vascular accidents (e.g., strokes), falls, explosions or blasts and other types of blunt force trauma. Other causes of TBI include the ingestion and/or exposure to one or more chemicals or toxins (such as fires, molds, asbestos, pesticides and insecticides, organic solvents, paints, glues, gases (such as carbon monoxide, hydrogen sulfide, and cyanide), organic metals (such as methyl mercury, tetraethyl lead and organic tin), one or more drugs of abuse or combinations thereof). Alternatively, TBI can occur in subjects suffering from an autoimmune disease, a metabolic disorder, a brain tumor, hypoxia, one or more viruses, meningitis, hydrocephalus or combinations thereof. Young adults and the elderly are the age groups at highest risk for TBI. In certain embodiments herein, traumatic brain injury or TBI does not include and specifically excludes cerebral vascular accidents such as strokes.

"Mild TBI" as used herein refers to a brain injury where loss of consciousness is brief and usually a few seconds or minutes and/or confusion and disorientation is shorter than 1 hour. Mild TBI is also referred to as a concussion, minor head trauma, minor TBI, minor brain injury, and minor head injury. While MRI and CT scans are often normal, the individual with mild TBI may have cognitive problems such as headache, difficulty thinking, memory problems, attention deficits, mood swings and frustration.

Mild TBI is the most prevalent TBI and is often missed at time of initial injury. Typically, a subject has a Glasgow Coma scale number of between 13-15 (such as 13-15 or 14-15). Fifteen percent (15%) of people with mild TBI have symptoms that last 3 months or more. Mild TBI is defined as the result of the forceful motion of the head or impact causing a brief change in mental status (confusion, disorientation or loss of memory) or loss of consciousness for less than 30 minutes. Common symptoms of mild TBI include fatigue, headaches, visual disturbances, memory loss, poor attention/-concentration, sleep disturbances, dizziness/loss of balance, irritability-emotional disturbances, feelings of depression, and seizures. Other symptoms associated with mild TBI include nausea, loss of smell, sensitivity to light and sounds, mood changes, getting lost or confused, and/or slowness in thinking.

"Moderate TBI" as used herein refers to a brain injury where loss of consciousness and/or confusion and disorientation is between 1 and 24 hours and the subject has a Glasgow Coma scale number of between 9-12. The individual with moderate TBI have abnormal brain imaging results. "Severe TBI" as used herein refers to a brain injury where loss of consciousness is more than 24 hours and memory loss after the injury or penetrating skull injury longer than 24 hours and the subject has a Glasgow Coma scale number between 3-8. The deficits range from impairment of higher level cognitive functions to comatose states. Survivors may have limited function of arms or legs, abnormal speech or language, loss of thinking ability or emotional problems. Individuals with severe injuries can be left in long-term unresponsive states. For many people with severe TBI, long-term rehabilitation is often necessary to maximize function and independence.

Common symptoms of moderate to severe TBI include cognitive deficits including difficulties with attention, concentration, distractibility, memory, speed of processing, confusion, perseveration, impulsiveness, language processing, and/or "executive functions", not understanding the spoken word (receptive aphasia), difficulty speaking and being understood (expressive aphasia), slurred speech, speaking very fast or very slow, problems reading, problems writing, difficulties with interpretation of touch, temperature, movement, limb position and fine, discrimination, the integration or patterning of sensory impressions into psychologically meaningful data, partial or total loss of vision, weakness of eye muscles and double vision (diplopia), blurred vision, problems judging distance, involuntary eye movements (nystagmus), intolerance of light (photophobia), hearing, such as decrease or loss of hearing, ringing in the ears (tinnitus), increased sensitivity to sounds, loss or diminished sense of smell (anosmia), loss or diminished sense of taste, the convulsions associated with epilepsy that can be several types and can involve disruption in consciousness, sensory perception, or motor movements, control of bowel and bladder, sleep disorders, loss of stamina, appetite changes, regulation of body temperature, menstrual difficulties, dependent behaviors, emotional ability, lack of motivation, irritability, aggression, depression, disinhibition, or denial/lack of awareness.

"Variant" is used herein to describe a peptide or polypeptide that differs in amino acid sequence by the insertion, deletion, or conservative substitution of amino acids, but retain at least one biological activity. Representative examples of "biological activity" include the ability to be bound by a specific antibody or to promote an immune response. Variant is also used herein to describe a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree, and distribution of charged regions) is recognized in the art as typically involving a minor change. These minor changes can be identified, in part, by considering the hydropathic index of amino acids, as understood in the art. Kyte et al., J. Mol. Biol. 157:105-132 (1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of $\pm 2$ are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity. U.S. Patent No. 4,554,101, incorporated fully herein by reference. Substitution of amino acids having similar hydrophilicity values can result in peptides retaining biological activity, for example immunogenicity, as is understood in the art. Substitutions may be performed with amino acids having hydrophilicity values within $\pm 2$ of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties. "Variant" also can be used to refer to an antigenically reactive fragment of an anti-GFAP antibody that differs from the corresponding fragment of anti-GFAP antibody in amino acid sequence but is still antigenically reactive and can compete with the corresponding fragment of anti-GFAP antibody for binding with GFAP. "Variant" also can be used to describe a polypeptide or a fragment thereof that has been differentially processed, such as by proteolysis, phosphorylation, or other post-translational modification, yet retains its antigen reactivity.

"Vector" is used herein to describe a nucleic acid molecule that can transport another nucleic acid to which it has been linked. One type of vector is a "plasmid", which refers to a circular double-stranded DNA loop into which additional DNA segments may be ligated. Another type of vector is a viral vector, wherein additional DNA segments may be ligated into the viral genome. Certain vectors can replicate autonomously in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. "Plasmid" and "vector" may be used interchangeably as the plasmid is the most commonly used form of vector. However, other forms of expression vectors, such as viral vectors (e.g., replication defective retroviruses, adenoviruses and adeno-associated viruses), which serve equivalent functions, can be used. In this regard, RNA versions of vectors (including RNA viral vectors) may also find use in the context of the present disclosure.

Unless otherwise defined herein, scientific and technical terms used in connection with the present disclosure shall have the meanings that are commonly understood by those of ordinary skill in the art. For example, any nomenclatures used in connection with,

and techniques of, cell and tissue culture, molecular biology, immunology, microbiology, genetics and protein and nucleic acid chemistry and hybridization described herein are those that are well known and commonly used in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

2. Methods Based on Glial Fibrillary Acidic Protein (GFAP) Status

The present disclosure relates to methods of assessing Glial fibrillary acidic protein (GFAP) status using an improved immunoassay to rapidly aid in the diagnosis of a traumatic brain injury (TBI), monitor progression and predict outcome in subjects in need thereof (including those subjects receiving treatment for TBI as well as those subjects not receiving treatment for TBI). The method is performed using a first specific binding member and the second specific binding member that each specifically bind to GFAP and form first complexes that includes the first specific binding member-GFAP-second specific binding member. In some embodiments, the second specific binding member is labeled with a detectable label. In some embodiments, the immunoassay is performed in a point-of-care device. An example of a point-of-care device that can be used is i-STAT® (Abbott, Laboratories, Abbott Park, IL).

The improved immunoassay can be used to determine GFAP at both low as well as higher levels of GFAP, thus providing a means to determine GFAP amount in a sample over an expanded or wider range of concentrations without the need for dilution or concentration of the biological sample. The immunoassay provides a more versatile and sensitive assay for assessing traumatic brain injury. The disclosed immunoassay provides sensitive serum detection of GFAP and a standardized assay that can be used to assess TBI, such as mild TBI. The immunoassay allows for the measure of up to 50,000 pg/mL of GFAP in a biological sample and does not require dilution of the biological sample. The immunoassay also allows for the measure of less than or equal to 50,000 pg/mL of GFAP in a biological sample and does not require dilution of the biological sample. The immunoassay also maintains assay linearity over the dynamic range of the assay. In some embodiments, the immunoassay has a dynamic range of 5 log. In other embodiments, the immunoassay has a dynamic range of 5 log and maintains assay linearity over the dynamic range. In still other embodiments, the low and higher levels of GFAP that can be assessed in a biological sample are within the dynamic range of the assay without the need to dilute or concentrate the biological sample. In still other embodiments, the immunoassay is capable of measuring an amount of GFAP that is less than or equal to 5 pg/mL in a volume of less than 20 microliters of test sample. In still other embodiments, the immunoassay: (1) is capable of measuring an amount of GFAP that is less than or equal to 50,000 pg/mL in a volume of less than 20 microliters of test sample; (2) has a dynamic range of 5 log; and (3) is linear over the dynamic range.

The increased range of concentrations of GFAP that can be measured with the disclosed immunoassay provides a more accurate and sensitive assay for aiding in the diagnosing of and distinguishing traumatic brain injury in a patient. Thus, the disclosed immunoassay may be used to measure or assess increased or decreased GFAP concentrations at low levels of GFAP in a diluted or undiluted sample compared to a control or calibrator sample and thus be used to identify TBI in a patient. The use of the GFAP immunoassay may provide an accurate aid in the diagnosis of and subsequent treatment of patients with traumatic brain injury. In addition, the disclosed immunoassay provides an expanded window of detection.

In some embodiments, ranges over which GFAP can be determined have at least about 5%, about 10%, about 25%, about 50%, about 75%, about 100%, about 110%, about 120%, about 130%, about 140%, about 150%, about 160%, about 170%, about 180%, about 190%, about 200%, about 210%, about 220%, about 230%, about 240%, about 250%, about 260%, about 270%, about 280%, about 290%, about 300%, about 400%, or about 500% improved range size compared to other commercially available GFAP immunoassays.

In some embodiments, ranges of about 0 pg/mL to about 150,000 pg/mL, about 0.0001 pg/mL to about 150,000 pg/mL, about 0.001 pg/mL to about 150,000 pg/mL, about 0.01 pg/mL to about 150,000 pg/mL, about 0.1 pg/mL to about 150,000 pg/mL, about 0.5 pg/mL to about 150,000 pg/mL, about 1.0 pg/mL to about 150,000 pg/mL, about 2.0 pg/mL to about 150,000 pg/mL, about 3.0 pg/mL to about 150,000 pg/mL, about 4.0 pg/mL to about 150,000 pg/mL, about 5.0 pg/mL to about 150,000 pg/mL, about 6.0 pg/mL to about 150,000 pg/mL, about 7.0 pg/mL to about 150,000 pg/mL, about 8.0 pg/mL to about 150,000 pg/mL, about 9.0 pg/mL to about 150,000 pg/mL, about 10.0 pg/mL to about 150,000 pg/mL, about 20 pg/mL to about 150,000 pg/mL, about 25 pg/mL to about 150,000 pg/mL, about 30 pg/mL to about 150,000 pg/mL, about 40 pg/mL to about 150,000 pg/mL, about 50 pg/mL to about 150,000 pg/mL, about 60 pg/mL to about 150,000 pg/mL, about 70 pg/mL to about 150,000 pg/mL, about 75 pg/mL to about 150,000 pg/mL, about 80 pg/mL to about 150,000 pg/mL, about 90 pg/mL to about 150,000 pg/mL, about 100 pg/mL to about 150,000 pg/mL, about 110 pg/mL to about 150,000 pg/mL, about 120 pg/mL to about 150,000 pg/mL, about 125 pg/mL to about 150,000 pg/mL, about 130 pg/mL to about 150,000 pg/mL, about 140 pg/mL to about 150,000 pg/mL, about 150 pg/mL to about 150,000 pg/mL, about 0.0001 pg/mL to about 140,000 pg/mL, about 0.001 pg/mL to about 140,000 pg/mL, about 0.01 pg/mL to about 140,000 pg/mL, about 0.1 pg/mL to about 140,000 pg/mL, about 0.5 pg/mL to about 140,000 pg/mL, about 1.0 pg/mL to about 140,000 pg/mL, about 2.0 pg/mL to about 140,000 pg/mL, about 3.0 pg/mL to about 140,000 pg/mL, about 4.0 pg/mL to about 140,000 pg/mL, about 5.0 pg/mL to about 140,000 pg/mL, about 6.0 pg/mL to about 140,000 pg/mL, about 7.0 pg/mL to about 140,000 pg/mL, about 8.0 pg/mL to about 140,000 pg/mL, about 9.0 pg/mL to about 140,000 pg/mL, about 10.0 pg/mL to about 140,000 pg/mL, about 20 pg/mL to about 140,000 pg/mL, about 25 pg/mL to about 140,000 pg/mL, about 30 pg/mL to about 140,000 pg/mL, about 40 pg/mL to about 140,000 pg/mL, about 50 pg/mL to about 140,000 pg/mL, about 60 pg/mL to about 140,000 pg/mL, about 70 pg/mL to about 140,000 pg/mL, about 75 pg/mL to about 140,000 pg/mL, about 80 pg/mL to about 140,000 pg/mL, about 90 pg/mL to about 140,000 pg/mL, about 100 pg/mL to about 140,000 pg/mL, about 110 pg/mL to about 140,000 pg/mL, about 120 pg/mL to about 140,000 pg/mL, about 125 pg/mL to about 140,000 pg/mL, about 130 pg/mL to about 140,000 pg/mL, about 140 pg/mL to about 140,000 pg/mL, about 150 pg/mL to about 140,000 pg/mL, about 0.0001 pg/mL to about 130,000 pg/mL, about 0.001 pg/mL to about 130,000 pg/mL, about 0.01 pg/mL to about 130,000 pg/mL, about 0.1 pg/mL to about 130,000 pg/mL, about 0.5 pg/mL to about 130,000 pg/mL, about 1.0 pg/mL to about 130,000 pg/mL, about 2.0 pg/mL to about 130,000 pg/mL, about 3.0 pg/mL to about 130,000 pg/mL, about 4.0 pg/mL to about 130,000 pg/mL, about 5.0 pg/mL to about 130,000 pg/mL, about 6.0 pg/mL to about 130,000 pg/mL, about 7.0 pg/mL to about 130,000 pg/mL, about 8.0 pg/mL to about 130,000 pg/mL, about 9.0 pg/mL to about 130,000 pg/mL, about 10.0 pg/mL to about 130,000 pg/mL, about 20 pg/mL to about 130,000 pg/mL, about 25 pg/mL to about 130,000 pg/mL, about 30 pg/mL to about 130,000 pg/mL, about 40 pg/mL to about 130,000 pg/mL, about 50 pg/mL to about 130,000 pg/mL, about 60 pg/mL to about 130,000 pg/mL, about 70 pg/mL to about 130,000 pg/mL, about 75 pg/mL to about 130,000 pg/mL, about 80 pg/mL to about 130,000 pg/mL, about 90 pg/mL to about 130,000 pg/mL, about 100 pg/mL to about 130,000 pg/mL, about 110 pg/mL to about 130,000 pg/mL, about 120 pg/mL to about 130,000 pg/mL, about 125 pg/mL to about 130,000 pg/mL, about 130 pg/mL to about 130,000 pg/mL, about 140 pg/mL to about 130,000 pg/mL, about 150 pg/mL to about 130,000 pg/mL, about 0.0001 pg/mL to about 125,000 pg/mL, about 0.001 pg/mL to about 125,000 pg/mL, about 0.01 pg/mL to about 125,000 pg/mL, about 0.1 pg/mL to about 125,000 pg/mL, about 0.5 pg/mL to about 125,000 pg/mL, about 1.0 pg/mL to about 125,000 pg/mL, about 2.0 pg/mL to about 125,000 pg/mL, about 3.0 pg/mL to about 125,000 pg/mL, about 4.0 pg/mL to about 125,000 pg/mL, about 5.0 pg/mL to about 125,000 pg/mL, about 6.0 pg/mL to about 125,000 pg/mL, about 7.0 pg/mL to about 125,000 pg/mL, about 8.0 pg/mL to about 125,000 pg/mL, about 9.0 pg/mL to about 125,000 pg/mL, about 10.0 pg/mL to about 125,000 pg/mL, about 20 pg/mL to about 125,000 pg/mL, about 25 pg/mL to about 125,000 pg/mL, about 30 pg/mL to about 125,000 pg/mL, about 40 pg/mL to about 125,000 pg/mL, about 50 pg/mL to about 125,000 pg/mL, about 60 pg/mL to about 125,000 pg/mL, about 70 pg/mL to about 125,000 pg/mL, about 75 pg/mL to about 125,000 pg/mL, about 80 pg/mL to about 125,000 pg/mL, about 90 pg/mL to about 125,000 pg/mL, about 100 pg/mL to about 125,000 pg/mL, about 110 pg/mL to about 125,000 pg/mL, about 120 pg/mL to about 125,000 pg/mL, about 125 pg/mL to about 125,000 pg/mL, about 130 pg/mL to about 125,000 pg/mL, about 140 pg/mL to about 125,000 pg/mL, about 150 pg/mL to about 125,000 pg/mL, about 0.0001 pg/mL to about 120,000 pg/mL, about 0.001 pg/mL to about 120,000 pg/mL, about 0.01 pg/mL to about 120,000 pg/mL, about 0.1 pg/mL to about 120,000 pg/mL, about 0.5 pg/mL to about 120,000 pg/mL, about 1.0 pg/mL to about 120,000 pg/mL, about 2.0 pg/mL to about 120,000 pg/mL, about 3.0 pg/mL to about 120,000 pg/mL, about 4.0 pg/mL to about 120,000 pg/mL, about 5.0 pg/mL to about 120,000 pg/mL, about 6.0 pg/mL to about 120,000 pg/mL, about 7.0 pg/mL to about 120,000 pg/mL, about 8.0 pg/mL to about 120,000 pg/mL, about 9.0 pg/mL to about 120,000 pg/mL, about 10.0 pg/mL to about 120,000 pg/mL, about 20 pg/mL to about 120,000 pg/mL, about 25 pg/mL to about 120,000 pg/mL, about 30 pg/mL to about 120,000 pg/mL, about 40 pg/mL to about 120,000 pg/mL, about 50 pg/mL to about 120,000 pg/mL, about 60 pg/mL to about 120,000 pg/mL, about 70 pg/mL to about 120,000 pg/mL, about 75 pg/mL to about 120,000 pg/mL, about 80 pg/mL to about 120,000 pg/mL, about 90 pg/mL to about 120,000 pg/mL, about 100 pg/mL to about 120,000 pg/mL, about 110 pg/mL to about 120,000 pg/mL, about 120 pg/mL to about 120,000 pg/mL, about 125 pg/mL to about 120,000 pg/mL, about 130 pg/mL to about 120,000 pg/mL, about 140 pg/mL to about 120,000 pg/mL, about 150 pg/mL to about 120,000 pg/mL, about 0.0001 pg/mL to about 110,000 pg/mL, about 0.001 pg/mL to about 110,000 pg/mL, about 0.01 pg/mL to about 110,000 pg/mL, about 0.1 pg/mL to about 110,000 pg/mL, about 0.5 pg/mL to about 110,000 pg/mL, about 1.0 pg/mL to about 110,000 pg/mL, about 2.0 pg/mL to about 110,000 pg/mL, about 3.0 pg/mL to about 110,000 pg/mL, about 4.0 pg/mL to about 110,000 pg/mL, about 5.0 pg/mL to about 110,000 pg/mL, about 6.0 pg/mL to about 110,000 pg/mL, about 7.0 pg/mL to about 110,000 pg/mL, about 8.0 pg/mL to about 110,000 pg/mL, about 9.0 pg/mL to about 110,000 pg/mL, about 10.0 pg/mL to about 110,000 pg/mL, about 20 pg/mL to about 110,000 pg/mL, about 25 pg/mL to about 110,000 pg/mL, about 30 pg/mL to about 110,000 pg/mL, about 40 pg/mL to about 110,000 pg/mL, about 50 pg/mL to about 110,000 pg/mL, about 60 pg/mL to about 110,000 pg/mL, about 70 pg/mL to about 110,000 pg/mL, about 75 pg/mL to about 110,000 pg/mL, about 80 pg/mL to about 110,000 pg/mL, about 90 pg/mL to about 110,000 pg/mL, about 100 pg/mL to about 110,000 pg/mL, about 110 pg/mL to about 110,000 pg/mL, about 120 pg/mL to about 110,000 pg/mL, about 125 pg/mL to about 110,000 pg/mL, about 130 pg/mL to about 110,000 pg/mL, about 140 pg/mL to about 110,000 pg/mL, about 150 pg/mL to about 110,000 pg/mL, about 0.0001 pg/mL to about 100,000 pg/mL, about 0.001 pg/mL to about 100,000 pg/mL, about 0.01 pg/mL to about 100,000 pg/mL, about 0.1 pg/mL to about 100,000 pg/mL, about 0.5 pg/mL to about 100,000 pg/mL, about 1.0 pg/mL to about 100,000 pg/mL, about 2.0 pg/mL to about 100,000 pg/mL, about 3.0 pg/mL to about 100,000 pg/mL, about 4.0 pg/mL to about 100,000 pg/mL, about 5.0 pg/mL to about 100,000 pg/mL, about 6.0 pg/mL to about 100,000 pg/mL, about 7.0 pg/mL to about 100,000 pg/mL, about 8.0 pg/mL to about 100,000 pg/mL, about 9.0 pg/mL to about 100,000 pg/mL, about 10.0 pg/mL to about 100,000 pg/mL, about 20 pg/mL to about 100,000 pg/mL, about 25 pg/mL to about 100,000 pg/mL, about 30 pg/mL to about 100,000 pg/mL, about 40 pg/mL to about 100,000 pg/mL, about 50 pg/mL to about 100,000 pg/mL, about 60 pg/mL to about 100,000 pg/mL, about 70 pg/mL to about 100,000 pg/mL, about 75 pg/mL to about 100,000 pg/mL, about 80 pg/mL to about 100,000 pg/mL, about 90 pg/mL to about 100,000 pg/mL, about 100 pg/mL to about 100,000 pg/mL, about 110 pg/mL to about 100,000 pg/mL, about 120 pg/mL to about 100,000 pg/mL, about 125 pg/mL to about 100,000 pg/mL, about 130 pg/mL to about 100,000 pg/mL, about 140 pg/mL to about 100,000 pg/mL, about 150 pg/mL to about 100,000 pg/mL, about 0.0001 pg/mL to about 90,000 pg/mL, about 0.001 pg/mL to about 90,000 pg/mL, about 0.01 pg/mL to about 90,000 pg/mL, about 0.1 pg/mL to about 90,000 pg/mL, about 0.5 pg/mL to about 90,000 pg/mL, about 1.0 pg/mL to about 90,000 pg/mL, about 2.0 pg/mL to about 90,000 pg/mL, about 3.0 pg/mL to about 90,000 pg/mL, about 4.0 pg/mL to about 90,000 pg/mL, about 5.0 pg/mL to about 90,000 pg/mL, about 6.0 pg/mL to about 90,000 pg/mL, about 7.0 pg/mL to about 90,000 pg/mL, about 8.0 pg/mL to about 90,000 pg/mL, about 9.0 pg/mL to about 90,000 pg/mL, about 10.0 pg/mL to about 90,000 pg/mL, about 20 pg/mL to about 90,000 pg/mL, about 25 pg/mL to about 90,000 pg/mL, about 30 pg/mL to about 90,000 pg/mL, about 40 pg/mL to about 90,000 pg/mL, about 50 pg/mL to about 90,000 pg/mL, about 60 pg/mL to about 90,000 pg/mL, about 70 pg/mL to about 90,000 pg/mL, about 75 pg/mL to about 90,000 pg/mL, about 80 pg/mL to about 90,000 pg/mL, about 90 pg/mL to about 90,000 pg/mL, about 100 pg/mL to about 90,000 pg/mL, about 110 pg/mL to about 90,000 pg/mL, about 120 pg/mL to about 90,000 pg/mL, about 125 pg/mL to about 90,000 pg/mL, about 130 pg/mL to about 90,000 pg/mL, about 140 pg/mL to about 90,000 pg/mL, about 150 pg/mL to about 90,000 pg/mL, about 0.0001 pg/mL to about 80,000 pg/mL, about 0.001 pg/mL to about 80,000 pg/mL, about 0.01 pg/mL to about 80,000 pg/mL, about 0.1 pg/mL to about 80,000 pg/mL, about 0.5 pg/mL to about 80,000 pg/mL, about 1.0 pg/mL to about 80,000 pg/mL, about 2.0 pg/mL to about 80,000 pg/mL, about 3.0 pg/mL to about 80,000 pg/mL, about 4.0 pg/mL to about 80,000 pg/mL, about 5.0 pg/mL to about 80,000 pg/mL, about 6.0 pg/mL to about 80,000 pg/mL, about 7.0 pg/mL to about 80,000 pg/mL, about 8.0 pg/mL to about 80,000 pg/mL, about 9.0 pg/mL to about 80,000 pg/mL, about 10.0 pg/mL to about 80,000 pg/mL, about 20 pg/mL to about 80,000 pg/mL, about 25 pg/mL to about 80,000 pg/mL, about 30 pg/mL to about 80,000 pg/mL, about 40 pg/mL to about 80,000 pg/mL, about 50 pg/mL to about 80,000 pg/mL, about 60 pg/mL to about 80,000 pg/mL, about 70 pg/mL to about 80,000 pg/mL, about 75 pg/mL to about 80,000 pg/mL, about 80 pg/mL to about 80,000 pg/mL, about 90 pg/mL to about 80,000 pg/mL, about 100 pg/mL to about 80,000 pg/mL, about 110 pg/mL to about 80,000 pg/mL, about 120 pg/mL to about 80,000 pg/mL, about 125 pg/mL to about 80,000 pg/mL, about 130 pg/mL to about 80,000 pg/mL, about 140 pg/mL to about 80,000 pg/mL, about 150 pg/mL to about 80,000 pg/mL, about 0.0001 pg/mL to about 75,000 pg/mL, about 0.001 pg/mL to about 75,000 pg/mL, about 0.01 pg/mL to about 75,000 pg/mL, about 0.1 pg/mL to about 75,000 pg/mL, about 0.5 pg/mL to about 75,000 pg/mL, about 1.0 pg/mL to about 75,000 pg/mL, about 2.0 pg/mL to about 75,000 pg/mL, about 3.0 pg/mL to about 75,000 pg/mL, about 4.0 pg/mL to about 75,000 pg/mL, about 5.0 pg/mL to about 75,000 pg/mL, about 6.0 pg/mL to about 75,000 pg/mL, about 7.0 pg/mL to about 75,000 pg/mL, about 8.0 pg/mL to about 75,000 pg/mL, about 9.0 pg/mL to about 75,000 pg/mL, about 10.0 pg/mL to about 75,000 pg/mL, about 20 pg/mL to about 75,000 pg/mL, about 25 pg/mL to about 75,000 pg/mL, about 30 pg/mL to about 75,000 pg/mL, about 40 pg/mL to about 75,000 pg/mL, about 50 pg/mL to about 75,000 pg/mL, about 60 pg/mL to about 75,000 pg/mL, about 70 pg/mL to about 75,000 pg/mL, about 75 pg/mL to about 75,000 pg/mL, about 80 pg/mL to about 75,000 pg/mL, about 90 pg/mL to about 75,000 pg/mL, about 100 pg/mL to about 75,000 pg/mL, about 110 pg/mL to about 75,000 pg/mL, about 120 pg/mL to about 75,000 pg/mL, about 125 pg/mL to about 75,000 pg/mL, about 130 pg/mL to about 75,000 pg/mL, about 140 pg/mL to about 75,000 pg/mL, about 150 pg/mL to about 75,000 pg/mL, about 0.0001 pg/mL to about 70,000 pg/mL, about 0.001 pg/mL to about 70,000 pg/mL, about 0.01 pg/mL to about 70,000 pg/mL, about 0.1 pg/mL to about 70,000 pg/mL, about 0.5 pg/mL to about 70,000 pg/mL, about 1.0 pg/mL to about 70,000 pg/mL, about 2.0 pg/mL to about 70,000 pg/mL, about 3.0 pg/mL to about 70,000 pg/mL, about 4.0 pg/mL to about 70,000 pg/mL, about 5.0 pg/mL to about 70,000 pg/mL, about 6.0 pg/mL to about 70,000 pg/mL, about 7.0 pg/mL to about 70,000 pg/mL, about 8.0 pg/mL to about 70,000 pg/mL, about 9.0 pg/mL to about 70,000 pg/mL, about 10.0 pg/mL to about 70,000 pg/mL, about 20 pg/mL to about 70,000 pg/mL, about 25 pg/mL to about 70,000 pg/mL, about 30 pg/mL to about 70,000 pg/mL, about 40 pg/mL to about 70,000 pg/mL, about 50 pg/mL to about 70,000 pg/mL, about 60 pg/mL to about 70,000 pg/mL, about 70 pg/mL to about 70,000 pg/mL, about 75 pg/mL to about 70,000 pg/mL, about 80 pg/mL to about 70,000 pg/mL, about 90 pg/mL to about 70,000 pg/mL, about 100 pg/mL to about 70,000 pg/mL, about 110 pg/mL to about 70,000 pg/mL, about 120 pg/mL to about 70,000 pg/mL, about 125 pg/mL to about 70,000 pg/mL, about 130 pg/mL to about 70,000 pg/mL, about 140 pg/mL to about 70,000 pg/mL, about 150 pg/mL to about 70,000 pg/mL, about 0.0001 pg/mL to about 60,000 pg/mL, about 0.001 pg/mL to about 60,000 pg/mL, about 0.01 pg/mL to about 60,000 pg/mL, about 0.1 pg/mL to about 60,000 pg/mL, about 0.5 pg/mL to about 60,000 pg/mL, about 1.0 pg/mL to about 60,000 pg/mL, about 2.0 pg/mL to about 60,000 pg/mL, about 3.0 pg/mL to about 60,000 pg/mL, about 4.0 pg/mL to about 60,000 pg/mL, about 5.0 pg/mL to about 60,000 pg/mL, about 6.0 pg/mL to about 60,000 pg/mL, about 7.0 pg/mL to about 60,000 pg/mL, about 8.0 pg/mL to about 60,000 pg/mL, about 9.0 pg/mL to about 60,000 pg/mL, about 10.0 pg/mL to about 60,000 pg/mL, about 20 pg/mL to about 60,000 pg/mL, about 25 pg/mL to about 60,000 pg/mL, about 30 pg/mL to about 60,000 pg/mL, about 40 pg/mL to about 60,000 pg/mL, about 50 pg/mL to about 60,000 pg/mL, about 60 pg/mL to about 60,000 pg/mL, about 70 pg/mL to about 60,000 pg/mL, about 75 pg/mL to about 60,000 pg/mL, about 80 pg/mL to about 60,000 pg/mL, about 90 pg/mL to about 60,000 pg/mL, about 100 pg/mL to about 60,000 pg/mL, about 110 pg/mL to about 60,000 pg/mL, about 120 pg/mL to about 60,000 pg/mL, about 125 pg/mL to about 60,000 pg/mL, about 130 pg/mL to about 60,000 pg/mL, about 140 pg/mL to about 60,000 pg/mL, about 150 pg/mL to about 60,000 pg/mL, about 0.0001 pg/mL to about 50,000 pg/mL, about 0.001 pg/mL to about 50,000 pg/mL, about 0.01 pg/mL to about 50,000 pg/mL, about 0.1 pg/mL to about 50,000 pg/mL, about 0.5 pg/mL to about 50,000 pg/mL, about 1.0 pg/mL to about 50,000 pg/mL, about 2.0 pg/mL to about 50,000 pg/mL, about 3.0 pg/mL to about 50,000 pg/mL, about 4.0 pg/mL to about 50,000 pg/mL, about 5.0 pg/mL to about 50,000 pg/mL, about 6.0 pg/mL to about 50,000 pg/mL, about 7.0 pg/mL to about 50,000 pg/mL, about 8.0 pg/mL to about 50,000 pg/mL, about 9.0 pg/mL to about 50,000 pg/mL, about 10.0 pg/mL to about 50,000 pg/mL, about 20 pg/mL to about 50,000 pg/mL, about 25 pg/mL to about 50,000 pg/mL, about 30 pg/mL to about 50,000 pg/mL, about 40 pg/mL to about 50,000 pg/mL, about 50 pg/mL to about 50,000 pg/mL, about 60 pg/mL to about 50,000 pg/mL, about 70 pg/mL to about 50,000 pg/mL, about 75 pg/mL to about 50,000 pg/mL, about 80 pg/mL to about 50,000 pg/mL, about 90 pg/mL to about 50,000 pg/mL, about 100 pg/mL to about 50,000 pg/mL, about 110 pg/mL to about 50,000 pg/mL, about 120 pg/mL to about 50,000 pg/mL, about 125 pg/mL to about 50,000 pg/mL, about 130 pg/mL to about 50,000 pg/mL, about 140 pg/mL to about 50,000 pg/mL, or about 150 pg/mL to about 50,000 pg/mL of GFAP may be determined, measured or assessed.

In some embodiments, a range selected from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL of GFAP may be determined, measured or assessed.

In some embodiments, a range of about 5 pg/mL to about 50,000 pg/mL may be determined, measured or assessed.

In some embodiments, a range of about 10 pg/mL to about 50,000 pg/mL may be determined, measured or assessed.

In some embodiments, a range of about 12 pg/mL to about 50,000 pg/mL may be determined, measured or assessed.

In some embodiments, a range of about 20 pg/mL to about 50,000 pg/mL may be determined, measured or assessed.

Some instruments (such as, for example the Abbott Laboratories instrument ARCHITECT®, and other core laboratory instruments) other than a point-of-care device may be capable of measuring levels of GFAP in a biological sample higher or greater than 50,000 pg/mL. Thus in some embodiments, the concentration of GFAP that can be measured according to the methods of the present disclosure may be greater than 50,000 pg/mL. Use of the methods as described herein may provide one or more of the benefits described herein on those devices (e.g., measure up to 50,000 pg/mL, dynamic range of 5 log, assay linearity over the dynamic range, measure of GFAP in a volume less than 20 microliters of sample, expanded window of detection, etc.).

Other methods of detection include the use of or can be adapted for use on a nanopore device or nanowell device, e.g. for single molecule detection. Examples of nanopore devices are described in International Patent Publication No. WO 2016/161402, which is hereby incorporated by reference in its entirety. Examples of nanowell device are described in International Patent Publication No. WO 2016/161400, which is hereby incorporated by reference in its entirety. Other devices and methods appropriate for single molecule detection also can be employed.

## a. Methods of Assessing GFAP Status as a Measure of Traumatic Brain Injury

In an embodiment, the methods described herein can be used to assess a subject's glial fibrillary acid protein (GFAP) status as a measure of traumatic brain injury. The method includes the steps of: a) contacting a biological sample with a first specific binding member and a second specific binding member, wherein the first specific binding member and the second specific binding member each specifically bind to GFAP thereby producing one or more first complexes comprising first specific binding member-GFAP-second specific binding member, wherein the second specific binding member comprises a detectable label; and b) assessing a signal from the one or more first complexes, wherein the presence of a detectable signal from the detectable label indicates that GFAP is present in the sample and the amount of detectable signal from the detectable label indicates the amount of GFAP present in the sample, such that the presence and/or amount of detectable signal from the detectable label can be employed to assess said subject's GFAP status as a measure of traumatic brain injury. The method (i) can be used to determine levels of up to 50,000 pg/mL of GFAP, (ii) does not require dilution of the biological sample, and (iii) is conducted using a point-of-care device. An example of a point-of-care device that can be used is i-STAT® (Abbott, Laboratories, Abbott Park, IL).

In an embodiment, the methods described herein can be used to assess a subject's glial fibrillary acid protein (GFAP) status as a measure of traumatic brain injury in a biological sample obtained from a human subject. Said subject may have sustained an injury to the head or is known to have sustained an injury to the head. The method comprises the steps of: (a) contacting a biological sample obtained from a human subject, either simultaneously or sequentially, in any order, with: (1) a capture antibody which is immobilized on a solid support and which binds to an epitope on human GFAP to form a capture antibody-GFAP antigen complex, and (2) a detection antibody which includes a detectable label and which binds to an epitope on human GFAP that is not bound by the capture antibody, to form a GFAP antigen-detection antibody complex, such that a capture antibody-GFAP antigen-detection antibody complex is formed, wherein the capture antibody and detection antibody are monoclonal antibodies, (b) determining the level of GFAP in the biological sample based on the signal generated by the detectable label in the capture antibody-GFAP antigen-detection antibody complex. The method is capable of quantitating the level of GFAP across a dynamic range from 5 pg/mL to 50,000 pg/mL with a precision of less than 10% CV and with less than 10% deviation from linearity (DL) achieved over the dynamic range.

In an embodiment, the methods described herein can be used to measure glial fibrillary acid protein (GFAP) status as a measure of traumatic brain injury in a subject that may have sustained an injury to the head or is known to have sustained an injury to the head. The method comprises the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first specific binding member-GFAP-second specific binding member, wherein the second specific binding member comprises a detectable label, wherein the first specific binding member is immobilized on a solid support; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, such that the amount of detectable signal from the detectable label can be employed to assess said subject's GFAP status as a measure of traumatic brain injury. Said assay is capable of determining the level of GFAP across a dynamic range from 20 pg/mL to 50,000 pg/mL with a precision of less than 10% CV and with less than 10% deviation from linearity (DL) achieved over the dynamic range in a volume of less than 20 microliters of test sample.

In some embodiments, the method can be used to determine levels of GFAP from about 10.0 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 100 pg/mL to about 50,000 pg/mL, from about 125 pg/mL to about 50,000 pg/mL, or from about 150 pg/mL to about 50,000 pg/mL. In some embodiments, the method can be used to determine levels of GFAP from about 10.0 pg/mL to about 150,000 pg/mL, from about 20 pg/mL to about 150,000 pg/mL, from about 25 pg/mL to about 150,000 pg/mL, from about 30 pg/mL to about 150,000 pg/mL, from about 40 pg/mL to about 150,000 pg/mL, from about 50 pg/mL to about 150,000 pg/mL, from about 60 pg/mL to about 150,000 pg/mL, from about 70 pg/mL to about 150,000 pg/mL, from about 75 pg/mL to about 150,000 pg/mL, from about 80 pg/mL to about 150,000 pg/mL, from about 90 pg/mL to about 150,000 pg/mL, from about 100 pg/mL to about 150,000 pg/mL, from about 100 pg/mL to about 150,000 pg/mL, from about 125 pg/mL to about 150,000 pg/mL, or from about 150 pg/mL to about 150,000 pg/mL. In some embodiments, the method can be used to determine levels of GFAP selected from the group consisting of from about 10.0 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 100 pg/mL to about 50,000 pg/mL, from about 125 pg/mL to about 50,000 pg/mL, from about 150 pg/mL to about 50,000 pg/mL, from about 100 pg/mL to about 50,000 pg/mL, from about 125 pg/mL to about 50,000 pg/mL, and from about 150 pg/mL to about 50,000 pg/mL. In some embodiments, the method can be used to determine levels of GFAP selected from the group consisting of from about 10.0 pg/mL to about 150,000 pg/mL, from about 20 pg/mL to about 150,000 pg/mL, from about 25 pg/mL to about 150,000 pg/mL, from about 30 pg/mL to about 150,000 pg/mL, from about 40 pg/mL to about 150,000 pg/mL, from about 50 pg/mL to about 150,000 pg/mL, from about 60 pg/mL to about 150,000 pg/mL, from about 70 pg/mL to about 150,000 pg/mL, from about 75 pg/mL to about 150,000 pg/mL, from about 80 pg/mL to about 150,000 pg/mL, from about 90 pg/mL to about 150,000 pg/mL, from about 100 pg/mL to about 150,000 pg/mL, from about 100 pg/mL to about 150,000 pg/mL, from about 125 pg/mL to about 150,000 pg/mL, or from about 150 pg/mL to about 150,000 pg/mL.

In some embodiments, levels of at least 0.5 pg/mL, 0.10 pg/mL, 0.50 pg/mL, 1 pg/mL, 5 pg/mL, 10 pg/mL, 15 pg/mL, 20 pg/mL, 31 pg/mL, 32 pg/mL, 33 pg/mL, 34 pg/mL, 35 pg/mL, 40 pg/mL, 50 pg/mL, 60 pg/mL, 70 pg/mL, 80 pg/mL, 90 pg/mL, or 100 pg/mL of GFAP (or GFAP fragment) in a biological sample may be detected. In some embodiments levels less than about 150,000 pg/mL, less than about 100,000 pg/mL, less than about 90,000 pg/mL, less than about 80,000 pg/mL, less than about 70,000 pg/mL, less than about 60,000 pg/mL, less than about 50,000 pg/mL, less than about 40,000 pg/mL, less than about 30,000 pg/mL, or less than about 25,000 pg/mL of GFAP (or GFAP fragment) in a biological sample may be detected.

In some embodiments, levels of at least about 10,000 pg/mL, at least about 15,000 pg/mL, at least about 20,000 pg/mL, at least about 25,000 pg/mL, at least about 30,000 pg/mL, at least about 35,000 pg/mL, at least about 40,000 pg/mL, at least about 45,000 pg/mL, at least about 50,000 pg/mL, at least about 60,000 pg/mL, at least about 70,000 pg/mL, at least about 80,000 pg/mL, at least about 90,000 pg/mL, at least about 100,000 pg/mL, or at least about 150,000 pg/mL of GFAP (or GFAP fragment) in a biological sample may be detected.

In some embodiments, the assay can have a lower end limit of detection (LoD) of about 10 pg/mL, about 15 pg/mL, about 20 pg/mL, about 25 pg/mL, about 30 pg/mL, about 40 pg/mL, or about 50 pg/mL.

In some embodiments, the assay can have less than 10% deviation from linearity (DL) over a range from about 10 pg/mL to about 50,000 pg/mL. For example, the assay can have less than 10%, less than 9%, less than 8%, less than 7%, less than 6%, less than 5%, less than 4%, less than 3%, less than 2%, less than 1%, less than 0.5% deviation from linearity over a range from about 10 pg/mL to about 50,000 pg/mL, a range from about 12 pg/mL to about 50,000 pg/mL, a range from about 12 pg/mL to about 13,660 pg/mL, a range from about 12 pg/mL to about 900 pg/mL, a range from about 370 pg/mL to about 50,000 pg/mL, or a range from about 420 pg/mL to about 50,000 pg/mL.

In some embodiments, the method has a GFAP quantitation range from 20 pg/mL to 50,000 pg/mL at 20% coefficient of variation (CV). In some embodiments, the method has a GFAP detection range from 20 pg/mL to 50,000 pg/mL at 20% coefficient of variation (CV).

In some embodiments, the first specific binding member is immobilized on a solid support. In some embodiments, the second specific binding member is immobilized on a solid support. In some embodiments, the first specific binding member is a GFAP antibody as described below. In some embodiments, the second specific binding member is a GFAP antibody as described below. In some embodiments, each of the first specific binding member and the second specific binding member is a GFAP antibody.

In some embodiments, the biological sample is diluted or undiluted. The biological sample can be from about 1 to about 25 microliters, about 1 to about 24 microliters, about 1 to about 23 microliters, about 1 to about 22 microliters, about 1 to about 21 microliters, about 1 to about 20 microliters, about 1 to about 18 microliters, about 1 to about 17 microliters, about 1 to about 16 microliters, about 15 microliters or about 1 microliter, about 2 microliters, about 3 microliters, about 4 microliters, about 5 microliters, about 6 microliters, about 7 microliters, about 8 microliters, about 9 microliters, about 10 microliters, about 11 microliters, about 12 microliters, about 13 microliters, about 14 microliters, about 15 microliters, about 16 microliters, about 17 microliters, about 18 microliters, about 19 microliters, about 20 microliters, about 21 microliters, about 22 microliters, about 23 microliters, about 24 microliters or about 25 microliters. In some embodiments, the biological sample is from about 1 to about 150 microliters or less or from about 1 to about 25 microliters or less.

In some embodiments, the subject is suspected to have sustained an injury to the head. In some embodiments, the subject is known to have sustained an injury to the head. In some embodiments, the subject is suspected to be suffering from mild, moderate or severe TBI. In some embodiments, the subject is suspected to be suffering from mild TBI. In some embodiments, the subject is suspected to be suffering from moderate TBI. In some embodiments, the subject is suspected to be suffering from severe TBI.

In some embodiments, the time between when the biological sample is obtained and when the subject suffers an injury is not known. In some embodiments, the biological sample is obtained within about 15 minutes, about 20 minutes, about 25 minutes, about 30 minutes, about 35 minutes, about 40 minutes, about 45 minutes, about 50 minutes, about 55 minutes, about 60 minutes, about 90 minutes, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, about 36 hours, about 37 hours, about 38 hours, about 39 hours, about 40 hours, about 41 hours, about 42 hours, about 43 hours, about 44 hours, about 45 hours, about 46 hours, about 47 hours, about 48 hours, about 3 days, about 4 days, about 5 days, about 6 days, about 7 days, about 8 days, about 9 days, about 10 days, about 11 days, about 12 days, about 3 weeks, about 4 weeks, about 5 weeks, about 6 weeks, about 7 weeks, about 8 weeks, about 9 weeks, about 10 weeks, about 11 weeks, about 12 weeks, about 3 months, about 4 months, about 5 months, about 6 months, about 7 months, about 8 months, about 9 months, about 10 months, about 11 months, about 12 months, about 13 months, about 14 months, about 15 months, about 16 months, about 17 months, about 18 months, about 19 months, about 20 months, about 21 months, about 22 months, about 23 months, about 24 months, about 3 years, about 4 years, about 5 years, about 6 years, about 7 years, about 8 years, about 9 years or about 10 years after the subject suffers an injury For example, the injury may be an injury to the head. In some embodiments, the biological sample is obtained within about 15 minutes, about 20 minutes, about 25 minutes, about 30 minutes, about 35 minutes, about 40 minutes, about 45 minutes, about 50 minutes, about 55 minutes, about 60 minutes, about 90 minutes, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, about 36 hours, about 37 hours, about 38 hours, about 39 hours, about 40 hours, about 41 hours, about 42 hours, about 43 hours, about 44 hours, about 45 hours, about 46 hours, about 47 hours, about 48 hours, about 3 days, about 4 days, about 5 days, about 6 days, about 7 days, about 8 days, about 9 days, about 10 days, about 11 days, about 12 days, about 3 weeks, about 4 weeks, about 5 weeks, about 6 weeks, about 7 weeks, about 8 weeks, about 9 weeks, about 10 weeks, about 11 weeks, about 12 weeks, about 3 months, about 4 months, about 5 months, about 6 months, about 7 months, about 8 months, about 9 months, about 10 months, about 11 months, about 12 months, about 13 months, about 14 months, about 15 months, about 16 months, about 17 months, about 18 months, about 19 months, about 20 months, about 21 months, about 22 months, about 23 months, about 24 months, about 3 years, about 4 years, about 5 years, about 6 years, about 7 years, about 8 years, about 9 years or about 10 years after the subject has ingested or been exposed to a chemical, toxin or a combination of a chemical or a toxin. Examples of such chemicals and/or toxins include, fires, molds, asbestos, pesticides and insecticides, organic solvents, paints, glues, gases (such as carbon monoxide, hydrogen sulfide, and cyanide), organic metals (such as methyl mercury, tetraethyl lead and organic tin) and/or one or more drugs of abuse. In some embodiments, the biological sample is obtained from a subject suffering from a disease, such as an autoimmune disease, a metabolic disorder, a brain tumor, hypoxia, one or more viruses, meningitis, hydrocephalus or combinations thereof. In some embodiments, the method is done either to confirm the occurrence of traumatic brain injury or the absence of traumatic brain injury. The method may be performed in from about 1 minutes, about 2 minutes, about 3 minutes, about 4 minutes, about 5 minutes, about 6 minutes about 7 minutes, about 8 minutes, about 9 minutes, about 10 minutes, about 11 minutes, about 12 minutes, about 13 minutes, about 14 minutes, about 15 minutes, about 16 minute about 17 minutes, about 18 minutes, about 19 minutes, about 20 minutes, about 30 minutes, about 45 minutes, about 60 minutes, about 90 minutes, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 21 hours, about 22 hours, about 23 hours, about 24 hours, about 36 hours, about 48 hours, hours 72 hours, etc.

**b. Methods of Providing an Aid in the Diagnosis of a Subject Having Traumatic Brain Injury**

In another embodiment, the methods described herein can be used to provide an aid in the diagnosis of a subject having traumatic brain injury by determining the levels of GFAP in a subject. The method may be used to detect or assess traumatic brain injury in a subject using the anti-GFAP antibodies described below, or antibody fragments thereof. The method includes the steps of (a) obtaining a biological sample from a subject, (b) determining the level of GFAP in the biological sample using anti-GFAP antibodies, or antibody fragments thereof, (c) comparing the level of GFAP in the biological sample to a reference level of GFAP, (d) identifying the subject as having traumatic brain injury if the level of GFAP in the biological sample is greater than the reference level of GFAP, and optionally (e) administering a treatment regimen to the subject identified as having traumatic brain injury. In some embodiments, the method is conducted using a point-of-care device.

By measuring and assessing GFAP, the method allows for more diseases to be more accurately diagnosed and subsequently treated more successfully, compared to other commercially available GFAP immunoassays. The method can be adapted for use in an automated system or a semi-automated system.

Generally, a predetermined level can be employed as a benchmark against which to assess results obtained upon assaying a test sample for GFAP. Generally, in making such a comparison, the predetermined level is obtained by running a particular assay a sufficient number of times and under appropriate conditions such that a linkage or association of analyte presence, amount or concentration with a particular stage or endpoint of TBI or with particular indicia can be made. Typically, the predetermined level is obtained with assays of reference subjects (or populations of subjects). The GFAP measured can include GFAP fragments thereof, degradation products thereof, and/or enzymatic cleavage products thereof.

The reference level in this method can be the level of GFAP in a patient having traumatic brain injury. In some embodiments, the reference level is within the dynamic range of the method described herein. In some embodiments, the dynamic range is about 5 pg/mL to about 50,000 pg/mL, about 10.0 pg/mL to about 50,000 pg/mL, about 12 pg/mL to about 50,000 pg/mL, or about 20 pg/mL to about 50,000 pg/mL. In some embodiments, levels higher than or equal to 5 pg/mL, 10 pg/mL, 20 pg/mL, 30 pg/mL, 40 pg/mL, 50 pg/mL, 60 pg/mL, 70 pg/mL, 80 pg/mL, 90 pg/mL, 100 pg/mL, 500 pg/mL, 1000 pg/mL, 5000 pg/mL, 10000 pg/mL, or 50000 pg/mL in serum of GFAP identify the subject as having traumatic brain injury. Optionally, in some cases, levels higher than or equal to 100000 pg/mL, 500000 pg/mL, 1000000 pg/mL, 150000 pg/mL, 200000 pg/mL, or 500000 pg/mL in serum of GFAP identify the subject as having traumatic brain injury. In some embodiments, levels higher than or equal to 5 pg/mL, 10 pg/mL, 20 pg/mL, 30 pg/mL, 40 pg/mL, 50 pg/mL, 60 pg/mL, 70 pg/mL, 80 pg/mL, 90 pg/mL, 100 pg/mL, 500 pg/mL, 1000 pg/mL, 5000 pg/mL, 10000 pg/mL, or 50000 pg/mL in plasma of GFAP identify the subject as having traumatic brain injury. Optionally, in some cases, levels higher than or equal to 100000 pg/mL, 500000 pg/mL, 1000000 pg/mL, 150000 pg/mL, 200000 pg/mL, or 500000 pg/mL in plasma of GFAP identify the subject as having traumatic brain injury.

### c. Methods for Predicting Whether a Subject Who has Suffered Traumatic Brain Injury is a Candidate for Therapy or Treatment

In yet another embodiment, the methods described herein also can be used to predict whether a subject who has previously suffered a TBI is a candidate for therapy by determining the levels of GFAP in a subject using the anti-GFAP antibodies described below, or antibody fragments thereof. Thus, in particular embodiments, the disclosure also provides a method for determining whether a subject having, or at risk for, traumatic brain injuries, discussed herein and known in the art, is a candidate for therapy or treatment. Generally, the subject is at least one who: (i) has experienced an injury to the head; (ii) ingested and/or been exposed to one or more chemicals and/or toxins; (iii) suffers from an autoimmune disease, a metabolic disorder, a brain tumor, hypoxia, one or more viruses, meningitis, hydrocephalus or suffers from any combinations thereof; or (iv) any combinations of (i)-(iii); or, who has actually been diagnosed as having, or being at risk for TBI (such as, for example, subjects suffering from an autoimmune disease, a metabolic disorder, a brain tumor, hypoxia, one or more viruses, meningitis, hydrocephalus or combinations thereof), and/or who demonstrates an unfavorable (i.e., clinically undesirable) concentration or amount of GFAP or GFAP fragment, as described herein.

Specifically, such a method can comprise the steps of: (a) determining the concentration or amount in a test sample from a subject of GFAP using the methods described herein, or methods known in the art); and (b) comparing the concentration or amount of GFAP determined in step (a) with a predetermined level, wherein, if the concentration or amount of GFAP determined in step (a) is favorable with respect to a predetermined level, then the subject is determined not to be a candidate for therapy or treatment. However, if the concentration or amount of GFAP determined in step (a) is unfavorable with respect to the predetermined level, then the subject is determined to be a candidate for therapy or treatment as discussed herein in section f and known in the art. In some embodiments, the method is conducted using a point-of-care device. An example of a point-of-care device that can be used is i-STAT® (Abbott, Laboratories, Abbott Park, IL).

### d. Methods of Monitoring the Progression of Traumatic Brain Injury in a Subject

In yet another embodiment, the methods described herein also can be used to monitor the progression of disease and/or injury, such as traumatic brain injury, in a subject by determining the levels of GFAP in a subject using the anti-GFAP antibodies described below, or antibody fragments thereof. Optimally, the method includes the steps of (a) determining the concentration or amount of GFAP in a test sample from a subject using the anti-GFAP antibodies described below, or antibody fragments thereof, (b) determining the concentration or amount of GFAP in a later test sample from a subject using the anti-GFAP antibodies described below, or antibody fragments thereof, and (c) comparing the concentration or amount of GFAP as determined in step (b) with the concentration or amount of GFAP determined in step (a), wherein if the concentration or amount determined in step (b) is unchanged or is unfavorable when compared to the concentration or amount of GFAP determined in step (a), then the disease in the subject is determined to have continued, progressed or worsened. By comparison, if the concentration or amount of GFAP as determined in step (b) is favorable when compared to the concentration or amount of GFAP as determined in step (a), then the disease in the subject is determined to have discontinued, regressed or improved. In some embodiments, the method is conducted using a point-of-care device. An example of a point-of-care device that can be used is i-STAT® (Abbott, Laboratories, Abbott Park, IL).

Optionally, the method further comprises comparing the concentration or amount of GFAP as determined in step (b), for example, with a predetermined level. Further, optionally the method comprises treating the subject with one or more pharmaceutical compositions for a period of time if the comparison shows that the concentration or amount of GFAP as determined in step (b), for example, is unfavorably altered with respect to the predetermined level.

Still further, the methods can be used to monitor treatment in a subject receiving treatment with one or more pharmaceutical compositions. Specifically, such methods involve providing a first test sample from a subject before the subject has been administered one or more pharmaceutical compositions. Next, the concentration or amount in a first test sample from a subject of GFAP is determined (e.g., using the methods described herein or as known in the art). After the concentration or amount of GFAP is determined, optionally the concentration or amount of GFAP is then compared with a predetermined level. If the concentration or amount of GFAP as determined in the first test sample is lower than the predetermined level, then the subject is not treated with one or more pharmaceutical compositions or alternatively, the subject may be treated with one or more pharmaceutical compositions. If the concentration or amount of GFAP as determined in the first test sample is higher than the predetermined level, then the subject is treated with one or more pharmaceutical compositions for a period of time or alternatively, the subject is not treated with one or more pharmaceutical compositions. The period of time that the subject is treated with the one or more pharmaceutical compositions can be determined by one skilled in the art (for example, the period of time can be from about seven (7) days to about two years, preferably from about fourteen (14) days to about one (1) year).

During the course of treatment with the one or more pharmaceutical compositions, second and subsequent test samples are then obtained from the subject. The number of test samples and the time in which said test samples are obtained from the subject are not critical. For example, a second test sample could be obtained seven (7) days after the subject is first administered the one or more pharmaceutical compositions, a third test sample could be obtained two (2) weeks after the subject is first administered the one or more pharmaceutical compositions, a fourth test sample could be obtained three (3) weeks after the subject is first administered the one or more pharmaceutical compositions, a fifth test sample could be obtained four (4) weeks after the subject is first administered the one or more pharmaceutical compositions, etc.

After each second or subsequent test sample is obtained from the subject, the concentration or amount of GFAP is determined in the second or subsequent test sample is determined (e.g., using the methods described herein or as known in the art). The concentration or amount of GFAP as determined in each of the second and subsequent test samples is then compared with the concentration or amount of GFAP as determined in the first test sample (e.g., the test sample that was originally optionally compared to the predetermined level). If the concentration or amount of GFAP as determined in step (c) is favorable when compared to the concentration or amount of GFAP as determined in step (a), then the disease in the subject is determined to have discontinued, regressed, or improved, and the subject can continue to be administered the one or pharmaceutical compositions of step (b). However, if the concentration or amount determined in step (c) is unchanged or is unfavorable when compared to the concentration or amount of GFAP as determined in step (a), then the disease in the subject is determined to have continued, progressed or worsened, and the subject can be treated with a higher concentration of the one or more pharmaceutical compositions administered to the subject in step (b) or the subject can be treated with one or more pharmaceutical compositions that are different from the one or more pharmaceutical compositions administered to the subject in step (b). Specifically, the subject can be treated with one or more pharmaceutical compositions that are different from the one or more pharmaceutical compositions that the subject had previously received to decrease or lower said subject's GFAP level.

Generally, for assays in which repeat testing may be done (e.g., monitoring disease progression and/or response to treatment), a second or subsequent test sample is obtained at a period in time after the first test sample has been obtained from the subject. Specifically, a second test sample from the subject can be obtained minutes, hours, days, weeks or years after the first test sample has been obtained from the subject. For example, the second test sample can be obtained from the subject at a time period of about 1 minute, about 5 minutes, about 10 minutes, about 15 minutes, about 30 minutes, about 45 minutes, about 60 minutes, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 2 days, about 3 days, about 4 days, about 5 days, about 6 days, about 7 days, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 1.5 years, about 2 years, about 2.5 years, about 3.0 years, about 3.5 years, about 4.0 years, about 4.5 years, about 5.0 years, about 5.5. years, about 6.0 years, about 6.5 years, about 7.0 years, about 7.5 years, about 8.0 years, about 8.5 years, about 9.0 years, about 9.5 years, or about 10.0 years after the first test sample from the subject is obtained.

When used to monitor disease progression, the above assay can be used to monitor the progression of disease in subjects suffering from acute conditions. Acute conditions, also known as critical care conditions, refer to acute, life-threatening diseases or other critical medical conditions involving, for example, the cardiovascular system or excretory system. Typically, critical care conditions refer to those conditions requiring acute medical intervention in a hospital-based setting (including, but not limited to, the emergency room, intensive care unit, trauma center, or other emergent care setting) or administration by a paramedic or other field-based medical personnel. For critical care conditions, repeat monitoring is generally done within a shorter time frame, namely, minutes, hours or days (e.g., about 1 minute, about 5 minutes, about 10 minutes, about 15 minutes, about 30 minutes, about 45 minutes, about 60 minutes, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 2 days, about 3 days, about 4 days, about 5 days, about 6 days or about 7 days), and the initial assay likewise is generally done within a shorter timeframe, e.g., about minutes, hours or days of the onset of the disease or condition.

The assays also can be used to monitor the progression of disease in subjects suffering from chronic or non-acute conditions. Non-critical care conditions or non-acute conditions, refers to conditions other than acute, life-threatening disease or other critical medical conditions involving, for example, the cardiovascular system and/or excretory system. Typically, non-acute conditions include those of longer-term or chronic duration. For non-acute conditions, repeat monitoring generally is done with a longer timeframe, e.g., hours, days, weeks, months or years (e.g., about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 2 days, about 3 days, about 4 days, about 5 days, about 6 days, about 7 days, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 1.5 years, about 2 years, about 2.5 years, about 3.0 years, about 3.5 years, about 4.0 years, about 4.5 years, about 5.0 years, about 5.5. years, about 6.0 years, about 6.5 years, about 7.0 years, about 7.5 years, about 8.0 years, about 8.5 years, about 9.0 years, about 9.5 years or about 10.0 years), and the initial assay likewise generally is done within a longer time frame, e.g., about hours, days, months or years of the onset of the disease or condition.

Furthermore, the above assays can be performed using a first test sample obtained from a subject where the first test sample is obtained from one source, such as urine, whole blood, serum, or plasma. Optionally the above assays can then be repeated using a second test sample obtained from the subject where the second test sample is obtained from another source. For example, if the first test sample was obtained from urine, the second test sample can be obtained from whole blood, serum or plasma. The results obtained from the assays using the first test sample and the second test sample can be compared. The comparison can be used to assess the status of a disease or condition in the subject.

In particular, with respect to a predetermined level as employed for monitoring disease progression and/or treatment or for determining the risk of a subject of developing traumatic brain injury, the amount or concentration of GFAP or GFAP fragment may be "unchanged," "favorable" (or "favorably altered"), or "unfavorable" (or "unfavorably altered"). "Elevated" or "increased" refers to an amount or a concentration in a test sample that is higher or greater than a typical or normal level or range (e.g., predetermined level), or is higher or greater than another reference level or range (e.g., earlier or baseline sample). The term "lowered" or "reduced" refers to an amount or a concentration in a test sample that is lower or less than a typical or normal level or range (e.g., predetermined level), or is lower or less than another reference level or range (e.g., earlier or baseline sample). The term "altered" refers to an amount or a concentration in a sample that is altered (increased or decreased) over a typical or normal level or range (e.g., predetermined level), or over another reference level or range (e.g., earlier or baseline sample).

The typical or normal level or range for GFAP is defined in accordance with standard practice. A so-called altered level or alteration can be considered to have occurred when there is any net change as compared to the typical or normal level or range, or reference level or range that cannot be explained by experimental error or sample variation. Thus, the level measured in a particular sample will be compared with the level or range of levels determined in similar samples from a so-called normal subject. In this context, a "normal subject" is an individual with no detectable disease or disorder, and a "normal" (sometimes termed "control") patient or population is/are one(s) that exhibit(s) no detectable disease or disorder, respectively, for example. An "apparently normal subject" is one in which GFAP has not been or is being assessed. The level of an analyte is said to be "elevated" when the analyte is normally undetectable (e.g., the normal level is zero, or within a range of from about 25 to about 75 percentiles of normal populations), but is detected in a test sample, as well as when the analyte is present in the test sample at a higher than normal level. Thus, inter alia, the disclosure provides a method of screening for a subject having, or at risk of having, traumatic brain injury.

**e. Other Factors**

The methods of aiding in the diagnosing, prognosticating, and/or assessing, as described above, can further include using other factors for aiding in the diagnosis of as well as the prognostication, and assessment. The methods of evaluating and predicting, as described above, can further include using other factors for the evaluating and predicting. In some embodiments, traumatic brain injury may be diagnosed using the Glasgow Coma Scale or the Extended Glasgow Outcome Scale (GOSE). Other tests, scales or indices can also be used either alone or in combination with the Glasgow Coma Scale. An example is the Ranchos Los Amigos Scale. The Ranchos Los Amigos Scale measures the levels of awareness, cognition, behavior and interaction with the environment. The Ranchos Los Amigos Scale includes: Level I: No Response; Level II: Generalized Response; Level III: Localized Response; Level IV: Confused-agitated; Level V: Confused-inappropriate; Level VI: Confused-appropriate; Level VII: Automatic-appropriate; and Level VIII: Purposeful-appropriate.

**f. Medical Treatment of Subjects Suffering from Traumatic Brain Injury**

The subject identified or assessed in the methods described above as having traumatic brain injury, such as mild traumatic brain injury or moderate to severe traumatic brain injury, may be treated using medical techniques known in the art. In some embodiments, the method further includes treating the human subject assessed as having traumatic brain injury with a traumatic brain injury treatment, such as any treatments known in the art. For example, treatment of traumatic brain injury can take a variety of forms depending on the severity of the injury to the head. For example, for subjects suffering from mild TBI, the treatment may include one or more of rest, abstaining from physical activities, such as sports, avoiding light or wearing sunglasses when out in the light, medication for relief of a headache or migraine, anti-nausea medication, etc. Treatment for patients suffering from severe TBI might include administration of one or more appropriate medications (such as, for example, diuretics, anti-convulsant medications, medications to sedate and put an individual in a drug-induced coma, or other pharmaceutical or biopharmaceutical medications (either known or developed in the future for treatment of TBI), one or more surgical procedures (such as, for example, removal of a hematoma, repairing a skull fracture, decompressive craniectomy, etc.) and one or more therapies (such as, for example one or more rehabilitation, cognitive behavioral therapy, anger management, counseling psychology, etc.).

**g. Monitoring of Subjects Suffering from Traumatic Brain Injury**

The subject identified or assessed in the methods described above as having traumatic brain injury, such as mild traumatic brain injury or moderate to severe traumatic brain injury, may be monitored using any methods known in the art. For example, the patient suffering from traumatic brain injury, such as mild traumatic brain injury or severe traumatic brain injury, may be monitored with CT scan or MRI.

**3. Combinations of GFAP with other Biomarkers**

As will be discussed in further detail below, the antibodies described herein can be used in a variety of methods to detect and measure levels and concentrations of GFAP in combination with one or more biomarkers or immunoassays specific for disease. The present disclosure contemplates that the combination of GFAP with one or more biomarkers or immunoassays specific for disease may provide a greater discrimination between healthy controls and individuals with disease compared to measuring GFAP alone. For example, measuring a panel of GFAP and additional traumatic brain injury biomarkers may provide a greater discrimination between healthy controls and individuals with disease compared to a panel of GFAP alone. The combination of GFAP with at least one or more biomarkers may provide greater discrimination between healthy controls and individuals who have traumatic brain injury. Examples of the one or more biomarkers include ubiquitin carboxy-terminal hydrolase L1 (UCH-L1), S100 calcium-binding protein B (S100b), brain lipid binding protein (BLBP), aldolase C (ALDOC), astrocytic phosphoprotein 15 (PEA15), glutamine synthetase (GS), crystallin B chain (CRYAB), neuron specific enolase (NSE), brain-derived neurotrophic factor (BDNF), Tau, P-tau, c-reactive protein (CRP), apolipoprotein A-I (ApoA1) and NFL. Such panel assays optionally can be carried out by comparing independent assays, (e.g., singleplex assays).

Alternately, the methods as described herein may be done using multiplex assays. Such multiplex methods optionally may include one or more (or alternately two or more) specific binding members to detect one or more (or alternately two or more) target analytes in the sample in a multiplexing assay. Each of the one or more (or alternately two or more) specific binding members optionally binds to a different target analyte and each specific binding member is conjugated to a different signal generating compound or signal generating substrate. For example, a first specific binding member binds to a first target analyte, a second specific binding member binds to a second target analyte, a third specific binding member binds to a third target analyte, etc. and the first specific binding member is labeled with a first signal generating compound or first signal generating substrate, the second specific binding member is labeled with a second signal generating compound or second signal generating substrate, the third specific binding member is labeled with a third signal generating compound or a third signal generating substrate, etc. In some embodiments, a first condition causes the activation, cleavage or release of the first signal generating compound or first signal generating substrate if the first specific binding member is labeled with a signal generating compound or first signal generating substrate, a second condition causes the activation, cleavage or release of the second signal generating compound or second signal generating substrate if the second specific binding member is labeled with a signal generating compound or signal generating substrate, a third condition causes the activation, cleavage or release of the third signal generating compound or third signal generating substrate if the third specific binding member is labeled with a signal generating compound or signal generating substrate, etc. In some embodiments, the conditions of the sample can be changed at various times during the assay, allowing detection of the first signal generating compound or first signal generating substrate, the second signal generating compound or second signal generating substrate, the third signal generating compound or third signal generating substrate, etc., thereby detecting one or more (or alternately two or more) target analytes. In some embodiments, the one or more (or alternately two or more) activated or cleaved signal generating compounds or signal generating substrates are detected simultaneously. In some embodiments, the one or more (or alternately two or more) activated or cleaved signal generating compounds or signal generating substrates are detected consecutively. In some embodiments, the one or more (or alternately two or more) activated or cleaved signal generating compounds or signal generating substrates generates a different detectable signal, such as a different wavelength of fluorescence signal.

Alternatively, each of the one or more (or alternately two or more) specific binding members binds to a different target analyte and each specific binding member is conjugated to a different solid support, e.g., such as a different fluorophore bead. For example, a first specific binding member binds to a first target analyte, a second specific binding member binds to a second target analyte, a third specific binding member binds to a third target analyte, etc., the first specific binding member is labeled with a first signal generating compound or first signal generating substrate, the second specific binding member is labeled with a second signal generating compound or second signal generating substrate, the third specific binding member is labeled with a third signal generating compound or a third signal generating substrate, etc., and the first specific binding member is immobilized on a first solid support, the second specific binding member is immobilized on a second solid support, the third specific binding member is immobilized on a third solid support, etc. In some embodiments, the one or more (or alternately two or more) activated or cleaved signal generating compounds or signal generating substrates generate a different detectable signal, such as a different wavelength or fluorescence signal, and the different solid supports is detected simultaneously or consecutively.

In some embodiments, a first specific binding member binds to a first target analyte, a second specific binding member binds to a second target analyte, a third specific binding member binds to a third target analyte, etc., the first specific binding member, the second specific binding member, the third specific binding member, etc. are labeled with a signal generating compound or a signal generating substrate, and the first specific binding member is immobilized on a first solid support, the second specific binding member is immobilized on a second solid support, the third specific binding member is immobilized on a third solid support, etc. In some embodiments, the activated or cleaved signal generating compounds or signal generating substrates generates a detectable signal, such as a different wavelength or fluorescence signal, and the different solid supports is detected simultaneously or consecutively.

## 4. GFAP Antibodies

The methods described herein may use an isolated antibody that specifically binds to human Glial fibrillary acidic protein ("GFAP") (or fragments thereof), referred to as "GFAP antibody." The GFAP antibodies specifically recognize and bind epitopes within GFAP break down products. The GFAP antibodies can be used to assess the GFAP status as a measure of traumatic brain injury, detect the presence of GFAP in a biological sample, quantify the amount of GFAP present in a biological sample, or detect the presence of and quantify the amount of GFAP in a biological sample.

### a. Glial fibrillary acidic protein (GFAP)

Glial fibrillary acidic protein (GFAP) is a 50 kDa intracytoplasmic filamentous protein that constitutes a portion of the cytoskeleton in astrocytes, and it has proved to be the most specific marker for cells of astrocytic origin. GFAP protein is encoded by the GFAP gene in humans. GFAP is the principal intermediate filament of mature astrocytes. In the central rod domain of the molecule, GFAP shares considerable structural homology with the other intermediate filaments. GFAP is involved in astrocyte motility and shape by providing structural stability to astrocytic processes. Glial fibrillary acidic protein and its breakdown products (GFAP-BDP) are brain-specific proteins released into the blood as part of the pathophysiological response after traumatic brain injury (TBI). Following injury to the human CNS caused by trauma, genetic disorders, or chemicals, astrocytes proliferate and show extensive hypertrophy of the cell body and processes, and GFAP is markedly upregulated. In contrast, with increasing astrocyte malignancy, there is a progressive loss of GFAP production. GFAP can also be detected in Schwann cells, enteric glia cells, salivary gland neoplasms, metastasizing renal carcinomas, epiglottic cartilage, pituicytes, immature oligodendrocytes, papillary meningiomas, and myoepithelial cells of the breast.

Human GFAP may have the following amino acid sequence:

MERRRITSAARRSYVSSGEMMVGGLAPGRRLGPGTRLSLARMPPPLPTRVDFS
LAGALNAGFKETRASERAEMMELNDRFASYIEKVRFLEQQNKALAAELNQLRAKEPTK
LADVYQAELRELRLRLDQLTANSARLEVERDNLAQDLATVRQKLQDETNLRLEAENNL
AAYRQEADEATLARLDLERKIESLEEEIRFLRKIHEEEVRELQEQLARQQVHVELDVAKP
DLTAALKEIRTQYEAMASSNMHEAEEWYRSKFADLTDAAARNAELLRQAKHEANDYR
RQLQSLTCDLESLRGTNESLERQMREQEERHVREAASYQEALARLEEEGQSLKDEMAR
HLQEYQDLLNVKLALDIEIATYRKLLEGEENRITIPVQTFSNLQIRETSLDTKSVSEGHLK
RNIVVKTVEMRDGEVIKESKQEHKDVM (SEQ ID NO: 1).

The human GFAP may be a fragment or variant of SEQ ID NO: 1. The fragment of GFAP may be between 5 and 400 amino acids, between 10 and 400 amino acids, between 50 and 400 amino acids, between 60 and 400 amino acids, between 65 and 400 amino acids, between 100 and 400 amino acids, between 150 and 400 amino acids, between 100 and 300 amino acids, or between 200 and 300 amino acids in length. The fragment may comprise a contiguous number of amino acids from SEQ ID NO: 1. The human GFAP fragment or variant of SEQ ID NO: 1 may be a GFAP breakdown product (BDP). The GFAP BDP may be 38 kDa, 42 kDa (fainter 41 kDa), 47 kDa (fainter 45 kDa); 25 kDa (fainter 23 kDa); 19 kDa, or 20 kDa.

### b. GFAP-Recognizing Antibody

The antibody is an antibody that binds to GFAP, a fragment thereof, an epitope of GFAP, or a variant thereof. The antibody may be a fragment of the anti-GFAP antibody or a variant or a derivative thereof. The antibody may be a polyclonal or monoclonal antibody. The antibody may be a chimeric antibody, a single chain antibody, an affinity matured antibody, a human antibody, a humanized antibody, a fully human antibody or an antibody fragment, such as a Fab fragment, or a mixture thereof. Antibody fragments or derivatives may comprise F(ab')$_2$, Fv or scFv fragments. The antibody derivatives can be produced by peptidomimetics. Further, techniques described for the production of single chain antibodies can be adapted to produce single chain antibodies.

The anti-GFAP antibodies may be a chimeric anti-GFAP or humanized anti-GFAP antibody. In one embodiment, both the humanized antibody and chimeric antibody are monovalent. In one embodiment, both the humanized antibody and chimeric antibody comprise a single Fab region linked to an Fc region.

Human antibodies may be derived from phage-display technology or from transgenic mice that express human immunoglobulin genes. The human antibody may be generated as a result of a human in vivo immune response and isolated. See, for example, Funaro et al., BMC Biotechnology, 2008(8):85. Therefore, the antibody may be a product of the human and not animal repertoire. Because it is of human origin, the risks of reactivity against self-antigens may be minimized. Alternatively, standard yeast display libraries and display technologies may be used to select and isolate human anti-GFAP antibodies. For example, libraries of naive human single chain variable fragments (scFv) may be used to select human anti-GFAP antibodies. Transgenic animals may be used to express human antibodies.

Humanized antibodies may be antibody molecules from non-human species antibody that binds the desired antigen having one or more complementarity determining regions (CDRs) from the non-human species and framework regions from a human immunoglobulin molecule.

The antibody is distinguishable from known antibodies in that it possesses different biological function(s) than those known in the art.

**(1) Epitope**

The antibody may immunospecifically bind to GFAP (SEQ ID NO: 1), a fragment thereof, or a variant thereof. The antibody may immunospecifically recognize and bind at least three amino acids, at least four amino acids, at least five amino acids, at least six amino acids, at least seven amino acids, at least eight amino acids, at least nine amino acids, or at least ten amino acids within an epitope region. The antibody may immunospecifically recognize and bind to an epitope that has at least three contiguous amino acids, at least four contiguous amino acids, at least five contiguous amino acids, at least six contiguous amino acids, at least seven contiguous amino acids, at least eight contiguous amino acids, at least nine contiguous amino acids, or at least ten contiguous amino acids of an epitope region.

**c. Antibody Preparation/Production**

Antibodies may be prepared by any of a variety of techniques, including those well known to those skilled in the art. In general, antibodies can be produced by cell culture techniques, including the generation of monoclonal antibodies via conventional techniques, or via transfection of antibody genes, heavy chains, and/or light chains into suitable bacterial or mammalian cell hosts, in order to allow for the production of antibodies, wherein the antibodies may be recombinant. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is possible to express the antibodies in either prokaryotic or eukaryotic host cells, expression of antibodies in eukaryotic cells is preferable, and most preferable in mammalian host cells, because such eukaryotic cells (and in particular mammalian cells) are more likely than prokaryotic cells to assemble and secrete a properly folded and immunologically active antibody.

Exemplary mammalian host cells for expressing the recombinant antibodies include Chinese Hamster Ovary (CHO cells) (including dhfr-CHO cells, described in Urlaub and Chasin, Proc. Natl. Acad. Sci. USA, 77: 4216-4220 (1980)), used with a DHFR selectable marker, e.g., as described in Kaufman and Sharp, J. Mol. Biol., 159: 601-621 (1982), NS0 myeloma cells, COS cells, and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce functional antibody fragments, such as Fab fragments or scFv molecules. It will be understood that variations on the above procedure may be performed. For example, it may be desirable to transfect a host cell with DNA encoding functional fragments of either the light chain and/or the heavy chain of an antibody. Recombinant DNA technology may also be used to remove some, or all, of the DNA encoding either or both of the light and heavy chains that is not necessary for binding to the antigens of interest. The molecules expressed from such truncated DNA molecules are also encompassed by the antibodies. In addition, bifunctional antibodies may be produced in which one heavy and one light chain are an antibody (i.e., binds human GFAP) and the other heavy and light chain are specific for an antigen other than human GFAP by crosslinking an antibody to a second antibody by standard chemical crosslinking methods.

In a preferred system for recombinant expression of an antibody, or antigen-binding portion thereof, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells, and recover the antibody from the culture medium. Still further, the method of synthesizing a recombinant antibody may be by culturing a host cell in a suitable culture medium until a recombinant antibody is synthesized. The method can further comprise isolating the recombinant antibody from the culture medium.

Methods of preparing monoclonal antibodies involve the preparation of immortal cell lines capable of producing antibodies having the desired specificity. Such cell lines may be produced from spleen cells obtained from an immunized animal. The animal may be immunized with GFAP or a fragment and/or variant thereof. The peptide used to immunize the animal may comprise amino acids encoding human Fc, for example the fragment crystallizable region or tail region of human antibody. The spleen cells may then be immortalized by, for example, fusion with a myeloma cell fusion partner. A variety of fusion techniques may be employed. For example, the spleen cells and myeloma cells may be combined with a nonionic detergent for a few minutes and then plated at low density on a selective medium that supports that growth of hybrid cells, but not myeloma cells. One such technique uses hypoxanthine, aminopterin, thymidine (HAT) selection. Another technique includes electrofusion. After a sufficient time, usually about 1 to 2 weeks, colonies of hybrids are observed. Single colonies are selected and their culture supernatants tested for binding activity against the polypeptide. Hybridomas having high reactivity and specificity may be used.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. Affinity chromatography is an example of a method that can be used in a process to purify the antibodies.

The proteolytic enzyme papain preferentially cleaves IgG molecules to yield several fragments, two of which (the F(ab) fragments) each comprise a covalent heterodimer that includes an intact antigen-binding site. The enzyme pepsin is able to cleave IgG molecules to provide several fragments, including the $F(ab')_2$ fragment, which comprises both antigen-binding sites.

The Fv fragment can be produced by preferential proteolytic cleavage of an IgM, and on rare occasions IgG or IgA immunoglobulin molecules. The Fv fragment may be derived using recombinant techniques. The Fv fragment includes a non-covalent VH::VL heterodimer including an antigen-binding site that retains much of the antigen recognition and binding capabilities of the native antibody molecule.

The antibody, antibody fragment, or derivative may comprise a heavy chain and a light chain complementarity determining region ("CDR") set, respectively interposed between a heavy chain and a light chain framework ("FR") set which provide support to the CDRs and define the spatial relationship of the CDRs relative to each other. The CDR set may contain three hypervariable regions of a heavy or light chain V region.

Other suitable methods of producing or isolating antibodies of the requisite specificity can be used, including, but not limited to, methods that select recombinant antibody from a peptide or protein library (e.g., but not limited to, a bacteriophage, ribosome, oligonucleotide, RNA, cDNA, yeast or the like, display library); e.g., as available from various commercial vendors such as Cambridge Antibody Technologies (Cambridgeshire, UK), MorphoSys (Martinsried/Pla-negg, Del.), Biovation (Aberdeen, Scotland, UK) BioInvent (Lund, Sweden), using methods known in the art. See U.S. Patent Nos. 4,704,692; 5,723,323; 5,763,192; 5,814,476; 5,817,483; 5,824,514; 5,976,862. Alternative methods rely upon immunization of transgenic animals (e.g., SCID mice, Nguyen et al. (1997) Microbiol. Immunol. 41:901-907; Sandhu et al. (1996) Crit. Rev. Biotechnol. 16:95-118; Eren et al. (1998) Immunol. 93:154-161) that are capable of producing a repertoire of human antibodies, as known in the art and/or as described herein. Such techniques, include, but are not limited to, ribosome display (Hanes et al. (1997) Proc. Natl. Acad. Sci. USA, 94:4937-4942; Hanes et al. (1998) Proc. Natl. Acad. Sci. USA, 95:14130-14135); single cell antibody producing technologies (e.g., selected lymphocyte antibody method ("SLAM") (U.S. Patent No. 5,627,052, Wen et al. (1987) J. Immunol. 17:887-892; Babcook et al. (1996) Proc. Natl. Acad. Sci. USA 93:7843-7848); gel microdroplet and flow cytometry (Powell et al. (1990) Biotechnol. 8:333-337; One Cell Systems, (Cambridge, Mass).; Gray et al. (1995) J. Imm. Meth. 182:155-163; Kenny et al. (1995) Bio/Technol. 13:787-790); B-cell selection (Steenbakkers et al. (1994) Molec. Biol. Reports 19:125-134 (1994)).

An affinity matured antibody may be produced by any one of a number of procedures that are known in the art. For example, see Marks et al., BioTechnology, 10: 779-783 (1992) describes affinity maturation by VH and VL domain shuffling. Random mutagenesis of CDR and/or framework residues is described by Barbas et al., Proc. Nat. Acad. Sci. USA, 91: 3809-3813 (1994); Schier et al., Gene, 169: 147-155 (1995); Yelton et al., J. Immunol., 155: 1994-2004 (1995); Jackson et al., J. Immunol., 154(7): 3310-3319 (1995); Hawkins et al, J. Mol. Biol., 226: 889-896 (1992). Selective mutation at selective mutagenesis positions and at contact or hypermutation positions with an activity enhancing amino acid residue is described in U.S. Patent No. 6,914,128 B1.

Antibody variants can also be prepared using delivering a polynucleotide encoding an antibody to a suitable host such as to provide transgenic animals or mammals, such as goats, cows, horses, sheep, and the like, that produce such antibodies in their milk. These methods are known in the art and are described for example in U.S. Patent Nos. 5,827,690; 5,849,992; 4,873,316; 5,849,992; 5,994,616; 5,565,362; and 5,304,489.

Antibody variants also can be prepared by delivering a polynucleotide to provide transgenic plants and cultured plant cells (e.g., but not limited to tobacco, maize, and duckweed) that produce such antibodies, specified portions or variants in the plant parts or in cells cultured therefrom. For example, Cramer et al. (1999) Curr. Top. Microbiol. Immunol. 240:95-118 and references cited therein, describe the production of transgenic tobacco leaves expressing large amounts of recombinant proteins, e.g., using an inducible promoter. Transgenic maize have been used to express mammalian proteins at commercial production levels, with biological activities equivalent to those produced in other recombinant systems or purified from natural sources. See, e.g., Hood et al., Adv. Exp. Med. Biol. (1999) 464:127-147 and references cited therein. Antibody variants have also been produced in large amounts from transgenic plant seeds including antibody fragments, such as single chain antibodies (scFv's), including tobacco seeds and potato tubers. See, e.g., Conrad et al. (1998) Plant Mol. Biol. 38:101-109 and reference cited therein. Thus, antibodies can also be produced using transgenic plants, according to known methods.

Antibody derivatives can be produced, for example, by adding exogenous sequences to modify immunogenicity or reduce, enhance or modify binding, affinity, on-rate, off-rate, avidity, specificity, half-life, or any other suitable characteristic. Generally, part or all of the non-human or human CDR sequences are maintained while the non-human sequences of the variable and constant regions are replaced with human or other amino acids.

Small antibody fragments may be diabodies having two antigen-binding sites, wherein fragments comprise a heavy chain variable domain (VH) connected to a light chain variable domain (VL) in the same polypeptide chain (VH VL). See for example, EP 404,097; WO 93/11161; and Hollinger et al., (1993) Proc. Natl. Acad. Sci. USA 90:6444-6448. By using a linker that is too short to allow pairing between the two domains on the same chain, the domains are forced to pair with the complementary domains of another chain and create two antigen-binding sites. See also, U.S. Patent No. 6,632,926 to Chen et al. which is hereby incorporated by reference in its entirety and discloses antibody variants that have one or more amino acids inserted into a hypervariable region of the parent antibody and a binding affinity for a target antigen which is at least about two fold stronger than the binding affinity of the parent antibody for the antigen.

The antibody may be a linear antibody. The procedure for making a linear antibody is known in the art and described in Zapata et al. (1995) Protein Eng. 8(10):1057-1062. Briefly, these antibodies comprise a pair of tandem Fd segments (VH-CH1-VH-CH1) which form a pair of antigen binding regions. Linear antibodies can be bispecific or monospecific.

The antibodies may be recovered and purified from recombinant cell cultures by known methods including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. High performance liquid chromatography ("HPLC") can also be used for purification.

It may be useful to detectably label the antibody. Methods for conjugating antibodies to these agents are known in the art. For the purpose of illustration only, antibodies can be labeled with a detectable moiety such as a radioactive atom, a chromophore, a fluorophore, or the like. Such labeled antibodies can be used for diagnostic techniques, either in vivo, or in an isolated test sample. They can be linked to a cytokine, to a ligand, to another antibody. Suitable agents for coupling to antibodies to achieve an anti-tumor effect include cytokines, such as interleukin 2 (IL-2) and Tumor Necrosis Factor (TNF); photosensitizers, for use in photodynamic therapy, including aluminum (III) phthalocyanine tetrasulfonate, hematoporphyrin, and phthalocyanine; radionuclides, such as iodine-131 (1311), yttrium-90 (90Y), bismuth-212 (212Bi), bismuth-213 (213Bi), technetium-99m (99mTc), rhenium-186 (186Re), and rhenium-188 (188Re); antibiotics, such as doxorubicin, adriamycin, daunorubicin, methotrexate, daunomycin, neocarzinostatin, and carboplatin; bacterial, plant, and other toxins, such as diphtheria toxin, pseudomonas exotoxin A, staphylococcal enterotoxin A, abrin-A toxin, ricin A (deglycosylated ricin A and native ricin A), TGF-alpha toxin, cytotoxin from chinese cobra (naja naja atra), and gelonin (a plant toxin); ribosome inactivating proteins from plants, bacteria and fungi, such as restrictocin (a ribosome inactivating protein produced by Aspergillus restrictus), saporin (a ribosome inactivating protein from Saponaria officinalis), and RNase; tyrosine kinase inhibitors; ly207702 (a difluorinated purine nucleoside); liposomes containing anti cystic agents (e.g., antisense oligonucleotides, plasmids which encode for toxins, methotrexate, etc.); and other antibodies or antibody fragments, such as F(ab).

Antibody production via the use of hybridoma technology, the selected lymphocyte antibody method (SLAM), transgenic animals, and recombinant antibody libraries is described in more detail below.


**(1) Anti-GFAP Monoclonal Antibodies Using Hybridoma Technology**


Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, second edition, (Cold Spring Harbor Laboratory Press, Cold Spring Harbor, 1988); Hammerling, et al., In Monoclonal Antibodies and T-Cell Hybridomas, (Elsevier, N.Y., 1981). It is also noted that the term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods of generating monoclonal antibodies as well as antibodies produced by the method may comprise culturing a hybridoma cell secreting an antibody of the invention wherein, preferably, the hybridoma is generated by fusing splenocytes isolated from an animal, e.g., a rat or a mouse, immunized with GFAP with myeloma cells and then screening the hybridomas resulting from the fusion for hybridoma clones that secrete an antibody able to bind a polypeptide of the invention. Briefly, rats can be immunized with a GFAP antigen. In a preferred embodiment, the GFAP antigen is administered with an adjuvant to stimulate the immune response. Such adjuvants include complete or incomplete Freund's adjuvant, RIBI (muramyl dipeptides) or ISCOM (immunostimulating complexes). Such adjuvants may protect the polypeptide from rapid dispersal by sequestering it in a local deposit, or they may contain substances that stimulate the host to secrete factors that are chemotactic for macrophages and other components of the immune system. Preferably, if a polypeptide is being administered, the immunization schedule will involve two or more administrations of the polypeptide, spread out over several weeks; however, a single administration of the polypeptide may also be used.

After immunization of an animal with a GFAP antigen, antibodies and/or antibody-producing cells may be obtained from the animal. An anti-GFAP antibody-containing serum is obtained from the animal by bleeding or sacrificing the animal. The serum may be used as it is obtained from the animal, an immunoglobulin fraction may be obtained from the serum, or the anti-GFAP antibodies may be purified from the serum. Serum or immunoglobulins obtained in this manner are polyclonal, thus having a heterogeneous array of properties.

Once an immune response is detected, e.g., antibodies specific for the antigen GFAP are detected in the rat serum, the rat spleen is harvested and splenocytes isolated. The splenocytes are then fused by well-known techniques to any suitable myeloma cells, for example, cells from cell line SP20 available from the American Type Culture Collection (ATCC, Manassas, Va., US). Hybridomas are selected and cloned by limited dilution. The hybridoma clones are then assayed by methods known in the art for cells that secrete antibodies capable of binding GFAP. Ascites fluid, which generally contains high levels of antibodies, can be generated by immunizing rats with positive hybridoma clones.

In another embodiment, antibody-producing immortalized hybridomas may be prepared from the immunized animal. After immunization, the animal is sacrificed and the splenic B cells are fused to immortalized myeloma cells as is well known in the art. See, e.g., Harlow and Lane, supra. In a preferred embodiment, the myeloma cells do not secrete immunoglobulin polypeptides (a non-secretory cell line). After fusion and antibiotic selection, the hybridomas are screened using GFAP, or a portion thereof, or a cell expressing GFAP. In a preferred embodiment, the initial screening is performed using an enzyme-linked immunosorbent assay (ELISA) or a radioimmunoassay (RIA), preferably an ELISA. An example of ELISA screening is provided in PCT Publication No. WO 00/37504.

Anti-GFAP antibody-producing hybridomas are selected, cloned, and further screened for desirable characteristics, including robust hybridoma growth, high antibody production, and desirable antibody characteristics. Hybridomas may be cultured and expanded in vivo in syngeneic animals, in animals that lack an immune system, e.g., nude mice, or in cell culture in vitro. Methods of selecting, cloning and expanding hybridomas are well known to those of ordinary skill in the art.

In a preferred embodiment, hybridomas are rat hybridomas. In another embodiment, hybridomas are produced in a non-human, non-rat species such as mice, sheep, pigs, goats, cattle, or horses. In yet another preferred embodiment, the hybridomas are human hybridomas, in which a human non-secretory myeloma is fused with a human cell expressing an anti-GFAP antibody.

Antibody fragments that recognize specific epitopes may be generated by known techniques. For example, Fab and F(ab')$_2$ fragments of the invention may be produced by proteolytic cleavage of immunoglobulin molecules, using enzymes such as papain (to produce two identical Fab fragments) or pepsin (to produce an F(ab')$_2$ fragment). A F(ab')$_2$ fragment of an IgG molecule retains the two antigen-binding sites of the larger ("parent") IgG molecule, including both light chains (containing the variable light chain and constant light chain regions), the CH1 domains of the heavy chains, and a disulfide-forming hinge region of the parent IgG molecule. Accordingly, an F(ab')$_2$ fragment is still capable of crosslinking antigen molecules like the parent IgG molecule.

### (2) Anti-GFAP Monoclonal Antibodies Using SLAM

In another aspect of the invention, recombinant antibodies are generated from single, isolated lymphocytes using a procedure referred to in the art as the selected lymphocyte antibody method (SLAM), as described in U.S. Patent No. 5,627,052; PCT Publication No. WO 92/02551; and Babcook et al., Proc. Natl. Acad. Sci. USA, 93: 7843-7848 (1996). In this method, single cells secreting antibodies of interest, e.g., lymphocytes derived from any one of the immunized animals are screened using an antigen-specific hemolytic plaque assay, wherein the antigen GFAP, a subunit of GFAP, or a fragment thereof, is coupled to sheep red blood cells using a linker, such as biotin, and used to identify single cells that secrete antibodies with specificity for GFAP. Following identification of antibody-secreting cells of interest, heavy- and light-chain variable region cDNAs are rescued from the cells by reverse transcriptase-PCR (RT-PCR) and these variable regions can then be expressed, in the context of appropriate immunoglobulin constant regions (e.g., human constant regions), in mammalian host cells, such as COS or CHO cells. The host cells transfected with the amplified immunoglobulin sequences, derived from in vivo selected lymphocytes, can then undergo further analysis and selection in vitro, for example, by panning the transfected cells to isolate cells expressing antibodies to GFAP. The amplified immunoglobulin sequences further can be manipulated in vitro, such as by in vitro affinity maturation method. See, for example, PCT Publication No. WO 97/29131 and PCT Publication No. WO 00/56772.

### (3) Anti-GFAP Monoclonal Antibodies Using Transgenic Animals

In another embodiment of the invention, antibodies are produced by immunizing a non-human animal comprising some, or all, of the human immunoglobulin locus with a GFAP antigen. In an embodiment, the non-human animal is a XENOMOUSE® transgenic mouse, an engineered mouse strain that comprises large fragments of the human immunoglobulin loci and is deficient in mouse antibody production. See, e.g., Green et al., Nature Genetics, 7: 13-21 (1994) and U.S. Patent Nos. 5,916,771; 5,939,598; 5,985,615; 5,998,209; 6,075,181; 6,091,001; 6,114,598; and 6,130,364. See also PCT Publication Nos. WO 91/10741; WO 94/02602; WO 96/34096; WO 96/33735; WO 98/16654; WO 98/24893; WO 98/50433; WO 99/45031; WO 99/53049; WO 00/09560; and WO 00/37504. The XENOMOUSE® transgenic mouse produces an adult-like human repertoire of fully human antibodies, and generates antigen-specific human monoclonal antibodies. The XENOMOUSE® transgenic mouse contains approximately 80% of the human antibody repertoire through introduction of megabase sized, germline configuration YAC fragments of the human heavy chain loci and x light chain loci. See Mendez et al., Nature Genetics, 15: 146-156 (1997), Green and Jakobovits, J. Exp. Med., 188: 483-495 (1998), the disclosures of which are hereby incorporated by reference.

### (4) Anti-GFAP Monoclonal Antibodies Using Recombinant Antibody Libraries

In vitro methods also can be used to make the antibodies of the invention, wherein an antibody library is screened to identify an antibody having the desired GFAP -binding specificity. Methods for such screening of recombinant antibody libraries are well known in the art and include methods described in, for example, U.S. Patent No. 5,223,409 (Ladner et al.); PCT Publication No. WO 92/18619 (Kang et al.); PCT Publication No. WO 91/17271 (Dower et al.); PCT Publication No. WO 92/20791 (Winter et al.); PCT Publication No. WO 92/15679 (Markland et al.); PCT Publication No. WO 93/01288 (Breitling et al.); PCT Publication No. WO 92/01047 (McCafferty et al.); PCT Publication No. WO 92/09690 (Garrard et al.); Fuchs et al., Bio/Technology, 9: 1369-1372 (1991); Hay et al., Hum. Antibod. Hybridomas, 3: 81-85 (1992); Huse et al., Science, 246: 1275-1281 (1989); McCafferty et al., Nature, 348: 552-554 (1990); Griffiths et al., EMBO J., 12: 725-734 (1993); Hawkins et al., J. Mol. Biol., 226: 889-896 (1992); Clackson et al., Nature, 352: 624-628 (1991); Gram et al., Proc. Natl. Acad. Sci. USA, 89: 3576-3580 (1992); Garrard et al., Bio/Technology, 9: 1373-1377 (1991); Hoogenboom et al., Nucl. Acids Res., 19: 4133-4137 (1991); Barbas et al., Proc. Natl. Acad. Sci. USA, 88: 7978-7982 (1991); U.S. Patent Application Publication No. 2003/0186374; and PCT Publication No. WO 97/29131, the contents of each of which are incorporated herein by reference.

The recombinant antibody library may be from a subject immunized with GFAP, or a portion of GFAP. Alternatively, the recombinant antibody library may be from a naive subject, i.e., one who has not been immunized with GFAP, such as a human antibody library from a human subject who has not been immunized with human GFAP. Antibodies of the invention are selected by screening the recombinant antibody library with the peptide comprising human GFAP to thereby select those antibodies that recognize GFAP. Methods for conducting such screening and selection are well known in the art, such as described in the references in the preceding paragraph. To select antibodies of the invention having particular binding affinities for GFAP, such as those that dissociate from human GFAP with a particular $K_{off}$ rate constant, the art-known method of surface plasmon resonance can be used to select antibodies having the desired $K_{off}$ rate constant. To select antibodies of the invention having a particular neutralizing activity for hGFAP, such as those with a particular $IC_{50}$, standard methods known in the art for assessing the inhibition of GFAP activity may be used.

In one aspect, the invention pertains to an isolated antibody, or an antigen-binding portion thereof, that binds human GFAP. Preferably, the antibody is a neutralizing antibody. In various embodiments, the antibody is a recombinant antibody or a monoclonal antibody.

For example, antibodies can also be generated using various phage display methods known in the art. In phage display methods, functional antibody domains are displayed on the surface of phage particles which carry the polynucleotide sequences encoding them. Such phage can be utilized to display antigen-binding domains expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage including fd and M13 binding domains expressed from phage with Fab, Fv, or disulfide stabilized Fv antibody domains recombinantly fused to either the phage gene III or gene VIII protein. Examples of phage display methods that can be used to make the antibodies include those disclosed in Brinkmann et al., J. Immunol. Methods, 182: 41-50 (1995); Ames et al., J. Immunol. Methods, 184:177-186 (1995); Kettleborough et al., Eur. J. Immunol., 24: 952-958 (1994); Persic et al., Gene, 187: 9-18 (1997); Burton et al., Advances in Immunology, 57: 191-280 (1994); PCT Publication No. WO 92/01047; PCT Publication Nos. WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743; and 5,969,108.

As described in the above references, after phage selection, the antibody coding regions from the phage can be isolated and used to generate whole antibodies including human antibodies or any other desired antigen binding fragment, and expressed in any desired host, including mammalian cells, insect cells, plant cells, yeast, and bacteria, e.g., as described in detail below. For example, techniques to recombinantly produce Fab, Fab', and F(ab')$_2$ fragments can also be employed using methods known in the art such as those disclosed in PCT publication No. WO 92/22324; Mullinax et al., BioTechniques, 12(6): 864-869 (1992); Sawai et al., Am. J. Reprod. Immunol., 34: 26-34 (1995); and Better et al., Science, 240: 1041-1043 (1988). Examples of techniques which can be used to produce single-chain Fvs and antibodies include those described in U.S. Patent Nos. 4,946,778 and 5,258,498; Huston et al., Methods in Enzymology, 203: 46-88 (1991); Shu et al., Proc. Natl. Acad. Sci. USA, 90: 7995-7999 (1993); and Skerra et al., Science, 240: 1038-1041 (1988).

Alternative to screening of recombinant antibody libraries by phage display, other methodologies known in the art for screening large combinatorial libraries can be applied to the identification of antibodies of the invention. One type of alternative expression system is one in which the recombinant antibody library is expressed as RNA-protein fusions, as described in PCT Publication No. WO 98/31700 (Szostak and Roberts), and in Roberts and Szostak, Proc. Natl. Acad. Sci. USA, 94: 12297-12302 (1997). In this system, a covalent fusion is created between an mRNA and the peptide or protein that it encodes by in vitro translation of synthetic mRNAs that carry puromycin, a peptidyl acceptor antibiotic, at their 3' end. Thus, a specific mRNA can be enriched from a complex mixture of mRNAs (e.g., a combinatorial library) based on the properties of the encoded peptide or protein, e.g., antibody, or portion thereof, such as binding of the antibody, or portion thereof, to the dual specificity antigen. Nucleic acid sequences encoding antibodies, or portions thereof, recovered from screening of such libraries can be expressed by recombinant means as described above (e.g., in mammalian host cells) and, moreover, can be subjected to further affinity maturation by either additional rounds of screening of mRNA-peptide fusions in which mutations have been introduced into the originally selected sequence(s), or by other methods for affinity maturation in vitro of recombinant antibodies, as described above. A preferred example of this methodology is PROfusion display technology.

In another approach, the antibodies can also be generated using yeast display methods known in the art. In yeast display methods, genetic methods are used to tether antibody domains to the yeast cell wall and display them on the surface of yeast. In particular, such yeast can be utilized to display antigen-binding domains expressed from a repertoire or combinatorial antibody library (e.g., human or murine). Examples of yeast display methods that can be used to make the antibodies include those disclosed in U.S. Patent No. 6,699,658 (Wittrup et al.) incorporated herein by reference.

### d. Production of Recombinant GFAP Antibodies

Antibodies may be produced by any of a number of techniques known in the art. For example, expression from host cells, wherein expression vector(s) encoding the heavy and light chains is (are) transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection, and the like. Although it is possible to express the antibodies of the invention in either prokaryotic or eukaryotic host cells, expression of antibodies in eukaryotic cells is preferable, and most preferable in mammalian host cells, because such eukaryotic cells (and in particular mammalian cells) are more likely than prokaryotic cells to assemble and secrete a properly folded and immunologically active antibody.

Exemplary mammalian host cells for expressing the recombinant antibodies of the invention include Chinese Hamster Ovary (CHO cells) (including dhfr-CHO cells, described in Urlaub and Chasin, Proc. Natl. Acad. Sci. USA, 77: 4216-4220 (1980), used with a DHFR selectable marker, e.g., as described in Kaufman and Sharp, J. Mol. Biol., 159: 601-621 (1982), NS0 myeloma cells, COS cells, and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce functional antibody fragments, such as Fab fragments or scFv molecules. It will be understood that variations on the above procedure may be performed. For example, it may be desirable to transfect a host cell with DNA encoding functional fragments of either the light chain and/or the heavy chain of an antibody of this invention. Recombinant DNA technology may also be used to remove some, or all, of the DNA encoding either or both of the light and heavy chains that is not necessary for binding to the antigens of interest. The molecules expressed from such truncated DNA molecules are also encompassed by the antibodies of the invention. In addition, bifunctional antibodies may be produced in which one heavy and one light chain are an antibody of the invention (i.e., binds human GFAP) and the other heavy and light chain are specific for an antigen other than human GF AP by crosslinking an antibody of the invention to a second antibody by standard chemical crosslinking methods.

In a preferred system for recombinant expression of an antibody, or antigen-binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells, and recover the antibody from the culture medium. Still further, the invention provides a method of synthesizing a recombinant antibody of the invention by culturing a host cell of the invention in a suitable culture medium until a recombinant antibody of the invention is synthesized. The method can further comprise isolating the recombinant antibody from the culture medium.

## (1) Humanized Antibody

The humanized antibody may be an antibody or a variant, derivative, analog or portion thereof which immunospecifically binds to an antigen of interest and which comprises a framework (FR) region having substantially the amino acid sequence of a human antibody and a complementary determining region (CDR) having substantially the amino acid sequence of a non-human antibody. The humanized antibody may be from a non-human species antibody that binds the desired antigen having one or more complementarity determining regions (CDRs) from the non-human species and framework regions from a human immunoglobulin molecule.

As used herein, the term "substantially" in the context of a CDR refers to a CDR having an amino acid sequence at least 90%, at least 95%, at least 98% or at least 99% identical to the amino acid sequence of a non-human antibody CDR. A humanized antibody comprises substantially all of at least one, and typically two, variable domains (Fab, Fab', F(ab')$_2$, FabC, Fv) in which all or substantially all of the CDR regions correspond to those of a non-human immunoglobulin (i.e., donor antibody) and all or substantially all of the framework regions are those of a human immunoglobulin consensus sequence. According to one aspect, a humanized antibody also comprises at least a portion of an immunoglobulin constant region (Fc), typically that of a human immunoglobulin. In some embodiments, a humanized antibody contains both the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or of a heavy chain.

The humanized antibody can be selected from any class of immunoglobulins, including IgM, IgG, IgD, IgA and IgE, and any isotype, including without limitation IgG 1, IgG2, IgG3, and IgG4. The humanized antibody may comprise sequences from more than one class or isotype, and particular constant domains may be selected to optimize desired effector functions using techniques well-known in the art.

The framework and CDR regions of a humanized antibody need not correspond precisely to the parental sequences, e.g., the donor antibody CDR or the consensus framework may be mutagenized by substitution, insertion and/or deletion of at least one amino acid residue so that the CDR or framework residue at that site does not correspond to either the donor antibody or the consensus framework. In one embodiment, such mutations, however, will not be extensive. Usually, at least 90%, at least 95%, at least 98%, or at least 99% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (See e.g., Winnaker, From Genes to Clones (Verlagsgesellschaft, Weinheim, Germany 1987)). In a family of immunoglobulins, each position in the consensus sequence is occupied by the amino acid occurring most frequently at that position in the family. If two amino acids occur equally frequently, either can be included in the consensus sequence.

The humanized antibody may be designed to minimize unwanted immunological response toward rodent anti-human antibodies, which limits the duration and effectiveness of therapeutic applications of those moieties in human recipients. The humanized antibody may have one or more amino acid residues introduced into it from a source that is non-human. These non-human residues are often referred to as "import" residues, which are typically taken from a variable domain. Humanization may be performed by substituting hypervariable region sequences for the corresponding sequences of a human antibody. Accordingly, such "humanized" antibodies are chimeric antibodies wherein substantially less than an intact human variable domain has been substituted by the corresponding sequence from a non-human species. For example, see U.S. Patent No. 4,816,567, the contents of which are herein incorporated by reference. The humanized antibody may be a human antibody in which some hypervariable region residues, and possibly some FR residues are substituted by residues from analogous sites in rodent antibodies. Humanization or engineering of antibodies of the present disclosure can be performed using any known method, such as but not limited to those described in U.S. Patent Nos. 5,723,323; 5,976,862; 5,824,514; 5,817,483; 5,814,476; 5,763,192; 5,723,323; 5,766,886; 5,714,352; 6,204,023; 6,180,370; 5,693,762; 5,530,101; 5,585,089; 5,225,539; and 4,816,567.

The humanized antibody may retain high affinity for GFAP and other favorable biological properties. The humanized antibody may be prepared by a process of analysis of the parental sequences and various conceptual humanized products using three-dimensional models of the parental and humanized sequences. Three-dimensional immunoglobulin models are commonly available. Computer programs are available that illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristics, such as increased affinity for GFAP, is achieved. In general, the hypervariable region residues may be directly and most substantially involved in influencing antigen binding.

As an alternative to humanization, human antibodies (also referred to herein as "fully human antibodies") can be generated. For example, it is possible to isolate human antibodies from libraries via PROfusion and/or yeast related technologies. It is also possible to produce transgenic animals (e.g. mice that are capable, upon immunization, of producing a full repertoire of human antibodies in the absence of endogenous immunoglobulin production. For example, the homozygous deletion of the antibody heavy-chain joining region ($J_H$) gene in chimeric and germ-line mutant mice results in complete inhibition of endogenous antibody production. Transfer of the human germ-line immunoglobulin gene array in such germ-line mutant mice will result in the production of human antibodies upon antigen challenge. The humanized or fully human antibodies may be prepared according to the methods described in U.S. Patent Nos. 5,770,429; 5,833,985; 5,837,243; 5,922,845; 6,017,517, 6,096,311; 6,111,166; 6,270,765; 6,303,755; 6,365,116; 6,410,690; 6,682,928; and 6,984,720, the contents each of which are herein incorporated by reference.

### e. Anti-GFAP antibodies

Anti-GFAP antibodies may be generated using the techniques described above as well as using routine techniques known in the art. In some embodiments, the anti-GFAP antibody may be an unconjugated GFAP antibody, such as GFAP antibodies available from Dako (Catalog Number: M0761), ThermoFisher Scientific (Catalog Numbers: MA5-12023, A-21282, 13-0300, MA1-19170, MA1-19395, MA5-15086, MA5-16367, MA1-35377, MA1-06701, or MA1-20035), AbCam (Catalog Numbers: ab10062, ab4648, ab68428, ab33922, ab207165, ab190288, ab115898, or ab21837), EMD Millipore (Catalog Numbers: FCMAB257P, MAB360, MAB3402, 04-1031, 04-1062, MAB5628), Santa Cruz (Catalog Numbers: sc-166481, sc-166458, sc-58766, sc-56395, sc-51908, sc-135921, sc-71143, sc-65343, or sc-33673), Sigma-Aldrich (Catalog Numbers: G3893 or G6171) or Sino Biological Inc. (Catalog Number: 100140-R012-50). The anti-GFAP antibody may be conjugated to a fluorophore, such as conjugated GFAP antibodies available from ThermoFisher Scientific (Catalog Numbers: A-21295 or A-21294), EMD Millipore (Catalog Numbers: MAB3402X, MAB3402B, MAB3402B, or MAB3402C3) or AbCam (Catalog Numbers: ab49874 or ab194325).

### 5. An Improvement of a Method of Assessing a Subject's GFAP Status as a Measure of Traumatic Brain **Injury**

In yet another embodiment, the present disclosure is directed to an improvement of a method of assessing a subject's GFAP status as a measure of traumatic brain injury by assessing the presence or amount of GFAP in a biological sample. The improvement is that the method allows for the assay to measure up to 50,000 pg/mL of GFAP and does not require dilution of the biological sample. In some embodiments, if GFAP is the only biomarker being assessed, the improvement further includes using the method with a point-of-care device. The method is performed using a first specific binding member and the second specific binding member that each specifically bind to GFAP and form first complexes that includes the first specific binding member-GFAP-second specific binding member. In some embodiments, the second specific binding member is each labeled with a detectable label.

Other methods of detection include the use of or can be adapted for use on a nanopore device or nanowell device, e.g. for single molecule detection. Examples of nanopore devices are described in International Patent Publication No. WO 2016/161402, which is hereby incorporated by reference in its entirety. Examples of nanowell device are described in International Patent Publication No. WO 2016/161400, which is hereby incorporated by reference in its entirety. Other devices and methods appropriate for single molecule detection also can be employed.

### 6. Variations on Methods

The disclosed methods of determining the presence or amount of analyte of interest (GFAP) present in a sample may be as described herein. The methods may also be adapted in view of other methods for analyzing analytes. Examples of well-known variations include, but are not limited to, immunoassay, such as sandwich immunoassay (e.g., monoclonal-monoclonal sandwich immunoassays, monoclonal-polyclonal sandwich immunoassays, including enzyme detection (enzyme immunoassay (EIA) or enzyme-linked immunosorbent assay (ELISA), competitive inhibition immunoassay (e.g., forward and reverse), enzyme multiplied immunoassay technique (EMIT), a competitive binding assay, bioluminescence resonance energy transfer (BRET), one-step antibody detection assay, homogeneous assay, heterogeneous assay, capture on the fly assay, etc.

### a. Immunoassay

The analyte of interest, and/or peptides of fragments thereof (e.g., GFAP, and/or peptides or fragments thereof, i.e., GFAP fragments), may be analyzed using GFAP antibodies in an immunoassay. The presence or amount of analyte (e.g., GFAP) can be determined using antibodies and detecting specific binding to the analyte (e.g., GFAP). For example, the antibody, or antibody fragment thereof, may specifically bind to the analyte (e.g., GFAP). If desired, one or more of the antibodies can be used in combination with one or more commercially available monoclonal/polyclonal antibodies. Such antibodies are available from companies such as R&D Systems, Inc. (Minneapolis, MN) and Enzo Life Sciences International, Inc. (Plymouth Meeting, PA).

The presence or amount of analyte (e.g., GFAP) present in a body sample may be readily determined using an immunoassay, such as sandwich immunoassay (e.g., monoclonal-monoclonal sandwich immunoassays, monoclonal-polyclonal sandwich immunoassays, including radioisotope detection (radioimmunoassay (RIA)) and enzyme detection (enzyme immunoassay (EIA) or enzyme-linked immunosorbent assay (ELISA) (e.g., Quantikine ELISA assays, R&D Systems, Minneapolis, MN)). An example of a point-of-care device that can be used is i-STAT® (Abbott, Laboratories, Abbott Park, IL). Other methods that can be used include a chemiluminescent microparticle immunoassay, in particular one employing the ARCHITECT® automated analyzer (Abbott Laboratories, Abbott Park, IL), as an example. Other methods include, for example, mass spectrometry, and immunohistochemistry (e.g. with sections from tissue biopsies), using anti-analyte (e.g., anti-GFAP) antibodies (monoclonal, polyclonal, chimeric, humanized, human, etc.) or antibody fragments thereof against analyte (e.g., GFAP). Other methods of detection include those described in, for example, U.S. Patent Nos. 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, each of which is hereby incorporated by reference in its entirety. Specific immunological binding of the antibody to the analyte (e.g., GFAP) can be detected via direct labels, such as fluorescent or luminescent tags, metals and radionuclides attached to the antibody or via indirect labels, such as alkaline phosphatase or horseradish peroxidase.

The use of immobilized antibodies or antibody fragments thereof may be incorporated into the immunoassay. The antibodies may be immobilized onto a variety of supports, such as magnetic or chromatographic matrix particles, the surface of an assay plate (such as microtiter wells), pieces of a solid substrate material, and the like. An assay strip can be prepared by coating the antibody or plurality of antibodies in an array on a solid support. This strip can then be dipped into the test biological sample and processed quickly through washes and detection steps to generate a measurable signal, such as a colored spot.

A homogeneous format may be used. For example, after the test sample is obtained from a subject, a mixture is prepared. The mixture contains the test sample being assessed for analyte (e.g., GFAP), a first specific binding partner, and a second specific binding partner. The order in which the test sample, the first specific binding partner, and the second specific binding partner are added to form the mixture is not critical. The test sample is simultaneously contacted with the first specific binding partner and the second specific binding partner. In some embodiments, the first specific binding partner and any GFAP contained in the test sample may form a first specific binding partner-analyte (e.g., GFAP)-antigen complex and the second specific binding partner may form a first specific binding partner-analyte of interest (e.g., GFAP)-second specific binding partner complex. In some embodiments, the second specific binding partner and any GFAP contained in the test sample may form a second specific binding partner-analyte (e.g., GFAP)-antigen complex and the first specific binding partner may form a first specific binding partner-analyte of interest (e.g., GFAP)-second specific binding partner complex. The first specific binding partner may be an anti-analyte antibody (e.g., anti-GFAP antibody that binds to an epitope having an amino acid sequence comprising at least three contiguous (3) amino acids of SEQ ID NO: 1). The second specific binding partner may be an anti-analyte antibody (e.g., anti-GFAP antibody that binds to an epitope having an amino acid sequence comprising at least three contiguous (3) amino acids of SEQ ID NO: 1). Moreover, the second specific binding partner is labeled with or contains a detectable label as described above.

A heterogeneous format may be used. For example, after the test sample is obtained from a subject, a first mixture is prepared. The mixture contains the test sample being assessed for analyte (e.g., GFAP) and a first specific binding partner, wherein the first specific binding partner and any GFAP contained in the test sample form a first specific binding partner-analyte (e.g., GFAP)-antigen complex. The first specific binding partner may be an anti-analyte antibody (e.g., anti-GFAP antibody that binds to an epitope having an amino acid sequence comprising at least three contiguous (3) amino acids of SEQ ID NO: 1). The order in which the test sample and the first specific binding partner are added to form the mixture is not critical.

The first specific binding partner may be immobilized on a solid phase. The solid phase used in the immunoassay (for the first specific binding partner and, optionally, the second specific binding partner) can be any solid phase known in the art, such as, but not limited to, a magnetic particle, a bead, a test tube, a microtiter plate, a cuvette, a membrane, a scaffolding molecule, a film, a filter paper, a disc, and a chip. In those embodiments where the solid phase is a bead, the bead may be a magnetic bead or a magnetic particle. Magnetic beads/particles may be ferromagnetic, ferrimagnetic, paramagnetic, superparamagnetic or ferrofluidic. Exemplary ferromagnetic materials include Fe, Co, Ni, Gd, Dy, $CrO_2$, MnAs, MnBi, EuO, and NiO/Fe. Examples of ferrimagnetic materials include $NiFe_2O_4$, $CoFe_2O_4$, $Fe_3O_4$ (or $FeO^.Fe_2O_3$). Beads can have a solid core portion that is magnetic and is surrounded by one or more non-magnetic layers. Alternately, the magnetic portion can be a layer around a non-magnetic core. The solid support on which the first specific binding member is immobilized may be stored in dry form or in a liquid. The magnetic beads may be subjected to a magnetic field prior to or after contacting with the sample with a magnetic bead on which the first specific binding member is immobilized.

After the mixture containing the first specific binding partner-analyte (e.g., GFAP) antigen complex is formed, any unbound analyte (e.g., GFAP) is removed from the complex using any technique known in the art. For example, the unbound analyte (e.g., GFAP) can be removed by washing. Desirably, however, the first specific binding partner is present in excess of any analyte (e.g., GFAP) present in the test sample, such that all analyte (e.g., GFAP) that is present in the test sample is bound by the first specific binding partner.

After any unbound analyte (e.g., GFAP) is removed, a second specific binding partner is added to the mixture to form a first specific binding partner-analyte of interest (e.g., GFAP)-second specific binding partner complex. The second specific binding partner may be an anti-analyte antibody (e.g., anti-GF AP antibody that binds to an epitope having an amino acid sequence comprising at least three contiguous (3) amino acids of SEQ ID NO: 1). Moreover, the second specific binding partner is labeled with or contains a detectable label as described above.

The use of immobilized antibodies or antibody fragments thereof may be incorporated into the immunoassay. The antibodies may be immobilized onto a variety of supports, such as magnetic or chromatographic matrix particles (such as a magnetic bead), latex particles or modified surface latex particles, polymer or polymer film, plastic or plastic film, planar substrate, the surface of an assay plate (such as microtiter wells), pieces of a solid substrate material, and the like. An assay strip can be prepared by coating the antibody or plurality of antibodies in an array on a solid support. This strip can then be dipped into the test biological sample and processed quickly through washes and detection steps to generate a measurable signal, such as a colored spot.

### (1) Sandwich immunoassay

A sandwich immunoassay measures the amount of antigen between two layers of antibodies *(i.e.,* at least one capture antibody) and a detection antibody (i.e. at least one detection antibody). The capture antibody and the detection antibody bind to different epitopes on the antigen, e.g., analyte of interest such as GFAP. Desirably, binding of the capture antibody to an epitope does not interfere with binding of the detection antibody to an epitope. Either monoclonal or polyclonal antibodies may be used as the capture and detection antibodies in the sandwich immunoassay.

Generally, at least two antibodies are employed to separate and quantify analyte (e.g., GFAP) in a test sample. More specifically, the at least two antibodies bind to certain epitopes of analyte (e.g., GFAP) forming an immune complex which is referred to as a "sandwich". One or more antibodies can be used to capture the analyte (e.g., GFAP) in the test sample (these antibodies are frequently referred to as a "capture" antibody or "capture" antibodies) and one or more antibodies is used to bind a detectable (namely, quantifiable) label to the sandwich (these antibodies are frequently referred to as the "detection" antibody or "detection" antibodies). In a sandwich assay, the binding of an antibody to its epitope desirably is not diminished by the binding of any other antibody in the assay to its respective epitope. Antibodies are selected so that the one or more first antibodies brought into contact with a test sample suspected of containing analyte (e.g., GFAP) do not bind to all or part of an epitope recognized by the second or subsequent antibodies, thereby interfering with the ability of the one or more second detection antibodies to bind to the analyte (e.g., GFAP).

The antibodies may be used as a first antibody in said immunoassay. The antibody immunospecifically binds to epitopes on analyte (e.g., GFAP). In addition to the antibodies of the present disclosure, said immunoassay may comprise a second antibody that immunospecifically binds to epitopes that are not recognized or bound by the first antibody.

A test sample suspected of containing analyte (e.g., GFAP) can be contacted with at least one first capture antibody (or antibodies) and at least one second detection antibodies either simultaneously or sequentially. In the sandwich assay format, a test sample suspected of containing analyte (e.g., GFAP) is first brought into contact with the at least one first capture antibody that specifically binds to a particular epitope under conditions which allow the formation of a first antibody-analyte (e.g., GFAP) antigen complex. If more than one capture antibody is used, a first multiple capture antibody-GFAP antigen complex is formed. In a sandwich assay, the antibodies, preferably, the at least one capture antibody, are used in molar excess amounts of the maximum amount of analyte (e.g., GFAP) expected in the test sample.

For example, from about 5 µg/mL to about 1 mg/mL of antibody per ml of microparticle coating buffer may be used.

### (a) Anti-GFAP Capture Antibody

Optionally, prior to contacting the test sample with the at least one first capture antibody, the at least one first capture antibody can be bound to a solid support which facilitates the separation the first antibody-analyte (e.g., GFAP) complex from the test sample. Any solid support known in the art can be used, including but not limited to, solid supports made out of polymeric materials in the forms of wells, tubes, or beads (such as a microparticle). The antibody (or antibodies) can be bound to the solid support by adsorption, by covalent bonding using a chemical coupling agent or by other means known in the art, provided that such binding does not interfere with the ability of the antibody to bind analyte (e.g., GFAP). Moreover, if necessary, the solid support can be derivatized to allow reactivity with various functional groups on the antibody. Such derivatization requires the use of certain coupling agents such as, but not limited to, maleic anhydride, N-hydroxysuccinimide and 1-ethyl-3-(3-dimethylaminopropyl)carbodiimide.

After the test sample suspected of containing analyte (e.g., GFAP) is incubated in order to allow for the formation of a first capture antibody (or multiple antibody)-analyte (e.g., GFAP) complex. The incubation can be carried out at a pH of from about 4.5 to about 10.0, at a temperature of from about 2°C to about 45°C, and for a period from at least about one (1) minute to about eighteen (18) hours, from about 2-6 minutes, from about 7 -12 minutes, from about 5-15 minutes, or from about 3-4 minutes.

### (b) Detection Antibody

After formation of the first/multiple capture antibody-analyte (e.g., GFAP) complex, the complex is then contacted with at least one second detection antibody (under conditions that allow for the formation of a first/multiple antibody-analyte (e.g., GFAP) antigen-second antibody complex). In some embodiments, the test sample is contacted with the detection antibody simultaneously with the capture antibody. If the first antibody-analyte (e.g., GFAP) complex is contacted with more than one detection antibody, then a first/multiple capture antibody-analyte (e.g., GFAP)-multiple antibody detection complex is formed. As with first antibody, when the at least second (and subsequent) antibody is brought into contact with the first antibody-analyte (e.g., GFAP) complex, a period of incubation under conditions similar to those described above is required for the formation of the first/multiple antibody-analyte (e.g., GFAP)-second/multiple antibody complex. Preferably, at least one second antibody contains a detectable label. The detectable label can be bound to the at least one second antibody prior to, simultaneously with or after the formation of the first/multiple antibody-analyte (e.g., GFAP)-second/multiple antibody complex. Any detectable label known in the art can be used.

Chemiluminescent assays can be performed in accordance with the methods described in Adamczyk et al., Anal. Chim. Acta 579(1): 61-67 (2006). While any suitable assay format can be used, a microplate chemiluminometer (Mithras LB-940, Berthold Technologies U.S.A., LLC, Oak Ridge, TN) enables the assay of multiple samples of small volumes rapidly. The chemiluminometer can be equipped with multiple reagent injectors using 96-well black polystyrene microplates (Costar #3792). Each sample can be added into a separate well, followed by the simultaneous/sequential addition of other reagents as determined by the type of assay employed. Desirably, the formation of pseudobases in neutral or basic solutions employing an acridinium aryl ester is avoided, such as by acidification. The chemiluminescent response is then recorded well-by-well. In this regard, the time for recording the chemiluminescent response will depend, in part, on the delay between the addition of the reagents and the particular acridinium employed.

The order in which the test sample and the specific binding partner(s) are added to form the mixture for chemiluminescent assay is not critical. If the first specific binding partner is detectably labeled with an acridinium compound, detectably labeled first specific binding partner-GFAP antigen complexes form. Alternatively, if a second specific binding partner is used and the second specific binding partner is detectably labeled with an acridinium compound, detectably labeled first specific binding partner-analyte (e.g., GFAP)-second specific binding partner complexes form. Any unbound specific binding partner, whether labeled or unlabeled, can be removed from the mixture using any technique known in the art, such as washing.

Hydrogen peroxide can be generated in situ in the mixture or provided or supplied to the mixture before, simultaneously with, or after the addition of an above-described acridinium compound. Hydrogen peroxide can be generated in situ in a number of ways such as would be apparent to one skilled in the art.

Alternatively, a source of hydrogen peroxide can be simply added to the mixture. For example, the source of the hydrogen peroxide can be one or more buffers or other solutions that are known to contain hydrogen peroxide. In this regard, a solution of hydrogen peroxide can simply be added.

Upon the simultaneous or subsequent addition of at least one basic solution to the sample, a detectable signal, namely, a chemiluminescent signal, indicative of the presence of GFAP is generated. The basic solution contains at least one base and has a pH greater than or equal to 10, preferably, greater than or equal to 12. Examples of basic solutions include, but are not limited to, sodium hydroxide, potassium hydroxide, calcium hydroxide, ammonium hydroxide, magnesium hydroxide, sodium carbonate, sodium bicarbonate, calcium hydroxide, calcium carbonate, and calcium bicarbonate. The amount of basic solution added to the sample depends on the concentration of the basic solution. Based on the concentration of the basic solution used, one skilled in the art can easily determine the amount of basic solution to add to the sample. Other labels other than chemiluminescent labels can be employed. For instance, enzymatic labels (including but not limited to alkaline phosphatase) can be employed.

The chemiluminescent signal, or other signal, that is generated can be detected using routine techniques known to those skilled in the art. Based on the intensity of the signal generated, the amount of analyte of interest (e.g., GFAP) in the sample can be quantified. Specifically, the amount of analyte (e.g., GFAP) in the sample is proportional to the intensity of the signal generated. The amount of analyte (e.g., GFAP) present can be quantified by comparing the amount of light generated to a standard curve for analyte (e.g., GFAP) or by comparison to a reference standard. The standard curve can be generated using serial dilutions or solutions of known concentrations of analyte (e.g., GFAP) by mass spectroscopy, gravimetric methods, and other techniques known in the art.

### (2) Forward Competitive Inhibition Assay

In a forward competitive format, an aliquot of labeled analyte of interest (e.g., analyte having a fluorescent label, a tag attached with a cleavable linker, etc.) of a known concentration is used to compete with analyte of interest in a test sample for binding to analyte of interest antibody.

In a forward competition assay, an immobilized specific binding partner (such as an antibody) can either be sequentially or simultaneously contacted with the test sample and a labeled analyte of interest, analyte of interest fragment or analyte of interest variant thereof. The analyte of interest peptide, analyte of interest fragment or analyte of interest variant can be labeled with any detectable label, including a detectable label comprised of tag attached with a cleavable linker. In this assay, the antibody can be immobilized on to a solid support. Alternatively, the antibody can be coupled to an antibody, such as an antispecies antibody, that has been immobilized on a solid support, such as a microparticle or planar substrate.

The labeled analyte of interest, the test sample and the antibody are incubated under conditions similar to those described above in connection with the sandwich assay format. Two different species of antibody-analyte of interest complexes may then be generated. Specifically, one of the antibody-analyte of interest complexes generated contains a detectable label (e.g., a fluorescent label, etc.) while the other antibody-analyte of interest complex does not contain a detectable label. The antibody-analyte of interest complex can be, but does not have to be, separated from the remainder of the test sample prior to quantification of the detectable label. Regardless of whether the antibody-analyte of interest complex is separated from the remainder of the test sample, the amount of detectable label in the antibody-analyte of interest complex is then quantified. The concentration of analyte of interest (such as membrane-associated analyte of interest, soluble analyte of interest, fragments of soluble analyte of interest, variants of analyte of interest (membrane-associated or soluble analyte of interest) or any combinations thereof) in the test sample can then be determined, e.g., as described above.

### (3) Reverse Competitive Inhibition Assay

In a reverse competition assay, an immobilized analyte of interest can either be sequentially or simultaneously contacted with a test sample and at least one labeled antibody.

The analyte of interest can be bound to a solid support, such as the solid supports discussed above in connection with the sandwich assay format.

The immobilized analyte of interest, test sample and at least one labeled antibody are incubated under conditions similar to those described above in connection with the sandwich assay format. Two different species analyte of interest-antibody complexes are then generated. Specifically, one of the analyte of interest-antibody complexes generated is immobilized and contains a detectable label (e.g., a fluorescent label, etc.) while the other analyte of interest-antibody complex is not immobilized and contains a detectable label. The non-immobilized analyte of interest-antibody complex and the remainder of the test sample are removed from the presence of the immobilized analyte of interest-antibody complex through techniques known in the art, such as washing. Once the non-immobilized analyte of interest antibody complex is removed, the amount of detectable label in the immobilized analyte of interest-antibody complex is then quantified following cleavage of the tag. The concentration of analyte of interest in the test sample can then be determined by comparing the quantity of detectable label as described above.

### (4) One-Step Immunoassay or "Capture on the Fly" Assay

In a capture on the fly immunoassay, a solid substrate is pre-coated with an immobilization agent. The capture agent, the analyte and the detection agent are added to the solid substrate together, followed by a wash step prior to detection. The capture agent can bind the analyte and comprises a ligand for an immobilization agent. The capture agent and the detection agents may be antibodies or any other moiety capable of capture or detection as described herein or known in the art. The ligand may comprise a peptide tag and an immobilization agent may comprise an anti-peptide tag antibody. Alternately, the ligand and the immobilization agent may be any pair of agents capable of binding together so as to be employed for a capture on the fly assay (e.g., specific binding pair, and others such as are known in the art). More than one analyte may be measured. In some embodiments, the solid substrate may be coated with an antigen and the analyte to be analyzed is an antibody.

In certain other embodiments, in a one-step immunoassay or "capture on the fly", a solid support (such as a microparticle) pre-coated with an immobilization agent (such as biotin, streptavidin, etc.) and at least a first specific binding member and a second specific binding member (which function as capture and detection reagents, respectively) are used. The first specific binding member comprises a ligand for the immobilization agent (for example, if the immobilization agent on the solid support is streptavidin, the ligand on the first specific binding member may be biotin) and also binds to the analyte of interest. The second specific binding member comprises a detectable label and binds to an analyte of interest. The solid support and the first and second specific binding members may be added to a test sample (either sequentially or simultaneously). The ligand on the first specific binding member binds to the immobilization agent on the solid support to form a solid support/first specific binding member complex. Any analyte of interest present in the sample binds to the solid support/first specific binding member complex to form a solid support/first specific binding member/analyte complex. The second specific binding member binds to the solid support/first specific binding member/analyte complex and the detectable label is detected. An optional wash step may be employed before the detection. In certain embodiments, in a one-step assay more than one analyte may be measured. In certain other embodiments, more than two specific binding members can be employed. In certain other embodiments, multiple detectable labels can be added. In certain other embodiments, multiple analytes of interest can be detected, or their amounts, levels or concentrations, measured, determined or assessed.

The use of a capture on the fly assay can be done in a variety of formats as described herein, and known in the art. For example the format can be a sandwich assay such as described above, but alternately can be a competition assay, can employ a single specific binding member, or use other variations such as are known.

### 7. Samples

### a. Test or Biological Sample

As used herein, "sample", "test sample", "biological sample" refer to fluid sample containing or suspected of containing GFAP. The sample may be derived from any suitable source. In some cases, the sample may comprise a liquid, fluent particulate solid, or fluid suspension of solid particles. In some cases, the sample may be processed prior to the analysis described herein. For example, the sample may be separated or purified from its source prior to analysis; however, in certain embodiments, an unprocessed sample containing the analyte may be assayed directly. The source of the analyte molecule may be synthetic (e.g., produced in a laboratory), the environment (e.g., air, soil, fluid samples, e.g., water supplies, etc.), an animal, e.g., a mammal, a plant, or any combination thereof. In a particular example, the source of an analyte is a human bodily substance (e.g., bodily fluid, blood, such as whole blood, serum, plasma, urine, saliva, sweat, sputum, semen, mucus, lacrimal fluid, lymph fluid, amniotic fluid, interstitial fluid, lung lavage, cerebrospinal fluid, feces, tissue, organ, or the like). Tissues may include, but are not limited to skeletal muscle tissue, liver tissue, lung tissue, kidney tissue, myocardial tissue, brain tissue, bone marrow, cervix tissue, skin, etc. The sample may be a liquid sample or a liquid extract of a solid sample. In certain cases, the source of the sample may be an organ or tissue, such as a biopsy sample, which may be solubilized by tissue disintegration/cell lysis.

A wide range of volumes of the fluid sample may be analyzed. In a few exemplary embodiments, the sample volume may be about 0.5 nL, about 1 nL, about 3 nL, about 0.01 $\mu$L, about 0.1 $\mu$L, about 1 $\mu$L, about 5 $\mu$L, about 10 $\mu$L, about 100 $\mu$L, about 1 mL, about 5 mL, about 10 mL, or the like. In some cases, the volume of the fluid sample is between about 0.01 $\mu$L and about 10 mL, between about 0.01 $\mu$L and about 1 mL, between about 0.01 $\mu$L and about 100 $\mu$L, or between about 0.1 $\mu$L and about 10 $\mu$L.

In some cases, the fluid sample may be diluted prior to use in an assay. For example, in embodiments where the source of an analyte molecule is a human body fluid (e.g., blood, serum), the fluid may be diluted with an appropriate solvent (e.g., a buffer such as PBS buffer). A fluid sample may be diluted about 1-fold, about 2-fold, about 3-fold, about 4-fold, about 5-fold, about 6-fold, about 10-fold, about 100-fold, or greater, prior to use. In other cases, the fluid sample is not diluted prior to use in an assay.

In some cases, the sample may undergo pre-analytical processing. Pre-analytical processing may offer additional functionality such as nonspecific protein removal and/or effective yet cheaply implementable mixing functionality. General methods of pre-analytical processing may include the use of electrokinetic trapping, AC electrokinetics, surface acoustic waves, isotachophoresis, dielectrophoresis, electrophoresis, or other pre-concentration techniques known in the art. In some cases, the fluid sample may be concentrated prior to use in an assay. For example, in embodiments where the source of an analyte molecule is a human body fluid (e.g., blood, serum), the fluid may be concentrated by precipitation, evaporation, filtration, centrifugation, or a combination thereof. A fluid sample may be concentrated about 1-fold, about 2-fold, about 3-fold, about 4-fold, about 5-fold, about 6-fold, about 10-fold, about 100-fold, or greater, prior to use.

**b. Controls**

It may be desirable to include a control sample. The control sample may be analyzed concurrently with the sample from the subject as described above. The results obtained from the subject sample can be compared to the results obtained from the control sample. Standard curves may be provided, with which assay results for the biological sample may be compared. Such standard curves present levels of marker as a function of assay units, i.e. fluorescent signal intensity, if a fluorescent label is used. Using samples taken from multiple donors, standard curves can be provided for control or reference levels of the GFAP in normal healthy tissue, as well as for "at-risk" levels of the GFAP in tissue taken from donors, who may have one or more of the characteristics set forth above.

Thus, in view of the above, a method for determining the presence, amount, or concentration of GFAP in a test sample is provided. The method comprises assaying the test sample for GFAP by an immunoassay, for example, employing at least one capture antibody that binds to an epitope on GFAP and at least one detection antibody that binds to an epitope on GFAP which is different from the epitope for the capture antibody and optionally includes a detectable label, and comprising comparing a signal generated by the detectable label as a direct or indirect indication of the presence, amount or concentration of GFAP in the test sample to a signal generated as a direct or indirect indication of the presence, amount or concentration of GFAP in a calibrator. The calibrator is optionally, and is preferably, part of a series of calibrators in which each of the calibrators differs from the other calibrators in the series by the concentration of GFAP. In some embodiments, the calibrator can include GFAP or a fragment thereof, as described above in Section 4a.

**8. Kit**

Provided herein is a kit, which may be used for assaying or assessing a test sample for GFAP or GFAP fragment. The kit comprises at least one component for assaying the test sample for GFAP instructions for assaying the test sample for GFAP. For example, the kit can comprise instructions for assaying the test sample for GFAP by immunoassay, e.g., chemiluminescent microparticle immunoassay. Instructions included in kits can be affixed to packaging material or can be included as a package insert. While the instructions are typically written or printed materials they are not limited to such. Any medium capable of storing such instructions and communicating them to an end user is contemplated by this disclosure. Such media include, but are not limited to, electronic storage media (e.g., magnetic discs, tapes, cartridges, chips), optical media (e.g., CD ROM), and the like. As used herein, the term "instructions" can include the address of an internet site that provides the instructions.

The at least one component may include at least one composition comprising one or more isolated antibodies or antibody fragments thereof that specifically bind to GFAP. The antibody may be a GFAP capture antibody and/or a GFAP detection antibody.

Alternatively or additionally, the kit can comprise a calibrator or control, e.g., purified, and optionally lyophilized, GFAP, and/or at least one container (e.g., tube, microtiter plates or strips, which can be already coated with an anti-GFAP monoclonal antibody) for conducting the assay, and/or a buffer, such as an assay buffer or a wash buffer, either one of which can be provided as a concentrated solution, a substrate solution for the detectable label (e.g., an enzymatic label), or a stop solution. In some embodiments, the calibrator or control can include a GFAP or fragment thereof, as described above in Section 4a. Preferably, the kit comprises all components, i.e., reagents, standards, buffers, diluents, etc., which are necessary to perform the assay. The instructions also can include instructions for generating a standard curve.

The kit may further comprise reference standards for quantifying GFAP. The reference standards may be employed to establish standard curves for interpolation and/or extrapolation of GFAP concentrations. The reference standards may include a high GFAP concentration level, for example, about 100000 pg/mL, about 125000 pg/mL, about 150000 pg/mL, about 175000 pg/mL, about 200000 pg/mL, about 225000 pg/mL, about 250000 pg/mL, about 275000 pg/mL, or about 300000 pg/mL; a medium GFAP concentration level, for example, about 25000 pg/mL, about 40000 pg/mL, about 45000 pg/mL, about 50000 pg/mL, about 55000 pg/mL, about 60000 pg/mL, about 75000 pg/mL or about 100000 pg/mL; and/or a low GFAP concentration level, for example, about 1 pg/mL, about 5 pg/mL, about 10 pg/mL, about 12.5 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, or about 100 pg/mL.

Any antibodies, which are provided in the kit, such as recombinant antibodies specific for GFAP, can incorporate a detectable label, such as a fluorophore, radioactive moiety, enzyme, biotin/avidin label, chromophore, chemiluminescent label, or the like, or the kit can include reagents for labeling the antibodies or reagents for detecting the antibodies (e.g., detection antibodies) and/or for labeling the analytes or reagents for detecting the analyte. The antibodies, calibrators, and/or controls can be provided in separate containers or pre-dispensed into an appropriate assay format, for example, into microtiter plates,

Optionally, the kit includes quality control components (for example, sensitivity panels, calibrators, and positive controls). Preparation of quality control reagents is well-known in the art and is described on insert sheets for a variety of immunodiagnostic products. Sensitivity panel members optionally are used to establish assay performance characteristics, and further optionally are useful indicators of the integrity of the immunoassay kit reagents, and the standardization of assays,

The kit can also optionally include other reagents required to conduct a diagnostic assay or facilitate quality control evaluations, such as buffers, salts, enzymes, enzyme co-factors, substrates, detection reagents, and the like. Other components, such as buffers and solutions for the isolation and/or treatment of a test sample (e.g., pretreatment reagents), also can be included in the kit. The kit can additionally include one or more other controls. One or more of the components of the kit can be lyophilized, in which case the kit can further comprise reagents suitable for the reconstitution of the lyophilized components.

The various components of the kit optionally are provided in suitable containers as necessary, e.g., a microtiter plate. The kit can further include containers for holding or storing a sample (e.g., a container or cartridge for a urine, whole blood, plasma, or serum sample). Where appropriate, the kit optionally also can contain reaction vessels, mixing vessels, and other components that facilitate the preparation of reagents or the test sample. The kit can also include one or more instrument for assisting with obtaining a test sample, such as a syringe, pipette, forceps, measured spoon, or the like.

If the detectable label is at least one acridinium compound, the kit can comprise at least one acridinium-9-carboxamide, at least one acridinium-9-carboxylate aryl ester, or any combination thereof. If the detectable label is at least one acridinium compound, the kit also can comprise a source of hydrogen peroxide, such as a buffer, solution, and/or at least one basic solution. If desired, the kit can contain a solid phase, such as a magnetic particle, bead, test tube, microtiter plate, cuvette, membrane, scaffolding molecule, film, filter paper, disc, or chip.

If desired, the kit can further comprise one or more components, alone or in further combination with instructions, for assaying the test sample for another analyte, which can be a biomarker, such as a biomarker of traumatic brain injury or disorder.

### a. Adaptation of Kit and Method

The kit (or components thereof), as well as the method for assessing or determining the concentration of GFAP in a test sample by an immunoassay as described herein, can be adapted for use in a variety of automated and semi-automated systems (including those wherein the solid phase comprises a microparticle), as described, e.g., U.S. Patent No. 5,063,081, U.S. Patent Application Publication Nos. 2003/0170881, 2004/0018577, 2005/0054078, and 2006/0160164 and as commercially marketed e.g., by Abbott Laboratories (Abbott Park, IL) as Abbott Point of Care (i-STAT® or i-STAT Alinity, Abbott Laboratories) as well as those described in U.S. Patent Nos. 5,089,424 and 5,006,309, and as commercially marketed, e.g., by Abbott Laboratories (Abbott Park, IL) as ARCHITECT® or the series of Abbott Alinity devices.

Some of the differences between an automated or semi-automated system as compared to a non-automated system (e.g., ELISA) include the substrate to which the first specific binding partner (e.g., analyte antibody or capture antibody) is attached (which can affect sandwich formation and analyte reactivity), and the length and timing of the capture, detection, and/or any optional wash steps. Whereas a non-automated format such as an ELISA may require a relatively longer incubation time with sample and capture reagent (e.g., about 2 hours), an automated or semi-automated format (e.g., ARCHITECT® and any successor platform, Abbott Laboratories) may have a relatively shorter incubation time (e.g., approximately 18 minutes for ARCHITECT®). Similarly, whereas a non-automated format such as an ELISA may incubate a detection antibody such as the conjugate reagent for a relatively longer incubation time (e.g., about 2 hours), an automated or semi-automated format (e.g., ARCHITECT® and any successor platform) may have a relatively shorter incubation time (e.g., approximately 4 minutes for the ARCHITECT® and any successor platform).

Other platforms available from Abbott Laboratories include, but are not limited to, AxSYM®, IMx® (see, e.g., U.S. Patent No. 5,294,404, which is hereby incorporated by reference in its entirety), PRISM®, EIA (bead), and Quantum™ II, as well as other platforms. Additionally, the assays, kits, and kit components can be employed in other formats, for example, on electrochemical or other hand-held or point-of-care assay systems. As mentioned previously, the present disclosure is, for example, applicable to the commercial Abbott Point of Care (i-STAT®, Abbott Laboratories) electrochemical immunoassay system that performs sandwich immunoassays. Immunosensors and their methods of manufacture and operation in single-use test devices are described, for example in, U.S. Patent No. 5,063,081, U.S. Patent App. Publication Nos. 2003/0170881, 2004/0018577, 2005/0054078, and 2006/0160164, which are incorporated in their entireties by reference for their teachings regarding same.

In particular, with regard to the adaptation of an assay to the i-STAT® system, the following configuration is preferred. A microfabricated silicon chip is manufactured with a pair of gold amperometric working electrodes and a silver-silver chloride reference electrode. On one of the working electrodes, polystyrene beads (0.2 mm diameter) with immobilized capture antibody are adhered to a polymer coating of patterned polyvinyl alcohol over the electrode. This chip is assembled into an i-STAT® cartridge with a fluidics format suitable for immunoassay. On a portion of the silicon chip, there is a specific binding partner for GFAP, such as one or more GFAP antibodies (one or more monoclonal/polyclonal antibody or a fragment thereof, a variant thereof, or a fragment of a variant thereof that can bind GFAP) or one or more anti-GFAP DVD-Igs (or a fragment thereof, a variant thereof, or a fragment of a variant thereof that can bind GFAP), any of which can be detectably labeled. Within the fluid pouch of the cartridge is an aqueous reagent that includes p-aminophenol phosphate.

In operation, a sample from a subject suspected of suffering from TBI is added to the holding chamber of the test cartridge, and the cartridge is inserted into the i-STAT® reader. A pump element within the cartridge pushes the sample into a conduit containing the chip. The sample is brought into contact with the sensors allowing the enzyme conjugate to dissolve into the sample. The sample is oscillated across the sensors to promote formation of the sandwich of approximately 2-12 minutes. In the penultimate step of the assay, the sample is pushed into a waste chamber and wash fluid, containing a substrate for the alkaline phosphatase enzyme, is used to wash excess enzyme conjugate and sample off the sensor chip. In the final step of the assay, the alkaline phosphatase label reacts with p-aminophenol phosphate to cleave the phosphate group and permit the liberated p-aminophenol to be electrochemically oxidized at the working electrode. Based on the measured current, the reader is able to calculate the amount of GFAP in the sample by means of an embedded algorithm and factory-determined calibration curve.

The methods and kits as described herein necessarily encompass other reagents and methods for carrying out the immunoassay. For instance, encompassed are various buffers such as are known in the art and/or which can be readily prepared or optimized to be employed, e.g., for washing, as a conjugate diluent, and/or as a calibrator diluent. An exemplary conjugate diluent is ARCHITECT® conjugate diluent employed in certain kits (Abbott Laboratories, Abbott Park, IL) and containing 2-(N-morpholino)ethanesulfonic acid (MES), a salt, a protein blocker, an antimicrobial agent, and a detergent. An exemplary calibrator diluent is ARCHITECT® human calibrator diluent employed in certain kits (Abbott Laboratories, Abbott Park, IL), which comprises a buffer containing MES, other salt, a protein blocker, and an antimicrobial agent. Additionally, as described in U.S. Patent Application No. 61/142,048 filed December 31, 2008, improved signal generation may be obtained, e.g., in an i-STAT® cartridge format, using a nucleic acid sequence linked to the signal antibody as a signal amplifier.

While certain embodiments herein are advantageous when employed to assess disease, such as traumatic brain injury, the assays and kits also optionally can be employed to assess GFAP in other diseases, disorders, and conditions as appropriate.

The method of assay also can be used to identify a compound that ameliorates diseases, such as traumatic brain injury. For example, a cell that expresses GFAP can be contacted with a candidate compound. The level of expression of GFAP in the cell contacted with the compound can be compared to that in a control cell using the method of assay described herein.

The present disclosure has multiple aspects, illustrated by the following non-limiting examples.

## 9. Examples

It will be readily apparent to those skilled in the art that other suitable modifications and adaptations of the methods of the present disclosure described herein are readily applicable and appreciable, and may be made using suitable equivalents without departing from the scope of the present disclosure or the aspects and embodiments disclosed herein. Having now described the present disclosure in detail, the same will be more clearly understood by reference to the following examples, which are merely intended only to illustrate some aspects and embodiments of the disclosure, and should not be viewed as limiting to the scope of the disclosure. The disclosures of all journal references, U.S. patents, and publications referred to herein are hereby incorporated by reference in their entireties.

Additionally, this application incorporates by reference the disclosures in U.S. Provisional Application No. 62/403,293, filed October 3, 2016, and U.S. Provisional Application No. 62/455,269, filed February 6, 2017, in their entirety.

This application also incorporates by reference the disclosures in U.S. Application No. XX/XXX,XXX and PCT/US2017/XXXXXX each titled "IMPROVED Methods of Assessing GFAP Status in patient samples" and U.S. Application No. XX/XXX,XXX and PCT/US2017XXXXXX each titled "IMPROVED Methods of Assessing UCH-L1 Status in patient samples," all of which are filed on October 2, 2017, in its entirety.

The present disclosure has multiple aspects, illustrated by the following non-limiting examples.

## Example 1

**i-STAT® GFAP Assay**

Antibodies were screened using the assay format of interest (i-STAT). Pairs of antibodies that generated signal in the assay were selected. The initial selection criteria were based on a number of factors that included detection of signal by the antibody pairs when screened using a low calibrator concentration. Monoclonal antibody pairs, such as Antibody A as a capture monoclonal antibody and Antibody B as a detection monoclonal antibody, were tested. Antibody A and Antibody B are exemplary anti-GFAP antibodies that were internally developed at Abbott Laboratories (Abbott Park, IL). Antibody A and Antibody B both bind to epitopes within the same GFAP breakdown product (BDP). The combination of the antibodies provided a synergistic effect when used together and provided for an increased signal. This data was generated by purchasing short overlapping peptide sequences and determining which peptide the antibody binds to in a 96-well plate format. The GFAP assay design was evaluated against key performance attributes. The cartridge configuration was Antibody Configuration: Antibody A (Capture Antibody)/Antibody B (Detection Antibody); Reagent conditions: 0.8% solids, 250 μg/mL Fab Alkaline Phosphatase cluster conjugate; and Sample Inlet Print: GFAP specific. The assay time was 10-15 min (with 7-12 min sample capture time).

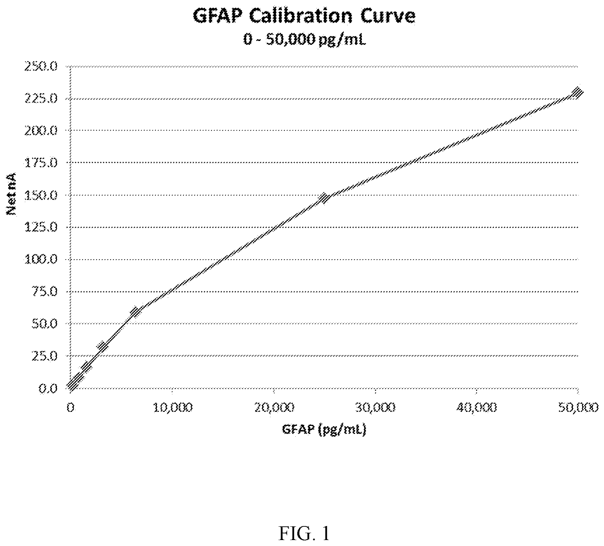

**Assay Calibration.** Calibrators were prepared using OriGene recombinant GFAP (0 - 50,000 pg/mL) (OriGene Technologies, Inc., Rockville, MD) in an EDTA plasma pool. The GFAP concentration was based on vendor label claim. The calibrator was aliquoted and stored frozen (-70 °C). The curve fit was 4PLC (4 parameter logistic curve). See FIG. 1; see also Table 2, which is based on n = 75 reps/cal level.

**Table 2**

| | Net Current (nA) | | Concentration (pg/mL) | |
|---|---|---|---|---|
| Cal (pg/mL) | Mean | %CV | Mean | %CV |
| 0 | -0.13 | -95.4 | 0.9 | n/a |
| 200 | 2.1 | 6.8 | 199.5 | 6.6 |
| 800 | 8.4 | 5.7 | 802.2 | 5.9 |
| 1600 | 16.5 | 5.6 | 1608.7 | 5.8 |
| 3200 | 31.5 | 4.8 | 3187.7 | 5.2 |
| 6400 | 59.0 | 4.7 | 6400.5 | 5.4 |
| 25000 | 147.6 | 4.8 | 23824.8 | 7.7 |
| 50000 | 229.6 | 6.0 | 53190.8 | 12.4 |

**Assay precision.** 5-Day precision study design was based on guidance from CLSI protocols (EP5-A2 (NCCLS. Evaluation of Precision Performance of Quantitative Measurement Methods; Approved Guideline - Second Edition. NCCLS document EP5-A2 [ISBN 1-56238-542-9]. NCCLS, 940 West Valley Road, Suite 1400, Wayne, Pennsylvania 19087-1898 USA, 2004.) and EP15-A2 (Clinical and Laboratory Standards Institute. User Verification of Performance for Precision and Trueness; Approved Guideline - Second Edition. CLSI document EP15-A2 [ISBN 1-56238-574-7]. Clinical and Laboratory Standards Institute, 940 West Valley Road, Suite 1400, Wayne, Pennsylvania 19087-1898 USA, 2005.)). The testing protocol included 5 Days, 2 Runs/Day, 4 Reps/Run (n=40 reps/sample). The analysis used JMP software program (a statistical discovery program from SAS, Cary, NC) to determine day, run, and rep variance components using a nested model. Panels (n=6) were prepared with target GFAP concentrations shown in Table 3.

**Table 3**

| Precision Panel | GFAP Concentration (pg/mL) |
|---|---|
| OriGene antigen spiked in serum matrix* | 100 |
| | 1000 |
| | 5000 |
| Spinal cord lysate (SCL) spiked in lithium heparin plasma pool | 3000 |

(continued)

| Precision Panel | GFAP Concentration (pg/mL) |
|---|---|
| Pooled TBI Specimens in EDTA plasma | 100 |
| | 200 |
| *Cliniqa (Fallbrook, CA) serum matrix was used as the matrix for i-STAT TBI quality control materials. SCL was from Analytical Biological Services, Inc. (Wilmington, DE). | |

As shown in Table 4, less than 10% Total CV was observed across all panels from 100 - 4,400 pg/mL on the individual cartridges for GFAP.

**Table 4**

| Panel (GFAP Target Conc.) | Mean (pg/mL) | Between Day | | Between Run | | Between Rep | | Total | |
|---|---|---|---|---|---|---|---|---|---|
| | | SD | %CV | SD | %CV | SD | %CV | SD | %CV |
| OriGene 100 | 138.1 | 4.8 | 3.4 | 0.0 | 0.0 | 9.1 | 6.6 | 10.3 | 7.4 |
| OriGene 1000 | 1200.7 | 16.6 | 1.4 | 0.0 | 0.0 | 96.8 | 8.1 | 98.2 | 8.2 |
| OriGene 5000 | 4406.2 | 30.5 | 0.7 | 0.0 | 0.0 | 274.7 | 6.2 | 276.4 | 6.3 |
| SCL 3000 | 3235.9 | 0.0 | 0.0 | 0.0 | 0.0 | 85.2 | 2.6 | 85.2 | 2.6 |
| Native 100 | 100.0 | 1.5 | 1.5 | 0.6 | 0.6 | 4.8 | 4.8 | 5.1 | 5.1 |
| Native 200 | 195.1 | 0.0 | 0.0 | 0.0 | 0.0 | 7.8 | 4.0 | 7.8 | 4.0 |

**Limit of detection (LoD).** LoD study design was based on guidance from Clinical and Laboratory Standards Institute (CLSI) protocol EP17-A2 ("Protocols for Determination of Limits of Detection and Limits of Quantitation; Approved Guideline - Second Edition", EP17A2E, by James F. Pierson-Perry et al., Clinical and Laboratory Standards Institute, June 1, 2012, 80 pages [ISBN: 1562387952]). The testing protocol utilized a zero level plasma pool to determine Limit of Blank (LoB). 60 reps total were tested. A 50 pg/mL GFAP panel was prepared by spiking an elevated GFAP sample into a plasma pool. Dilutions were prepared to a target concentration of 10 - 40 pg/mL. 40 reps were tested for the GFAP panel across 3 days. The Data Analysis was as follows: LoB = 95th percentile of zero-analyte sample concentrations; LoD = LoB + Cp × SD(within-lab), Cp is a multiplier to give 95th percentile of SD(within-lab). SD(within-lab) was pooled Standard Deviation across all five panels.
**Results:** Precision profile for each panel shows that CVs range from 5 - 8% for Panels >20 pg/mL. See Table 5.

Results are used to determine functional sensitivity by fitting the equation: $\%CV = a + \dfrac{b}{[\text{GFAP}]}$ .

**Table 5**

| | Panel | | | | | |
|---|---|---|---|---|---|---|
| | 0 | 10 | 20 | 30 | 40 | 50 |
| Mean (pg/mL) | 1.1 | 13.6 | 26.5 | 38.5 | 51.0 | 65.1 |
| Std Dev | 1.8 | 2.2 | 2.0 | 2.5 | 2.6 | 3.1 |
| %CV | N/A | 16.3 | 7.7 | 6.6 | 5.1 | 4.7 |
| *One data point >10 SD from mean was replaced with a repeat test result | | | | | | |

LoD was determined to be <10 pg/mL. The results were based on a single reagent lot and cartridge lot. The Functional Sensitivity (at 20% CV) was <20 pg/mL. See Table 6. Functional Sensitivity is an estimation of Limit of Quantitation (LoQ).

**Table 6**

| Assay | LoB (pg/mL) | LoD (pg/mL) | Functional Sensitivity, 20% CV (pg/mL) |
|---|---|---|---|
| GFAP | 4 | 8 | 11 |

**Linearity/Assay Range.** Assay linearity was evaluated using a series of dilutions as follows. In each dilution study, a series of dilutions was prepared by blending the high and low concentration samples. The first dilution that utilized a high concentration sample was prepared by spiking tissue lysate into an EDTA plasma pool to a target GFAP concentration of about 15,000 pg/mL. A second dilution utilized pooled EDTA plasma specimens from suspected TBI patients. The target starting GFAP concentration was about 1,000 pg/mL. A third dilution study was evaluated using a spiked tissue lysate into an EDTA plasma pool to a target GFAP concentration of about 50,000 pg/mL. A fourth dilution study was evaluated using a spiked tissue lysate into a serum pool to a target GFAP concentration of about 50,000 pg/mL. The data was analyzed as follows: plot expected vs. observed concentrations, determine correlation coefficient. Linearity was assessed per CLSI EP6-A by fitting the data to a first, second, third-order polynomial regressions. The best fitting model was used to determine deviation from linearity.

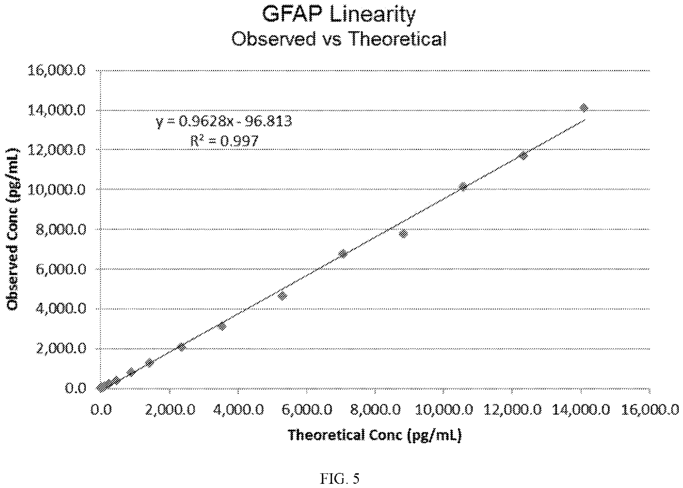

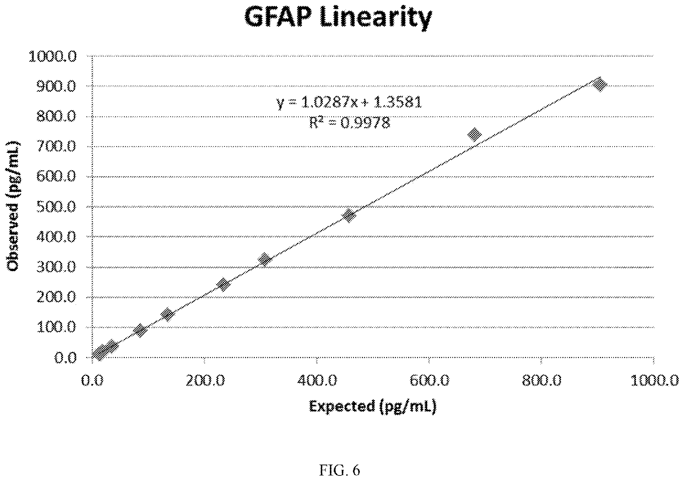

**Results:** Dilution 1: The correlation coefficient (Observed vs. Expected) was r = 0.9985. Table 7; FIG. 5. Less than 10% deviation from linearity (DL) was achieved from 20 to 13,660 pg/mL. Dilution 2: The correlation coefficient (Observed vs. Expected) was r = 0.9989. Table 8; FIG. 6. Less than 10% deviation from linearity (DL) was achieved from 12 to 900 pg/mL. Dilution 3: The correlation coefficient (Observed vs. Expected) was r = 0.9990. Table 9. Less than 10% deviation from linearity was achieved from 420 - >50,000 pg/mL. Dilution 4: The correlation coefficient (Observed vs. Expected) was r = 0.9993. Table 10. Less than 10% deviation from linearity was achieved from 370 - >50,000 pg/mL.

**Table 7**

| Dilution | Expected (pg/mL) | Observed (pg/mL) | %CV | %Bias | Predicted Linear Fit | Predicted 2nd order | Deviation from Linearity DL | Deviation from Linearity % DL |
|---|---|---|---|---|---|---|---|---|
| 1 | 14,109.0 | 14,109.0 | 5.7 | 0.0 | 12,682.1 | 13,665.9 | 983.77 | 7.8 |
| 2 | 12,347.8 | 11,710.7 | 2.1 | 5.4 | 11,099.2 | 11,792.3 | 693.15 | 6.2 |
| 3 | 10,586.6 | 10,134.9 | 2.2 | 4.5 | 9,516.3 | 9,966.8 | 450.47 | 4.7 |
| 4 | 8,825.4 | 7,755.1 | 4.8 | 13.8 | 7,933.4 | 8,189.1 | 255.74 | 3.2 |
| 5 | 7,064.1 | 6,774.6 | 1.8 | 4.3 | 6,350.5 | 6,459.4 | 108.96 | 1.7 |
| 6 | 5,302.9 | 4,623.1 | 1.4 | 14.7 | 4,767.5 | 4,777.7 | 10.12 | 0.2 |
| 7 | 3,541.7 | 3,120.3 | 2.0 | 13.5 | 3,184.6 | 3,143.9 | -40.77 | -1.3 |
| 8 | 2,367.3 | 2,050.1 | 2.3 | 15.5 | 2,129.1 | 2,081.1 | -48.07 | -2.3 |
| 9 | 1,428.2 | 1,248.9 | 2.0 | 14.4 | 1,285.1 | 1,246.6 | -38.56 | -3.0 |
| 10 | 899.9 | 793.3 | 3.6 | 13.4 | 810.3 | 783.0 | -27.21 | -3.4 |
| 11 | 459.6 | 404.7 | 4.8 | 13.6 | 414.5 | 400.1 | -14.47 | -3.5 |
| 12 | 239.4 | 213.5 | 1.9 | 12.2 | 216.7 | 209.7 | -6.97 | -3.2 |
| 13 | 129.3 | 116.3 | 3.2 | 11.2 | 117.7 | 114.8 | -2.94 | -2.5 |
| 14 | 74.3 | 69.8 | 7.9 | 6.4 | 68.3 | 67.4 | -0.85 | -1.2 |
| 15 | 46.8 | 47.5 | 10.4 | -1.6 | 43.5 | 43.7 | 0.21 | 0.5 |
| 16 | 33.0 | 33.8 | 6.0 | -2.2 | 31.2 | 31.9 | 0.74 | 2.4 |
| 17 | 26.1 | 22.3 | 9.8 | 17.2 | 25.0 | 26.0 | 1.01 | 4.0 |
| 18 | 19.3 | 19.3 | 9.4 | 0.0 | 18.8 | 20.1 | 1.28 | 6.8 |

**Table 8**

| Dilution | Expected (pg/mL) | Observed (pg/mL) | %CV | %Bias | Predicted Linear Fit | Deviation from Linearity DL | Deviation from Linearity % DL |
|---|---|---|---|---|---|---|---|
| 1 | 904.5 | 904.5 | 1.8 | 0.0 | 932.0 | -27.5 | -2.9 |
| 2 | 681.2 | 738.9 | 3.4 | 8.5 | 701.8 | 37.1 | 5.3 |
| 3 | 457.8 | 469.7 | 5.0 | 2.6 | 471.5 | -1.8 | -0.4 |
| 4 | 309.1 | 324.1 | 2.0 | 4.9 | 318.1 | 6.0 | 1.9 |

(continued)

| Dilution | Expected (pg/mL) | Observed (pg/mL) | %CV | %Bias | Predicted Linear Fit | Deviation from Linearity | |
|---|---|---|---|---|---|---|---|
| | | | | | | DL | % DL |
| 5 | 235.1 | 242.0 | 3.1 | 2.9 | 241.9 | 0.1 | 0.0 |
| 6 | 135.8 | 141.2 | 3.1 | 4.0 | 139.5 | 1.7 | 1.2 |
| 7 | 86.3 | 88.7 | 3.9 | 2.8 | 88.4 | 0.2 | 0.3 |
| 8 | 36.7 | 36.8 | 6.0 | 0.1 | 37.3 | -0.5 | -1.5 |
| 9 | 20.2 | 20.9 | **14.1** | 3.1 | 20.3 | 0.6 | 2.8 |
| 10 | 14.7 | 12.9 | **18.0** | -12.4 | 14.6 | -1.7 | **-11.8** |
| 11 | 12.0 | 12.0 | 8.9 | 0.0 | 11.8 | 0.2 | 1.4 |

**Table 9**

| Dilution | Expected (pg/mL) | Observed (pg/mL) | %CV | %Bias | Predicted | | Deviation from Linearity | |
|---|---|---|---|---|---|---|---|---|
| | | | | | Linear Fit | 2nd order | DL | % DL |
| 1.0 | 61542.3 | 61542.3 | 5.94 | 0.0 | 65867.6 | 63298.2 | -2569.4 | -3.9 |
| 0.9 | 55388.1 | 55945.6 | 2.41 | 1.0 | 59280.9 | 57309.0 | -1971.8 | -3.3 |
| 0.8 | 49233.9 | 51399.4 | 2.22 | 4.4 | 52694.1 | 51244.1 | -1450.0 | -2.8 |
| 0.7 | 43079.6 | 43921.4 | 2.59 | 2.0 | 46107.4 | 45103.6 | -1003.8 | -2.2 |
| 0.6 | 36925.4 | 40163.4 | 1.96 | 8.8 | 39520.6 | 38887.3 | -633.3 | -1.6 |
| 0.5 | 30771.2 | 33418.6 | 2.72 | 8.6 | 32933.8 | 32595.3 | -338.5 | -1.0 |
| 0.4 | 24617.0 | 26643.4 | 3.42 | 8.2 | 26347.1 | 26227.7 | -119.4 | -0.5 |
| 0.3 | 18462.7 | 19740.8 | 1.93 | 6.9 | 19760.3 | 19784.3 | 24.0 | 0.1 |
| 0.2 | 12308.5 | 13213.4 | 2.28 | 7.4 | 13173.6 | 13265.2 | 91.7 | 0.7 |
| 0.1 | 6154.3 | 6830.2 | 1.71 | 11.0 | 6586.8 | 6670.5 | 83.7 | 1.3 |
| 0.05 | 3077.2 | 3329.2 | 2.13 | 8.2 | 3293.4 | 3344.7 | 51.3 | 1.6 |
| 0.025 | 1538.6 | 1674.4 | 2.31 | 8.8 | 1646.8 | 1674.8 | 28.0 | 1.7 |
| 0.0125 | 769.3 | 776.7 | 3.16 | 1.0 | 823.4 | 838.0 | 14.6 | 1.8 |
| 0.00625 | 384.7 | 418.5 | 1.41 | 8.8 | 411.7 | 419.2 | 7.4 | 1.8 |
| 0.0 | 0.1 | 0.1 | 173.21 | 0.0 | 0.1 | 0.1 | 0.0 | -5.4 |

**Table 10**

| Dilution | Expected (pg/mL) | Observed (pg/mL) | %CV | %Bias | Predicted | | Deviation from Linearity | |
|---|---|---|---|---|---|---|---|---|
| | | | | | Linear Fit | 2nd order | DL | % DL |
| 1.0 | 55311.1 | 55311.1 | 6.38 | 0.0 | 57560.4 | 53532.1 | -4028.3 | -7.0 |
| 0.9 | 49780.1 | 47896.4 | 5.83 | 3.8 | 51804.5 | 48707.9 | -3096.6 | -6.0 |
| 0.8 | 44249.1 | 43349.0 | 2.94 | 2.0 | 46048.6 | 43766.2 | -2282.5 | -5.0 |
| 0.7 | 38718.1 | 37727.6 | 4.26 | 2.6 | 40292.7 | 38706.9 | -1585.8 | -3.9 |
| 0.6 | 33187.1 | 34070.3 | 1.37 | 2.7 | 34536.8 | 33530.1 | -1006.7 | -2.9 |
| 0.5 | 27656.1 | 28379.7 | 0.83 | 2.6 | 28780.9 | 28235.8 | -545.1 | -1.9 |
| 0.4 | 22125.1 | 23003.8 | 1.31 | 4.0 | 23025.0 | 22823.9 | -201.1 | -0.9 |
| 0.3 | 16594.1 | 17330.2 | 2.35 | 4.4 | 17269.1 | 17294.5 | 25.4 | 0.1 |
| 0.2 | 11063.1 | 11268.7 | 0.81 | 1.9 | 11513.2 | 11647.6 | 134.4 | 1.2 |
| 0.1 | 5532.1 | 6030.8 | 1.67 | 9.0 | 5757.3 | 5883.2 | 125.9 | 2.2 |
| 0.05 | 2766.6 | 3048.3 | 1.83 | 10.2 | 2879.4 | 2956.9 | 77.5 | 2.7 |
| 0.025 | 1383.8 | 1501.5 | 4.12 | 8.5 | 1440.4 | 1482.7 | 42.3 | 2.9 |
| 0.0125 | 692.4 | 744.5 | 1.17 | 7.5 | 720.9 | 742.9 | 22.0 | 3.1 |
| 0.00625 | 346.7 | 397.4 | 1.95 | 14.6 | 361.2 | 372.3 | 11.1 | 3.1 |
| 0.0 | 1.1 | 1.1 | 58.00 | 0.0 | 1.4 | 1.2 | -0.2 | **-13.7** |

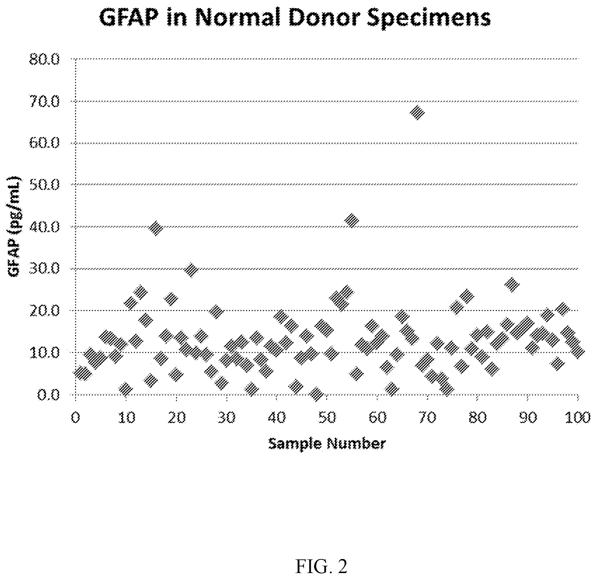

**Normal Donor Samples.** 100 plasma samples from apparently healthy donors, acquired from commercial sources, were tested in the i-STAT GFAP assay. The GFAP levels were quite low with a median value of 12 pg/mL and the maximum value less than 70 pg/mL. See FIG. 2 and Table 11.

**Table 11**

|  | GFAP (pg/mL) |
|---|---|
| Mean | 13.0 |
| SD | 9.1 |
| **Quartiles** |  |
| 25th %ile | 8.1 |
| Median | 12.1 |
| 75th %ile | 15.6 |

The GFAP assay shows: Limit of Detection (LoD) <20 pg/mL; Assay calibration to 50,000 pg/mL, and assay linearity to 50,000 pg/mL; Precision <10% CV from 100 - 4,000 pg/mL; Results in ≤ 15 min.

**Example 2**

**Potential Assay Interferences**

The GFAP assay was evaluated for the potential interferences and crossreactants as shown in Table 12. In summary, interferences were spiked into a GFAP panel with target concentrations of 150 - 200 pg/mL. Interfering substance test concentrations were based on CLSI EP7-A2 guidance (Clinical and Laboratory Standards Institute. Interference Testing in Clinical Chemistry; Approved Guideline - Second Edition. CLSI document EP7-A2 [ISBN 1-56238-584-4]. Clinical and Laboratory Standards Institute, 940 West Valley Road, Suite 1400, Wayne, Pennsylvania 19087-1898 USA, 2005.). Potential cross reactants were tested at 500 ng/mL. Acceptance criteria was <10% interference.

Specifically, the GFAP assay was evaluated for potential endogenous interferences. The potentially interfering substance was prepared in a buffer/solvent of choice and added to a test sample containing the analyte of interest. A control sample was prepared where only the buffer/solvent of choice was added. The interference was calculated based on the % difference of the measured results between the control sample and the test sample containing the interferent.

Samples containing the following potentially interfering endogenous substances were evaluated for interference: Bilirubin (Unconjugated & Conjugated); Triglycerides; Hemoglobin; Total Protein; Heparin; and Endogenous antibodies (HAMA, RF). The GFAP panel was prepared in Li heparin plasma pool using tissue lysate targeting 150-200 pg/mL. All the samples were tested on the GFAP cartridge. The % interference was calculated by taking the difference between the test solution and control solution divided by control solution multiplied by 100, as shown in the equation: % Interference = 100 x (Test - Control) / Control.

**Bilirubin:** Conjugated and unconjugated bilirubin were tested separately for interference as recommended in CLSI EP7-A2 guidance, as described above. The bilirubin concentration was confirmed by testing the samples on the ARCHITECT clinical chemistry analyzer. Table 12 shows <10% interference at >20 mg/dL of bilirubin for GFAP.

**Triglycerides:** The triglyceride stock (Intralipid) was spiked into the sample containing GFAP and the buffer/matrix was spiked to the sample containing GFAP to prepare the control sample. The triglycerides concentration was confirmed by testing the samples on the ARCHITECT clinical chemistry analyzer. Table 12 shows <10% interference at >3,000 mg/dL of triglycerides for GFAP.

**Hemoglobin:** Hemoglobin as evaluated due to the potential for hemolysis in specimens. The source for hemoglobin was typically a hemolysate prepared from washed red blood cells. The amount of hemoglobin in the sample was confirmed on a hematology analyzer. Table 12 shows <10% effect at >500 mg/dL of hemoglobin for GFAP.

**Total Protein:** The total protein content in human specimens showed some variability, with the normal ranging from 6.4 - 8.3 g/dL (Tietz) and 99% of specimens were <9 g/dL (internal analysis). To evaluate the effect of total protein, samples were supplemented with HSA (human serum albumin) and compared to a normal sample. The protein content of the samples was independently determined using ARCHITECT clinical chemistry total protein test. Table 12 shows <10% interference at 8.8 g/dL of total protein for GFAP.

**Heparin:** Heparin is used as an anticoagulant in blood collection tubes and is evaluated as a potential interferent since heparinized whole blood and plasma are potential sample types in the i-STAT assays. A heparin tube contains approximately 15 U/mL heparin, so the test concentration represents a higher concentration that might be present if a collection tube is not completely filled (short draw). Table 12 shows <10% interference due to 90 U/mL of heparin in the sample for GFAP.

**Endogenous antibodies (HAMA, RF):** Immunoassays rely on specific interactions between the antibodies and analyte of interest for optimal performance. However, some specimens may contain endogenous antibodies that cross-react with the antibodies employed in the assay. Non-specific antibodies were added to the assay as blocking proteins of nonspecific interactions and reduced the potential for interference. Two of the commonly identified sources of potential interference in immunoassays were used; HAMA (human anti mouse antibody) and RF (Rheumatoid Factor). HAMA and RF concentrates were obtained from commercial sources; Roche, Scantibodies, and Bioreclamation. These concentrates were spiked into a plasma pool that had also been spiked with low amounts of GFAP to evaluate the potential interference. A control sample was prepared by spiking buffer into the corresponding samples. The recovery was determined relative to the HAMA or RF spiked plasma pool. The results are presented in Table 12. GFAP recovery was within $100 \pm 10\%$ in the presence of HAMA and RF.

**Cross Reactivity and Interference of homologous proteins: Potential GFAP cross reactants** - Both vimentin and desmin have a high (~60%) sequence homology to GFAP based on primary sequence alignment. Both cross-reactivity (absence of GFAP) and interference (presence of GFAP) of vimentin and desmin were evaluated at a concentration of 500 ng/mL. Recombinant vimentin and desmin were purchased from OriGene. % Cross reactivity was determined by the equation: 100* (Test - Control)/Cross reactant concentration. The results shown in Table 12 indicates there is no significant cross reactivity and <10% interference with vimentin and desmin in the GFAP assay.

**Table 12**

| Potential Interferent | Interference |
|---|---|
| Bilirubin (unconjugated & conjugated) | <10% |
| Triglycerides | <10% |
| Hemoglobin | <10% |
| Total protein (9 g/dL) | <10% |
| Heparin | <10% |
| Endogenous antibodies (HAMA & RF) | <10% |
| Cross reactants (vimentin, desmin) | <10% <br> <0.001% cross reactivity |

**Example 3**

**TBI Population Study**

The i-STAT GFAP assay was used in a TBI patient population study.

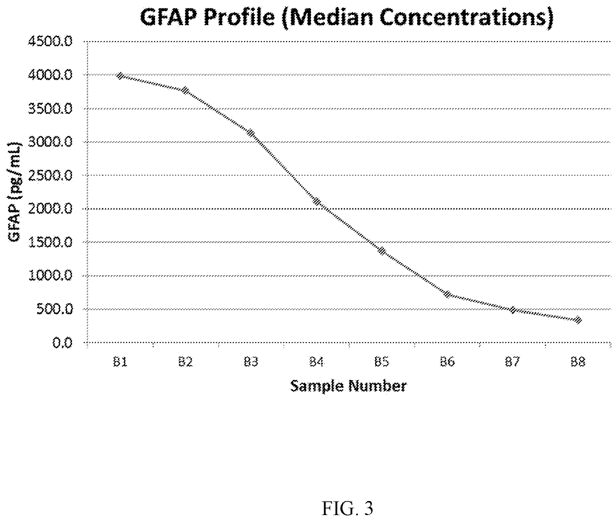

**Study Specimens:** 260 total subjects with moderate to severe TBI were enrolled with up to 8 timepoints/subject; all samples were serum. Table 13; FIG. 3.

**Table 13**

| SAMPLE | SAMPLE_TIME_POINT |
|---|---|
| B1 | Pre Infusion |
| B2 | Infusion: + 12 hrs |
| B3 | infusion: + 24 hrs |
| B4 | infusion: + 36 hrs |
| B5 | Infusion: + 48 hrs |
| B6 | Infusion: + 72 hrs |
| B7 | Infusion: + 96 hrs |
| B8 | Infusion: + 120 hrs |

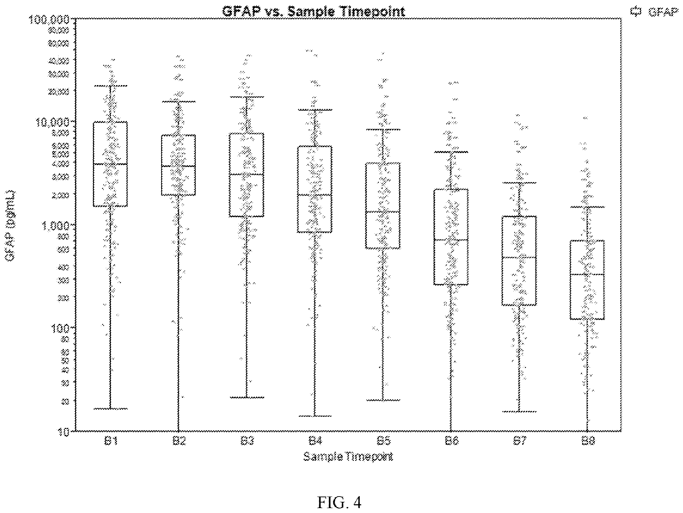

**Distribution of Study Specimens.** FIG. 3 shows the median results of all GFAP assay results at each timepoint, this shows that GFAP is high at the B1 sample and tends to decrease with later timepoints. FIG. 4 shows box plot (log scale) at each sample timepoint which shows a wide distribution of GFAP results across the patient population. The boxes represent interquartile ranges (25th, 50th, and 75th percentiles).

In summary, 250 total subjects were available for testing. All timepoints were not necessarily available from all subjects. Some samples had limited or insufficient volume. The GFAP results spanned the entire assay range (<0.1 to >50 ng/mL) and 20 samples were read at greater than 50,000 pg/mL.

It is understood that the foregoing detailed description and accompanying examples are merely illustrative and are not to be taken as limitations upon the scope of the invention, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the invention, may be made without departing from the spirit and scope thereof.

For reasons of completeness, various aspects of the invention are set out in the following numbered clauses:

Clause 1. A method of measuring glial fibrillary acid protein (GFAP) in a biological sample from a subject that may have sustained an injury to the head, the method comprising (a) obtaining a biological sample from said subject, (b) contacting the biological sample with, either simultaneously or concurrently, in any order:

(1) a capture antibody, which binds to an epitope on GFAP or GFAP fragment to form a capture antibody-GFAP antigen complex, and (2) a detection antibody which includes a detectable label and binds to an epitope on GFAP that is not bound by the capture antibody, to form a GFAP antigen-detection antibody complex,

such that a capture antibody-GFAP antigen-detection antibody complex is formed, and
(c) determining the amount or concentration of GFAP in the biological sample based on the signal generated by the detectable label in the capture antibody-GFAP antigen-detection antibody complex, wherein the method can be used to determine levels of GFAP in an amount of less than or equal to 50,000 pg/mL in a volume of less than 20 microliters of said biological sample, and wherein said method has a dynamic range of 5 log, and is linear over said dynamic range.

Clause 2. A method of assessing a subject's glial fibrillary acid protein (GFAP) status as a measure of traumatic brain injury wherein said subject may have sustained an injury to the head, the method comprising the step of:
detecting at least one biomarker in a biological sample from said subject wherein at least one of the biomarkers is GFAP and wherein the method (i) can be used to determine levels of GFAP in an amount less than or equal to 50,000 pg/mL in a volume of less than 20 microliters of said biological sample, (ii) has a dynamic range of 5 log, and (iii) is linear over the dynamic range.

Clause 3. A method of assessing glial fibrillary acid protein (GFAP) status as a measure of traumatic brain injury in a subject that may have sustained an injury to the head, the method comprising the step of: detecting at least one biomarker in a biological sample from said subject wherein at least one of the biomarkers is GFAP and wherein the method (i) has a dynamic range of 5 log and (ii) is linear over said dynamic range.

Clause 4. A method of assessing glial fibrillary acid protein (GFAP) status as a measure of traumatic brain injury in a subject that may have sustained an injury to the head, the method comprising the steps of:

a) contacting a biological sample from said subject with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; and
b) assessing a signal from the one or more first complexes, wherein the presence of a detectable signal from the detectable label indicates that GFAP is present in the sample and the presence of detectable signal from the detectable label can be employed to assess said subject's GFAP status as a measure of traumatic brain injury,

wherein said assay is capable of detecting an amount of GFAP less than or equal to 50,000 pg/mL in a volume of less than 20 microliters of test sample, wherein said assay has a dynamic range of 5 log, and is linear over said dynamic range.

Clause 5. A method of assessing a subject's glial fibrillary acid protein (GFAP) status as a measure of traumatic brain injury wherein said subject may have sustained an injury to the head, the method comprising the steps of:

a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein either the first or second specific binding member comprises a detectable label; and

b) assessing a signal from the one or more first complexes, wherein the amount of detectable signal from the detectable label indicates the amount of GFAP present in the sample, such that the amount of detectable signal from the detectable label can be employed to assess said subject's GFAP status as a measure of traumatic brain injury,

wherein the method (i) can be used to determine levels of up to 50,000 pg/mL of GFAP, (ii) does not require dilution of the biological sample, and (iii) is conducted using a point-of-care device.

Clause 6. A method of measuring GFAP in a biological sample from a subject that may have sustained an injury to the head, the method comprising (a) obtaining a biological sample from said subject, (b) contacting the biological sample with, either simultaneously or sequentially, in any order:

(1) a capture antibody, which binds to an epitope on GFAP or GFAP fragment to form a capture antibody-GFAP antigen complex, and (2) a detection antibody which includes a detectable label and binds to an epitope on GFAP that is not bound by the capture antibody, to form a GFAP antigen-detection antibody complex,

such that a capture antibody-GFAP antigen-detection antibody complex is formed, and

(c) determining the amount or concentration of GFAP in the biological sample based on the signal generated by the detectable label in the capture antibody-GFAP antigen-detection antibody complex, wherein the method can be used to determine levels of GFAP in an amount of less than or equal to 50,000 pg/mL, and wherein said method has a dynamic range of 5 log, and is linear over said dynamic range.

Clause 7. A method of assessing a subject's glial fibrillary acid protein (GFAP) status as a measure of traumatic brain injury wherein said subject may have sustained an injury to the head, the method comprising the step of:

detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

Clause 8. A method of assessing glial fibrillary acid protein (GFAP) status as a measure of traumatic brain injury in a subject that may have sustained an injury to the head, the method comprising the steps of:

a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; and

b) assessing a signal from the one or more first complexes, wherein the presence of a detectable signal from the detectable label indicates that GFAP is present in the sample, and the presence of detectable signal from the detectable label can be employed to assess said subject's GFAP status as a measure of traumatic brain injury,

wherein the method can be used to determine levels of GFAP in an amount of less than or equal to 50,000 pg/mL, and wherein said method has a dynamic range of 5 log, and is linear over said dynamic range.

Clause 9. A method of measuring glial fibrillary acid protein (GFAP) status as a measure of traumatic brain injury in a subject that may have sustained an injury to the head, the method comprising the steps of:

a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label;

b) detecting a signal from the one or more first complexes, wherein the presence of a detectable signal from the detectable label indicates that GFAP is present in the sample, and

c) measuring the amount of detectable signal from the detectable label indicates the amount of GFAP present in the sample, such that the amount of detectable signal from the detectable label can be employed to assess said subject's GFAP status as a measure of traumatic brain injury,

wherein said assay is capable of determining an amount of GFAP less than or equal to 50,000 pg/mL in a volume of less than 20 microliters of test sample, wherein said assay has a dynamic range of 5 log, and is linear over said dynamic range.

Clause 10. The method of any of clauses 5-8, wherein said wherein said method is done using a volume of less than 20 microliters of said biological sample.

Clause 11. The method of any of clauses 5-10, wherein the method can be used to determine levels of GFAP selected from the group consisting of from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL.

Clause 12. The method of any of clauses 5, 8 or 9, wherein either the first specific binding member or second specific binder member, whichever does not comprise the detectable label, is immobilized on a solid support.

Clause 13. The method of any of clauses 5-12, wherein GFAP is assessed along with one or more other biomarker.

Clause 14. The method of any of clauses 6-13, wherein the biological sample does not require dilution.

Clause 15. The method of any of clauses 5-14, wherein the biological sample is selected from the group consisting of a whole blood sample, a serum sample, a cerebrospinal fluid sample and a plasma sample.

Clause 16. The method of any of clauses 5-15, wherein the method is performed in from about 5 to about 20 minutes.

Clause 17. The method of any of clauses 5-16, wherein the method is performed in about 15 minutes.

Clause 18. The method of any of clauses 5-17, wherein the biological sample is from about 1 to about 25 microliters.

Clause 19. The method of any of clauses 5-18, wherein the time between when the biological sample is obtained and when the subject may have sustained an injury to the head is not known.

Clause 20. The method of any of clauses 5-19, wherein the time between when the biological sample is obtained and when the subject may have sustained an injury to the head is selected from the group consisting of from zero to about 12 hours, from about 12 to about 24 hours, from about 24 to about 36 hours, from about 36 to about 48 hours, from about 48 to about 72 hours, from about 72 to about 96 hours, from about 96 to about 120 hours, from about 120 hours to about 7 days, from about 7 days to about 1 month, from about 1 month to about 3 months, from about 3 months to about 6 months, from about 6 months to about 1 year, from about 1 year to about 3 years, from about 3 years to about 6 years, from about 6 years to about 12 years, from about 12 years to about 20 years, from about 20 years to about 30 years, and from about 30 years to about 50 years.

Clause 21. The method of any of clauses 5-20, wherein the biological sample is obtained after the subject may have sustained an injury to the head caused by physical shaking, blunt impact by an external mechanical or other force that results in a closed or open head trauma, one or more falls, explosions or blasts or other types of blunt force trauma.

Clause 22. The method of any of clauses 5-20, wherein the biological sample is obtained after the subject has ingested or been exposed to a chemical, toxin or combination of a chemical and toxin.

Clause 23. The method of clause 22, wherein the chemical or toxin is fire, mold, asbestos, a pesticide, an insecticide, an organic solvent, a paint, a glue, a gas, an organic metal, a drug of abuse or one or more combinations thereof.

Clause 24. The method of any of clauses 5-20, wherein the biological sample is obtained from a subject that suffers from an autoimmune disease, a metabolic disorder, a brain tumor, hypoxia, a virus, meningitis, hydrocephalus or combinations thereof.

Clause 25. The method of any of clauses 5-24, wherein the method is done either to confirm the occurrence of traumatic brain injury or the absence of traumatic brain injury.

Clause 26. The method of any of clauses 5-24, wherein the traumatic brain injury is mild traumatic brain injury.

Clause 27. The method of any of clauses 5, 6, 8 and 9, wherein said contacting is done simultaneously.

Clause 28. The method of any of clauses 5, 6, 8, and 9, wherein said contacting is done sequentially.

Clause 29. The method of any of clauses 5, 7, 8 and 9, wherein status is being assessed by measuring the level or amount of GFAP at a single point in time.

Clause 30. The method of any of clauses 5, 7, 8 and 9, wherein status is being assessed by measuring the level or amount of GFAP done with monitoring.

Clause 31. The method of any of clauses 5-24, wherein said method has a lower end limit of detection (LoD) of about 10 pg/mL.

Clause 32. The method of any of clauses 5-24, wherein said method has a lower end limit of detection (LoD) of about 20 pg/mL.

Clause 33. The method of any of clauses 5-24, wherein said method provides an expanded window of detection.

Clause 34. The method of any of clauses 5-24, wherein said method can be carried out on any subject without regard to the subject's clinical condition, laboratory values, clinical condition and laboratory values, classification as suffering from mild, moderate or severe TBI, exhibition of low or high levels of GFAP, and/or without regard to the timing of any event wherein a subject may have sustained an injury to the head.

Clause 35. The method of any of clauses 1-34, wherein the method is performed using a point-of-care device.

Clause 36. A method of assessing a subject's glial fibrillary acid protein (GFAP) status as a measure of traumatic brain injury wherein said subject may have sustained an injury to the head, the method comprising the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein either the first or second specific binding member comprises a detectable label; and b) assessing a signal from the one or more first complexes, wherein the amount of detectable signal from the detectable label indicates the amount of GFAP present in the sample, such that the amount of detectable signal from the detectable label can be employed to assess said subject's GFAP status as a measure of traumatic brain injury, wherein the method (i) can be used to determine levels of up to 50,000 pg/mL of GFAP, (ii) does not require dilution of the biological sample, and (iii) is conducted using a point-of-care device.

Clause 37. A method of measuring GFAP in a biological sample from a subject that may have sustained an injury to the head, the method comprising (a) obtaining a biological sample from said subject, (b) contacting the biological sample with, either simultaneously or sequentially, in any order: (1) a capture antibody, which binds to an epitope on GFAP or GFAP fragment to form a capture antibody-GFAP antigen complex, and (2) a detection antibody which includes a detectable label and binds to an epitope on GFAP that is not bound by the capture antibody, to form a GFAP antigen-detection antibody complex, such that a capture antibody-GFAP antigen-detection antibody complex is formed, and (c) determining the amount or concentration of GFAP in the biological sample based on the signal generated by the detectable label in the capture antibody-GFAP antigen-detection antibody complex, wherein the method can be used to determine levels of GFAP in an amount of less than or equal to 50,000 pg/mL, and wherein said method has a dynamic range of 5 log, and is linear over said dynamic range.

Clause 38. A method of assessing a subject's glial fibrillary acid protein (GFAP) status as a measure of traumatic brain injury wherein said subject may have sustained an injury to the head, the method comprising the step of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

Clause 39. A method of assessing glial fibrillary acid protein (GFAP) status as a measure of traumatic brain injury in a subject that may have sustained an injury to the head, the method comprising the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; and b) assessing a signal from the one or more first complexes, wherein the presence of a detectable signal from the detectable label indicates that GFAP is present in the sample, and the presence of detectable signal from the detectable label can be employed to assess said subject's GFAP status as a measure of traumatic brain injury, wherein the method can be used to determine levels of GFAP in an amount of less than or equal to 50,000 pg/mL, and wherein said method has a dynamic range of 5 log, and is linear over said dynamic range.

Clause 40. A method of measuring glial fibrillary acid protein (GFAP) status as a measure of traumatic brain injury in a subject that may have sustained an injury to the head, the method comprising the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, such that the amount of detectable signal from the detectable label can be employed to assess said subject's GFAP status as a measure of traumatic brain injury, wherein said assay is capable of determining an amount of GFAP less than or equal to 50,000 pg/mL in a volume of less than 20 microliters of test sample, wherein said assay has a dynamic range of 5 log, and is linear over said dynamic range.

Clause 41. A method of assessing a subject's glial fibrillary acid protein (GFAP) status as a measure of traumatic brain injury in a biological sample obtained from a human subject, wherein said subject may have sustained an injury to the head, the method comprising the steps of: (a) contacting a biological sample obtained from a human subject, either simultaneously or sequentially, in any order, with: (1) a capture antibody which is immobilized on a solid support and which binds to an epitope on human GFAP to form a capture antibody-GFAP antigen complex, and (2) a detection antibody which includes a detectable label and which binds to an epitope on human GFAP that is not bound by the capture antibody, to form a GFAP antigen-detection antibody complex, such that a capture antibody-GFAP antigen-detection antibody complex is formed, wherein the capture antibody and detection antibody are monospecific antibodies, and optionally are monoclonal antibodies, (b) detecting a signal generated by the detectable label in the capture antibody-GFAP antigen-detection antibody complex, wherein the presence of a detectable signal from the detectable label indicates that GFAP is present in the sample, and (c) measuring the amount of detectable signal from the detectable label indicates the amount of GFAP present in the sample, such that the amount of detectable signal from the detectable label can be employed to assess said subject's GFAP status as a measure of traumatic brain injury, wherein the method is capable of quantitating the level of GFAP across a dynamic range from about 5 pg/mL to about 50,000 pg/mL with a precision of ≤10 % CV and with less than 10% deviation from linearity (DL) is achieved over the dynamic range.

Clause 42. A method of measuring glial fibrillary acid protein (GFAP) status as a measure of traumatic brain injury in a subject that may have sustained an injury to the head, the method comprising the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label, wherein the first specific binding member is immobilized on a solid support; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, such that the amount of detectable signal from the detectable label can be employed to assess said subject's GFAP status as a measure of traumatic brain injury, wherein said assay is capable of determining the level of GFAP across a dynamic range from about 20 pg/mL to about 50,000 pg/mL with a precision of ≤10 % CV and with less than 10% deviation from linearity (DL) is achieved over the dynamic range in a volume of less than 20 microliters of test sample.

Clause 43. A method of assessing a subject's glial fibrillary acid protein (GFAP) status, the method comprising the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein either the first or second specific binding member comprises a detectable label; and b) assessing a signal from the one or more first complexes, wherein the amount of detectable signal from the detectable label indicates the amount of GFAP present in the sample, wherein the method (i) can be used to determine levels of up to 50,000 pg/mL of GFAP, (ii) does not require dilution of the biological sample, and (iii) is conducted using a point-of-care device.

Clause 44. A method of measuring GFAP in a biological sample from a subject, the method comprising (a) obtaining a biological sample from said subject, (b) contacting the biological sample with, either simultaneously or sequentially, in any order: (1) a capture antibody, which binds to an epitope on GFAP or GFAP fragment to form a capture antibody-GFAP antigen complex, and (2) a detection antibody which includes a detectable label and binds to an epitope on GFAP that is not bound by the capture antibody, to form a GFAP antigen-detection antibody complex, such that a capture antibody-GFAP antigen-detection antibody complex is formed, and (c) determining the amount or concentration of GFAP in the biological sample based on the signal generated by the detectable label in the capture antibody-GFAP antigen-detection antibody complex, wherein the method can be used to determine levels of GFAP in an amount of less than or equal to 50,000 pg/mL, and wherein said method has a dynamic range of 5 log, and is linear over said dynamic range.

Clause 45. A method of assessing a subject's glial fibrillary acid protein (GFAP) status, the method comprising the step of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

Clause 46. A method of assessing glial fibrillary acid protein (GFAP) status, the method comprising the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; and b) assessing a signal from the one or more first complexes, wherein the presence of a detectable signal from the detectable label indicates that GFAP is present in the sample, wherein the method can be used to determine levels of GFAP in an amount of less than or equal to 50,000 pg/mL, and wherein said method has a dynamic range of 5 log, and is linear over said dynamic range.

Clause 47. A method of measuring glial fibrillary acid protein (GFAP) status, the method comprising the steps of: a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, wherein said assay is capable of determining an amount of GFAP less than or equal to 50,000 pg/mL in a volume of less than 20 microliters of test sample, wherein said assay has a dynamic range of 5 log, and is linear over said dynamic range.

Clause 48. The method of any of clauses 43 and 46 and 47, wherein said method is done as a measure of traumatic brain injury wherein said subject may have sustained an injury to the head, such that the amount of detectable signal from the detectable label can be employed to assess said subject's GFAP status as a measure of traumatic brain injury.

Clause 49. The method of 48, wherein said method is done such that the amount of detectable signal from the detectable label can be employed to assess said subject's GFAP status as a measure of traumatic brain injury.

Clause 50. The method of clause 48 or 49, wherein said method is done as a measure of traumatic brain injury wherein said subject may have sustained an injury to the head.

Clause 51. The method of any of clauses 43 to 50, wherein said wherein said method is done using a volume of less than 20 microliters of said biological sample.

Clause 52. The method of any of clauses 43 to 51, wherein the method can be used to determine levels of GFAP selected from the group consisting of from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL.

Clause 53. The method of any of clauses 43, 46 or 47, wherein either the first specific binding member or second specific binder member, whichever does not comprise the detectable label, is immobilized on a solid support.

Clause 54. The method of any of clauses 43-53, wherein GFAP is assessed along with one or more other biomarker.

Clause 55. The method of any of clauses 44-54, wherein the biological sample does not require dilution.

Clause 56. The method of any of clauses 43-55, wherein the biological sample is selected from the group consisting of a whole blood sample, a serum sample, a cerebrospinal fluid sample and a plasma sample.

Clause 57. The method of any of clauses 43-56, wherein the method is performed in from about 5 to about 20 minutes.

Clause 58. The method of any of clauses 43-57, wherein the method is performed in about 435 minutes.

Clause 59. The method of any of clauses 43-58, wherein the biological sample is from about 43 to about 25 microliters.

Clause 60. The method of any of clauses 43-59, wherein the time between when the biological sample is obtained and when the subject may have sustained an injury to the head is not known.

Clause 61. The method of any of clauses 43-60, wherein the time between when the biological sample is obtained and when the subject may have sustained an injury to the head is selected from the group consisting of from zero to about 12 hours, from about 12 to about 24 hours, from about 24 to about 36 hours, from about 36 to about 48 hours, from about 48 to about 72 hours, from about 72 to about 96 hours, from about 96 to about 120 hours, from about 120 hours to about 7 days, from about 7 days to about 1 month, from about 1 month to about 3 months, from about 3 months to about 6 months, from about 6 months to about 1 year, from about 1 year to about 3 years, from about 3 years to about 6 years, from about 6 years to about 12 years, from about 12 years to about 20 years, from about 20 years to about 30 years, and from about 30 years to about 50 years.

Clause 62. The method of any of clauses 43-61, wherein the biological sample is obtained after the subject may have sustained an injury to the head caused by physical shaking, blunt impact by an external mechanical or other force that results in a closed or open head trauma, one or more falls, explosions or blasts or other types of blunt force trauma.

Clause 63. The method of any of clauses 43-61, wherein the biological sample is obtained after the subject has ingested or been exposed to a chemical, toxin or combination of a chemical and toxin.

Clause 64. The method of clause 63, wherein the chemical or toxin is fire, mold, asbestos, a pesticide, an insecticide, an organic solvent, a paint, a glue, a gas, an organic metal, a drug of abuse or one or more combinations thereof.

Clause 65. The method of any of clauses 43-61, wherein the biological sample is obtained from a subject that suffers from an autoimmune disease, a metabolic disorder, a brain tumor, hypoxia, a virus, meningitis, hydrocephalus or combinations thereof.

Clause 66. The method of any of clauses 43-65, wherein the method is done either to confirm the occurrence of traumatic brain injury or the absence of traumatic brain injury.

Clause 67. The method of any of clauses 43-65, wherein the traumatic brain injury is mild traumatic brain injury.

Clause 68. The method of any of clauses 43, 44, 46 and 47, wherein said contacting is done simultaneously.

Clause 69. The method of any of clauses 43, 44, 46 and 47, wherein said contacting is done sequentially.

Clause 70. The method of any of clauses 43, 45, 46 and 47, wherein status is being assessed by measuring the level or amount of GFAP at a single point in time.

Clause 71. The method of any of clauses 43, 45, 46 and 47, wherein status is being assessed by measuring the level or amount of GFAP done with monitoring.

Clause 72. The method of any of clauses 43-71, wherein said method has a lower end limit of detection (LoD) of about 10 pg/mL.

Clause 73. The method of any of clauses 43-71, wherein said method has a lower end limit of detection (LoD) of about 20 pg/mL.

Clause 74. The method of any of clauses 43-71, wherein said method provides an expanded window of detection.

Clause 75. The method of any of clauses 43-74, wherein said method can be carried out on any subject without regard to factors selected from the group consisting of the subject's clinical condition, the subject's laboratory values, the subject's classification as suffering from mild, moderate or severe TBI, the subject's exhibition of low or high levels of GFAP, and the timing of any event wherein said subject may have sustained an injury to the head.

Clause 76. The method of any one of clauses 43-75, wherein the method is performed using a point-of-care device.

Clause 77. A method of assessing a subject's glial fibrillary acid protein (GFAP) status in a biological sample obtained from a human subject, the method comprising the steps of:

(a) contacting a biological sample obtained from a human subject, either simultaneously or sequentially, in any order, with:

(1) a capture antibody which is immobilized on a solid support and which binds to an epitope on human GFAP to form a capture antibody-GFAP antigen complex, and

(2) a detection antibody which includes a detectable label and which binds to an epitope on human GFAP that is not bound by the capture antibody, to form a GFAP antigen-detection antibody complex,

such that a capture antibody-GFAP antigen-detection antibody complex is formed,
wherein the capture antibody and detection antibody are monospecific antibodies, and optionally are monoclonal antibodies,

(b) detecting a signal generated by the detectable label in the capture antibody-GFAP antigen-detection antibody complex, wherein the presence of a detectable signal from the detectable label indicates that GFAP is present in the sample, and

(c) measuring the amount of detectable signal from the detectable label indicates the amount of GFAP present in the sample,

wherein the method is capable of quantitating the level of GFAP across a dynamic range from about 5 pg/mL to about 50,000 pg/mL with a precision of ≤10 % CV and with less than 10% deviation from linearity (DL) is achieved over the dynamic range.

Clause 78. The method of clause 77, wherein said method is done using a volume of less than 20 microliters of said biological sample.

Clause 79. A method of measuring glial fibrillary acid protein (GFAP) status, the method comprising the steps of:

a) contacting a biological sample from said subject, either simultaneously or sequentially, in any order, with a first specific binding member and a second specific binding member, wherein the first specific binding member and the second specific binding member each specifically bind to GFAP thereby producing one or more first complexes comprising first binding member-GFAP-second binding member, wherein the second specific binding member comprises a detectable label, wherein the first specific binding member is immobilized on a solid support;

b) detecting a signal from the one or more first complexes, wherein the presence of a detectable signal from the detectable label indicates that GFAP is present in the sample, and

c) measuring the amount of detectable signal from the detectable label indicates the amount of GFAP present in the sample,

wherein said assay is capable of determining the level of GFAP across a dynamic range from about 20 pg/mL to about 50,000 pg/mL with a precision of ≤10 % CV and with less than 10% deviation from linearity (DL) is achieved over the dynamic range in a volume of less than 20 microliters of test sample.

Clause 80. The method of clause 77 or 79, wherein said method is done to assess a subject's glial fibrillary acid protein (GFAP) status as a measure of traumatic brain injury, wherein said subject may have sustained an injury to the head and the amount of detectable signal from the detectable label measured is step (c) can be employed to assess said subject's GFAP status as a measure of traumatic brain injury

63

Clause 81. The method of any of clauses 77-80, wherein the method can be used to determine levels of GFAP selected from the group consisting of from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL.

Clause 82. The method of any of clauses 77-81, wherein GFAP is assessed along with one or more other biomarker.

Clause 83. The method of any of clauses 77-82, wherein the biological sample does not require dilution.

Clause 84. The method of any of clauses 77-83, wherein the biological sample is selected from the group consisting of a whole blood sample, a serum sample, a cerebrospinal fluid sample and a plasma sample.

Clause 85. The method of any of clauses 77-84, wherein the method is performed in from about 5 to about 20 minutes.

Clause 86. The method of any of clauses 77-85, wherein the method is performed in about 10 minutes.

Clause 87. The method of any of clauses 77-86, wherein the time between when the biological sample is obtained and when the subject may have sustained an injury to the head is not known.

Clause 88. The method of any of clauses 77-87, wherein the time between when the biological sample is obtained and when the subject may have sustained an injury to the head is selected from the group consisting of from zero to about 12 hours, from about 12 to about 24 hours, from about 24 to about 36 hours, from about 36 to about 48 hours, from about 48 to about 72 hours, from about 72 to about 96 hours, from about 96 to about 120 hours, from about 120 hours to about 7 days, from about 7 days to about 1 month, from about 1 month to about 3 months, from about 3 months to about 6 months, from about 6 months to about 1 year, from about 1 year to about 3 years, from about 3 years to about 6 years, from about 6 years to about 12 years, from about 12 years to about 20 years, from about 20 years to about 30 years, and from about 30 years to about 50 years.

Clause 89. The method of any of clauses 77-88, wherein the biological sample is obtained after the subject may have sustained an injury to the head caused by physical shaking, blunt impact by an external mechanical or other force that results in a closed or open head trauma, one or more falls, explosions or blasts or other types of blunt force trauma.

Clause 90. The method of any of clauses 77-88, wherein the biological sample is obtained after the subject has ingested or been exposed to a chemical, toxin or combination of a chemical and toxin.

Clause 91. The method of clause 90, wherein the chemical or toxin is fire, mold, asbestos, a pesticide, an insecticide, an organic solvent, a paint, a glue, a gas, an organic metal, a drug of abuse or one or more combinations thereof.

Clause 92. The method of any of clauses 77-88, wherein the biological sample is obtained from a subject that suffers from an autoimmune disease, a metabolic disorder, a brain tumor, hypoxia, a virus, meningitis, hydrocephalus or combinations thereof.

Clause 93. The method of any of clauses 77-92, wherein the method is done either to confirm the occurrence of traumatic brain injury or the absence of traumatic brain injury.

Clause 94. The method of any of clauses 77-92, wherein the traumatic brain injury is mild traumatic brain injury.

Clause 95. The method of any of clauses 77-94, wherein said contacting is done simultaneously.

Clause 96. The method of any of clauses 77-94, wherein said contacting is done sequentially.

Clause 97. The method of any of clauses 77-96, wherein status is being assessed by measuring the level or amount of GFAP at a single point in time.

Clause 98. The method of any of clauses 77-97, wherein status is being assessed by measuring the level or amount of GFAP done with monitoring.

Clause 99. The method of any of clauses 77-98, wherein said method has a lower end limit of detection (LoD) of about 10 pg/mL.

Clause 100. The method of any of clauses 77-98, wherein said method has a lower end limit of detection (LoD) of about 20 pg/mL.

Clause 101. The method of any of clauses 77-100, wherein said method provides an expanded window of detection.

Clause 102. The method of any of clauses 77-101, wherein said method can be carried out on any subject without regard to factors selected from the group consisting of the subject's clinical condition, the subject's laboratory values, the subject's classification as suffering from mild, moderate or severe TBI, the subject's exhibition of low or high levels of GFAP, and the timing of any event wherein said subject may have sustained an injury to the head.

Clause 103. The method of any one of clauses 77-102, wherein the method is performed using a point-of-care device.

Clause 104. A method of assessing a subject's glial fibrillary acid protein (GFAP) status, the method comprising the steps of: a) contacting a biological sample from said subject, either simultaneously or sequentially, in any order, with at least one first specific binding member and at least one second specific binding member, wherein the first specific binding member and the second specific binding member each specifically bind to GFAP thereby producing one or more first complexes comprising the at least one first specific binding member-GFAP-at least one second specific binding member, wherein either at least one of the first specific binding member or the at least one second specific binding member comprise a detectable label; and b) assessing a signal from the one or more first complexes, wherein the amount of detectable signal from the detectable label indicate the amount of GFAP present in the sample, wherein the method (i) can be used to determine levels of up to 50,000 pg/mL of GFAP, (ii) does not require dilution of the biological sample, and (iii) is conducted using a point-of-care device.

Clause 105. A method of measuring GFAP in a biological sample from a subject, the method comprising: (a) obtaining a biological sample from said subject, (b) contacting the biological sample with, either simultaneously or sequentially, in any order: (1) at least one capture antibody, which binds to an epitope on GFAP or GFAP fragment to form at least one capture antibody-GFAP antigen complex, and (2) at least one detection antibody which includes a detectable label and binds to an epitope on GFAP that is not bound by the at least one capture antibody, to form an at least one capture GFAP antigen-at least one detection antibody complex, and (c) determining the amount or concentration of GFAP in the biological sample based on the signal generated by the detectable label in the at least one capture antibody-GFAP antigen-at least one detection antibody complex, wherein the method can be used to determine levels of GFAP in an amount of less than or equal to 50,000 pg/mL, and wherein said method has a dynamic range of 5 log, and is linear over said dynamic range.

Clause 106. A method of assessing a subject's glial fibrillary acid protein (GFAP) status, the method comprising the step of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

Clause 107. A method of assessing glial fibrillary acid protein (GFAP) status in a subject, the method comprising the steps of: a) contacting a biological sample from said subject, either simultaneously or sequentially, in any order, with at least one first specific binding member and at least one second specific binding member, wherein the at least one first specific binding member and the at least one second specific binding member each specifically bind to GFAP thereby producing one or more first complexes comprising at least one first specific binding member-GFAP-at least one second specific binding member, wherein the at least one second specific binding member comprises a detectable label; and b) assessing a signal from the one or more first complexes, wherein the presence of a detectable signal from the detectable label indicates that GFAP is present in the sample, wherein the method can be used to determine levels of GFAP in an amount of less than or equal to 50,000 pg/mL, and wherein said method has a dynamic range of 5 log, and is linear over said dynamic range.

Clause 108. A method of measuring glial fibrillary acid protein (GFAP) status, the method comprising the steps of: a) contacting a biological sample from said subject, either simultaneously or sequentially, in any order, with at least one first specific binding member and at least one second specific binding member, wherein the at least one first specific binding member and the at least one second specific binding member each specifically bind to GFAP thereby producing one or more first complexes comprising at least one first specific binding member-GFAP- at least one second specific binding member, wherein the at least one second specific binding member comprises a detectable label; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, such that the amount of detectable signal from the detectable label can be employed to assess said subject's GFAP status, wherein said assay is capable of determining an amount of GFAP less than or equal to 50,000 pg/mL in a volume of less than 20 microliters of test sample, wherein said assay has a dynamic range of 5 log, and is linear over said dynamic range.

Clause 109. The method of any of clauses 104 and 107 and 108, wherein said method is done as a measure of traumatic brain injury wherein said subject may have sustained an injury to the head, such that the amount of detectable signal from the detectable label can be employed to assess said subject's GFAP status as a measure of traumatic brain injury.

Clause 110. The method of clauses 106 and 109, wherein said method is done such that the amount of detectable signal from the detectable label can be employed to assess said subject's GFAP status as a measure of traumatic brain injury.

Clause 111. The method of clause 109 or 110, wherein said method is done as a measure of traumatic brain injury wherein said subject may have sustained an injury to the head.

Clause 112. The method of any of clauses 104-111, wherein said wherein said method is done using a volume of less than 20 microliters of said biological sample.

Clause 113. The method of any of clauses 104-112, wherein the method can be used to determine levels of GFAP selected from the group consisting of from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL.

Clause 114. The method of any of clauses 104, 106 or 107, wherein either the at least one first specific binding member or at least one second specific binder member, whichever does not comprise the detectable label, is immobilized on a solid support.

Clause 115. The method of any of clauses 104-114, wherein GFAP is assessed along with one or more other biomarker.

Clause 116. The method of any of clauses 105-115, wherein the biological sample does not require dilution.

Clause 117. The method of any of clauses 104-116, wherein the biological sample is selected from the group consisting of a whole blood sample, a serum sample, a cerebrospinal fluid sample and a plasma sample.

Clause 118. The method of any of clauses 104-117, wherein the method is performed in from about 5 to about 20 minutes.

Clause 119. The method of any of clauses 104-118, wherein the method is performed in about 15 minutes.

Clause 120. The method of any of clauses 104-119, wherein the biological sample is from about 1 to about 25 microliters.

Clause 121. The method of any of clauses 104-120, wherein said method has a lower end limit of detection (LoD) of about 10 pg/mL.

Clause 122. The method of any of clauses 104-120, wherein said method has a lower end limit of detection (LoD) of about 20 pg/mL.

Clause 123. The method of any of clauses 104-122, wherein said method provides an expanded window of detection.

Clause 124. The method of any of clauses 104-123, wherein said method can be carried out on any subject without regard to factors selected from the group consisting of the subject's clinical condition, the subject's laboratory values, the subject's classification as suffering from mild, moderate or severe TBI, the subject's exhibition of low or high levels of GFAP, and the timing of any event wherein said subject may have sustained an injury to the head.

Clause 125. The method of any of clauses 105-124, wherein the method is performed using a point-of-care device.

Clause 126. The method of any of clauses 104-125, wherein the method is done either to confirm the occurrence of traumatic brain injury or the absence of traumatic brain injury.

Clause 127. The method of any of clauses 104-125, wherein the traumatic brain injury is mild traumatic brain injury.

Clause 128. The method of any of clauses 104, 105, 107 and 108, wherein said contacting is done simultaneously.

Clause 129. The method of any of clauses 104, 105, 107 and 108, wherein said contacting is done sequentially.

Clause 130. The method of any of clauses 104, 106, 107 and 108, wherein status is being assessed by measuring the level or amount of GFAP at a single point in time.

Clause 131. The method of any of clauses 104, 106, 107 and 108, wherein status is being assessed by measuring the level or amount of GFAP done with monitoring.

Clause 132. The method of any of clauses 104-131, wherein said method has a lower end limit of detection (LoD) of about 10 pg/mL.

Clause 133. The method of any of clauses 104-132, wherein said method has a lower end limit of detection (LoD) of about 20 pg/mL.

Clause 134. The method of any of clauses 104-133, wherein said method provides an expanded window of detection.

Clause 135. The method of any of clauses 104-134, wherein said method can be carried out on any subject without regard to factors selected from the group consisting of the subject's clinical condition, the subject's laboratory values, the subject's classification as suffering from mild, moderate or severe TBI, the subject's exhibition of low or high levels of GFAP, and the timing of any event wherein said subject may have sustained an injury to the head.

Clause 136. The method of any one of clauses 104-135, wherein the method is performed using a point-of-care device.

Clause 137. A method of assessing a subject's glial fibrillary acid protein (GFAP) status, the method comprising the steps of: (a) contacting a biological sample obtained from a human subject, either simultaneously or sequentially, in any order, with: (1) at least one capture antibody which is immobilized on a solid support and which binds to an epitope on human GFAP to form at least one capture antibody-GFAP antigen complex, and (2) at least one detection antibody which includes a detectable label and which binds to an epitope on human GFAP that is not bound by the capture antibody, to form at least one capture antibody-GFAP antigen-at least one detection antibody complex, wherein the at least one capture antibody and at least one detection antibody are monospecific antibodies, and optionally, are monoclonal antibodies, (b) detecting a signal generated by the detectable label in the at least one capture antibody-GFAP antigen-at least one detection antibody complex, wherein the presence of a detectable signal from the detectable label indicate that GFAP is present in the sample, and (c) measuring the amount of detectable signal from the detectable label indicates the amount of GFAP present in the sample, wherein the method is capable of quantitating the level of GFAP across a dynamic range from about 5 pg/mL to about 50,000 pg/mL with a precision of less than 10 % CV and with less than 10% deviation from linearity (DL) is achieved over the dynamic range.

Clause 138. The method of clause 137, wherein said method is done using a volume of less than 20 microliters of said biological sample.

Clause 139. A method of measuring glial fibrillary acid protein (GFAP) status, the method comprising the steps of: a) contacting a biological sample from said subject, either simultaneously or sequentially, in any order, with at least one first specific binding member and at least one second specific binding member, wherein the at least one first specific binding member and the at least one second specific binding member each specifically bind to GFAP thereby producing one or more first complexes comprising the at least one first specific binding member-GFAP-at least one second specific binding member, wherein the at least one second specific binding member comprises a detectable label, wherein the at least one first specific binding member is immobilized on a solid support; b) detecting a signal from the one or more first complexes, wherein the presence of a detectable signal from the detectable label indicates that GFAP is present in the sample, and c) measuring the amount of detectable signal from the detectable label indicates the amount of GFAP present in the sample, wherein said assay is capable of determining the level of GFAP across a dynamic range from about 20 pg/mL to about 50,000 pg/mL with a precision of less than 10 % CV and with less than 10% deviation from linearity (DL) is achieved over the dynamic range in a volume of less than 20 microliters of test sample.

Clause 140. The method of any one clauses 137-139, wherein said method is done to assess a subject's GFAP status as a measure of traumatic brain injury, wherein said subject may have sustained an injury to the head and the amount of detectable signal from the detectable label measured is step (c) can be employed to assess said subject's GFAP status as a measure of traumatic brain injury

Clause 141. The method of any of clauses 137-140, wherein GFAP is assessed along with one or more other biomarker.

Clause 142. The method of any of clauses 137-141, wherein the biological sample does not require dilution.

Clause 143. The method of any of clauses 137-142, wherein the biological sample is selected from the group consisting of a whole blood sample, a serum sample, a cerebrospinal fluid sample and a plasma sample.

Clause 144. The method of any of clauses 137-143, wherein the method is performed in from about 5 to about 20 minutes.

Clause 145. The method of any of clauses 137-144, wherein the method is performed in about 10 minutes.

Clause 146. The method of any of clauses 137-145, wherein the method is done either to confirm the occurrence of traumatic brain injury or the absence of traumatic brain injury.

Clause 147. The method of any of clauses 137-146, wherein said contacting is done simultaneously.

Clause 148. The method of any of clauses 137-146, wherein said contacting is done sequentially.

Clause 149. The method of any of clauses 137-148, wherein said method has a lower end limit of detection (LoD) of about 10 pg/mL.

Clause 150. The method of any of clauses 137-148, wherein said method has a lower end limit of detection (LoD) of about 20 pg/mL.

Clause 151. The method of any of clauses 137-150, wherein said method provides an expanded window of detection.

Clause 152. The method of any of clauses 137-151, wherein said method can be carried out on any subject without regard to factors selected from the group consisting of the subject's clinical condition, the subject's laboratory values, the subject's classification as suffering from mild, moderate or severe TBI, the subject's exhibition of low or high levels of GFAP, and the timing of any event wherein said subject may have sustained an injury to the head.

Clause 153. The method of any one of clauses 137-152, wherein the method is performed using a point-of-care device.

Clause 154. A method of assessing a subject's glial fibrillary acid protein (GFAP) status, the method comprising the step of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

Clause 155. A method of assessing a subject's glial fibrillary acid protein (GFAP) status, the method comprising the steps of: a) contacting a biological sample from said subject, either simultaneously or sequentially, in any order, with at least one first specific binding member and at least one second specific binding member, wherein the first specific binding member and the second specific binding member each specifically bind to GFAP thereby producing one or more first complexes comprising the first specific binding member-GFAP-second specific binding member; and b) detecting GFAP in the one or more first complexes present in the sample, wherein the method: (i) can be used to determine levels less than or equal to 50,000 pg/mL of GFAP and does not require dilution of the biological sample; or (ii) can be used to determine levels of GFAP in an amount of less than or equal to 50,000 pg/mL, and wherein said method has a dynamic range of 5 log, and is linear over said dynamic range, or (iii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

Clause 156. A method of assessing a subject's glial fibrillary acid protein (GFAP) status, the method comprising the steps of: a) contacting a biological sample from said subject, either simultaneously or sequentially, in any order, with at least one first specific binding member and at least one second specific binding member, wherein the first specific binding member and the second specific binding member each specifically bind to GFAP thereby producing one or more first complexes comprising the first specific binding member-GFAP-second specific binding member, wherein either the first specific binding member or second specific binding member, comprise a detectable label; and b) assessing a signal from the one or more first complexes, wherein the amount of detectable signal from the detectable label indicates the amount of GFAP present in the sample, wherein the method: (i) can be used to determine levels of up to 50,000 pg/mL of GFAP and does not require dilution of the biological sample; or (ii) can be used to determine levels of GFAP in an amount of less than or equal to 50,000 pg/mL, and wherein said method has a dynamic range of 5 log, and is linear over said dynamic range, or (iii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

Clause 157. A method of measuring GFAP in a biological sample from a subject, the method comprising (a) obtaining a biological sample from said subject; (b) contacting the biological sample with, either simultaneously or sequentially, in any order: (1) at least one capture antibody, which binds to an epitope on GFAP or GFAP fragment to form a capture antibody-GFAP antigen complex, and (2) at least one first detection antibody which includes a detectable label and binds to an epitope on GFAP that is not bound by the capture antibody, to form at least one capture antibody-GFAP antigen-at least one first detection antibody-complex, and (c) determining the amount or concentration of GFAP in the biological sample based on the signal generated by the detectable label in the at least one capture antibody-GFAP antigen-at least one first detection antibody complex, wherein the method: (i)can be used to determine levels of GFAP in an amount of less than or equal to 50,000 pg/mL, and wherein said method has a dynamic range of 5 log, and is linear over said dynamic range; or (ii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

Clause 158. The method of clause 154 or 155, wherein the GFAP is detected by an immunoassay or a single molecule detection assay.

Clause 159. The method of any one of clauses 154-158, wherein the method can be used to determine levels of GFAP selected from the group consisting of from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL.

Clause 160. The method of clause 155 or 156, wherein either the first specific binding member and second specific binding member whichever does not comprise the detectable label, is immobilized on a solid support.

Clause 161. The method of any one of clauses 154-160, wherein the method is performed using a point-of-care device.

Clause 162. The method of any one of clauses 154-161, wherein GFAP is assessed along with one or more other biomarkers.

Clause 163. The method of any one of clauses 154-162, wherein the method detects levels of GFAP selected from the group consisting of from about 10 pg/mL to about 50,000 pg/mL, from about 35 pg/mL to about 50,000 pg/mL, from about 100 pg/mL to about 50,000 pg/mL, from about 125 pg/mL to about 50,000 pg/mL, from about 150 pg/mL to about 15,000 pg/mL and from about 175 pg/mL to about 10,000 pg/mL.

Clause 164. The method of any one of clauses 155-163, wherein said contacting is done simultaneously.

Clause 165. The method of any one of clauses 155-163, wherein said contacting is done sequentially.

Clause 166. The method of any one of clauses 157-165, wherein the at least one capture antibody is immobilized on a solid support.

Clause 167. The method of any one of clauses 154-166, wherein the method is performed in from about 5 to about 20 minutes.

Clause 168. The method of any one of clauses 154-167, wherein the method is performed in about 15 minutes.

Clause 169. The method of any one of clauses 154-168, wherein the biological sample is selected from the group consisting of a whole blood sample, a serum sample, a cerebrospinal fluid sample and a plasma sample.

Clause 170. The method of any one of clauses 154-169, wherein the method is done either to confirm the occurrence of traumatic brain injury or the absence of traumatic brain injury.

Clause 171. The method of clause 170, wherein the traumatic brain injury is mild traumatic brain injury.

Clause 172. The method of any one of clauses 154, 155, 156, 158-165, or 167-171, wherein status is being assessed by measuring the level or amount of GFAP at a single point in time.

Clause 173. The method of any one of clauses 154, 155, 156, 158-165, or 167-171, wherein status is being assessed by measuring the level, or amount of GFAP done with monitoring.

Clause 174. The method of any one of clauses 154-173, wherein said method can be carried out on any subject without regard to factors selected from the group consisting of the subject's clinical condition, the subject's laboratory values, the subject's classification as suffering from mild, moderate or severe TBI, the subject's exhibition of low or high levels of GFAP, and the timing of any event wherein said subject may have sustained an injury to the head.

Clause 175. The method of any one of clauses 154-174, wherein said wherein said method is done using a volume of less than 20 microliters of said biological sample.

Clause 176. The method of any one of clauses 154, 157-159, or 161-175, wherein the biological sample does not require dilution.

Clause 177. The method of any one of clauses 154-176, wherein said method has a lower end limit of detection (LoD) of about 10 pg/mL.

Clause 178. The method of any one of clauses 154-176, wherein said method has a lower end limit of detection (LoD) of about 20 pg/mL.

Clause 179. The method of any one of clauses 154-178, wherein said method provides an expanded window of detection.

Clause 180. The method of any one of clauses 155, 156, or 160, wherein the one first GFAP specific binding member is immobilized on a solid support.

Clause 181. The method of any one of clauses 155, 156, or 160, wherein at least one second GFAP specific binding member is immobilized a solid support.

Clause 182. The method of any one of clauses 155, 156, or 160, wherein the at least one first GFAP specific binding member and the at least one second GFAP specific binding member are monospecific antibodies.

Clause 183. The method of claim 169, wherein the biological sample is either diluted or undiluted.

**NUMBERED EMBODIMENTS**

1. A method of assessing a subject's glial fibrillary acid protein (GFAP) status, the method comprising the step of: detecting at least one biomarker in a biological sample from said subject wherein at least one of the biomarkers is GFAP and wherein the method (i) can be used to determine levels of GFAP in an amount less than or equal to 50,000 pg/mL, (ii) has a dynamic range of 5 log, and (iii) is linear over the dynamic range.

2. A method of assessing a subject's glial fibrillary acid protein (GFAP) status, the method comprising the steps of:

a) contacting a biological sample from said subject, either simultaneously or sequentially, in any order, with at least one first specific binding member and at least one second specific binding member, wherein the first specific binding member and the second specific binding member each specifically bind to GFAP thereby producing one or more first complexes comprising the first specific binding member-GFAP-second specific binding member; and

b) detecting GFAP in the one or more first complexes present in the sample,

wherein the method:

(i) can be used to determine levels less than or equal to 50,000 pg/mL of GFAP and does not require dilution of the biological sample; or
(ii) can be used to determine levels of GFAP in an amount of less than or equal to 50,000 pg/mL, and wherein said method has a dynamic range of 5 log, and is linear over said dynamic range, or
(iii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

3. A method of assessing a subject's glial fibrillary acid protein (GFAP) status, the method comprising the steps of:

a) contacting a biological sample from said subject, either simultaneously or sequentially, in any order, with at least one first specific binding member and at least one second specific binding member, wherein the first specific binding member and the second specific binding member each specifically bind to GFAP thereby producing one or more first complexes comprising the first specific binding member-GFAP-second specific binding member, wherein either the first specific binding member or second specific binding member, comprise a detectable label; and

EP 4 729 943 A2

b) assessing a signal from the one or more first complexes, wherein the amount of detectable signal from the detectable label indicates the amount of GFAP present in the sample,

wherein the method:

(i) can be used to determine levels less than or equal to 50,000 pg/mL of GFAP and does not require dilution of the biological sample; or
(ii) can be used to determine levels of GFAP in an amount of less than or equal to 50,000 pg/mL, and wherein said method has a dynamic range of 5 log, and is linear over said dynamic range, or
(iii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

4. A method of measuring GFAP in a biological sample from a subject, the method comprising

(a) obtaining a biological sample from said subject;

(b) contacting the biological sample with, either simultaneously or sequentially, in any order:

(1) at least one capture antibody, which binds to an epitope on GFAP or GFAP fragment to form a capture antibody-GFAP antigen complex, and (2) at least one first detection antibody which includes a detectable label and binds to an epitope on GFAP that is not bound by the capture antibody, to form at least one capture antibody-GFAP antigen-at least one first detection antibody-complex, and

(c) determining the amount or concentration of GFAP in the biological sample based on the signal generated by the detectable label in the at least one capture antibody-GFAP antigen-at least one first detection antibody complex,

wherein the method:

(i) can be used to determine levels of GFAP in an amount of less than or equal to 50,000 pg/mL, and wherein said method has a dynamic range of 5 log, and is linear over said dynamic range; or
(ii) is capable of quantitating the level of GFAP across a dynamic range from about 5 pg/mL to about 50,000 pg/mL with a precision of less than 10 % CV and with less than 10% deviation from linearity (DL) is achieved over the dynamic range.

5. The method of embodiment 1 or 2, wherein the GFAP is detected by an immunoassay or a single molecule detection assay.

6. The method of any one of embodiments 1-5, wherein the method can be used to determine levels of GFAP selected from the group consisting of from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL.

7. The method of embodiment 2 or 3, wherein either the first specific binding member and second specific binding member whichever does not comprise the detectable label, is immobilized on a solid support.

8. The method of any one of embodiments 1-7, wherein the method is performed using a point-of-care device.

9. The method of any one of embodiments 1-8, wherein GFAP is assessed along with one or more other biomarkers.

10. The method of any one of embodiments 1-9, wherein the method detects levels of GFAP selected from the group consisting of from about 10 pg/mL to about 50,000 pg/mL, from about 35 pg/mL to about 50,000 pg/mL, from about 100 pg/mL to about 50,000 pg/mL, from about 125 pg/mL to about 50,000 pg/mL, from about 150 pg/mL to about 15,000 pg/mL and from about 175 pg/mL to about 10,000 pg/mL.

11. The method of any one of embodiments 2-10, wherein said contacting is done simultaneously.

12. The method of any one of embodiments 2-10, wherein said contacting is done sequentially.

13. The method of any one of embodiments 4-12, wherein the at least one capture antibody is immobilized on a solid support.

14. The method of any one of embodiments 1-13, wherein the method is performed in from about 5 to about 20 minutes.

15. The method of any one of embodiments 1-14, wherein the method is performed in about 15 minutes.

16. The method of any one of embodiments 1-15, wherein the biological sample is selected from the group consisting of a whole blood sample, a serum sample, a cerebrospinal fluid sample and a plasma sample.

17. The method of any one of embodiments 1-16, wherein the method is done either to confirm the occurrence of traumatic brain injury or the absence of traumatic brain injury.

18. The method of embodiment 17, wherein the traumatic brain injury is mild traumatic brain injury.

19. The method of any one of embodiments 1, 2, 3, 5-12, or 14-18, wherein status is being assessed by measuring the level or amount of GFAP at a single point in time.

20. The method of any one of embodiments 1, 2, 3, 5-12, or 14-18, wherein status is being assessed by measuring the level, or amount of GFAP done with monitoring.

21. The method of any one of embodiments 1-20, wherein said method can be carried out on any subject without regard to factors selected from the group consisting of the subject's clinical condition, the subject's laboratory values, the subject's classification as suffering from mild, moderate or severe TBI, the subject's exhibition of low or high levels of GFAP, and the timing of any event wherein said subject may have sustained an injury to the head.

22. The method of any one of embodiments 1-21, wherein said wherein said method is done using a volume of less than 20 microliters of said biological sample.

23. The method of any one of embodiments 1, 4-6, or 8-22, wherein the biological sample does not require dilution.

24. The method of any one of embodiments 1-23, wherein said method has a lower end limit of detection (LoD) of about 10 pg/mL.

25. The method of any one of embodiments 1-23, wherein said method has a lower end limit of detection (LoD) of about 20 pg/mL.

26. The method of any one of embodiments 1-25, wherein said method provides an expanded window of detection.

27. The method of embodiment 2, 3, or 7, wherein the one first GFAP specific binding member is immobilized on a solid support.

28. The method of embodiment 2, 3, or 7, wherein at least one second GFAP specific binding member is immobilized a solid support.

29. The method of embodiment 2, 3, or 7, wherein the at least one first GFAP specific binding member and the at least one second GFAP specific binding member are monospecific antibodies.

30. The method of embodiment 16, wherein the biological sample is either diluted or undiluted.

**Claims**

1.  A method of measuring a human glial fibrillary acid protein (GFAP) in a biological sample obtained from a subject, the method comprising the steps of:

    a) contacting the biological sample with, either simultaneously or sequentially, in any order:

    (1) at least one first specific binding member and at least one second specific binding member, wherein the first specific binding member binds to an epitope on a 38 kDa GFAP breakdown product (BDP) defined by amino acids 60-383 of SEQ ID NO: 1, and the at least one second specific binding member includes a detectable label and binds to an epitope on a 38kDa GFAP BDP defined by amino acids 60-383 of SEQ ID NO: 1 that is not bound by the first specific binding member, thereby producing one or more first complexes comprising the first specific binding member-GFAP BDP-second specific binding member; and

    b) determining the amount or concentration of the GFAP in the biological sample based on a signal generated by the detectable label of the at least one second specific binding member in the first specific binding member-GFAP BDP-second specific binding member complex,

    wherein the method: (i) determines levels of GFAP in an amount of less than or equal to 50,000 pg/mL; (ii) has a dynamic range of 5 log, and (iii) is linear over the dynamic range.

2.  A method of measuring a human glial fibrillary acid protein (GFAP) in a biological sample obtained from a subject, the method comprising the steps of:

    a) contacting the biological sample with, either simultaneously or sequentially, in any order:

    (1) at least one first specific binding member and at least one second specific binding member, wherein the first specific binding member binds to an epitope on a 38 kDa GFAP BDP defined by amino acids 60-383 of SEQ ID NO: 1, and the at least one second specific binding member includes a detectable label and binds to an epitope on a 38kDa GFAP BDP defined by amino acids 60-383 of SEQ ID NO: 1 that is not bound by the first specific binding member, thereby producing one or more first complexes comprising the first specific binding member-GFAP BDP-second specific binding member; and

    b) determining the amount or concentration of the GFAP in the biological sample based on a signal generated by the detectable label of the at least one second specific binding member in the first specific binding member-GFAP BDP-second specific binding member complex,

    wherein the method:

    (i) determines levels less than or equal to 50,000 pg/mL of GFAP and does not require dilution of the biological sample;
    (ii) determines levels less than or equal to 50,000 pg/mL of GFAP, has a dynamic range of 5 log, and is linear over said dynamic range, or
    (iii) quantitates the level of GFAP across a dynamic range from about 5 pg/mL to about 50,000 pg/mL with a precision of less than 10 % CV and with less than 10% deviation from linearity (DL) achieved over the dynamic range.

3.  The method of any one of claims 1-2, wherein the method can be used to determine levels of GFAP selected from the group consisting of from about 10 pg/mL to about 50,000 pg/mL, from about 20 pg/mL to about 50,000 pg/mL, from about 25 pg/mL to about 50,000 pg/mL, from about 30 pg/mL to about 50,000 pg/mL, from about 40 pg/mL to about 50,000 pg/mL, from about 50 pg/mL to about 50,000 pg/mL, from about 60 pg/mL to about 50,000 pg/mL, from about 70 pg/mL to about 50,000 pg/mL, from about 75 pg/mL to about 50,000 pg/mL, from about 80 pg/mL to about 50,000 pg/mL, from about 90 pg/mL to about 50,000 pg/mL, from about 100 pg/mL to about 50,000 pg/mL, from about 125 pg/mL to about 50,000 pg/mL, and from about 150 pg/mL to about 50,000 pg/mL.

4.  The method of claim 1, wherein the first specific binding member is immobilized on a solid support.

5.  The method of any one of claims 1-4, wherein

(a) the method is performed using a point-of-care device; and/or
(b) GFAP is assessed along with one or more other biomarkers; and/or
(c) wherein the method detects levels of GFAP selected from the group consisting of from about 10 pg/mL to about 50,000 pg/mL, from about 35 pg/mL to about 50,000 pg/mL, from about 100 pg/mL to about 50,000 pg/mL, from about 125 pg/mL to about 50,000 pg/mL, from about 150 pg/mL to about 15,000 pg/mL and from about 175 pg/mL to about 10,000 pg/mL.

6. The method of any one of claims 1-5, wherein said contacting is done simultaneously or wherein said contacting is done sequentially.

7. The method of any one of claims 1-6, wherein

(a) the method is performed in less than about 30 minutes;
(b) the method is performed in from about 5 to about 20 minutes; and/or
(c) the method is performed in about 15 minutes; and/or

8. The method of any one of claims 1-7, wherein the biological sample is selected from the group consisting of a whole blood sample, a serum sample, and a plasma sample, optionally wherein the biological sample is either diluted or undiluted;

9. The method of any one of claims 1-8, wherein the method is done either to confirm the occurrence of traumatic brain injury or the absence of traumatic brain injury; optionally wherein the traumatic brain injury is mild traumatic brain injury.

10. The method of any one of claims 1-9, wherein said method can be carried out on any subject without regard to factors selected from the group consisting of the subject's clinical condition, the subject's laboratory values, the subject's classification as suffering from mild, moderate or severe TBI, the subject's exhibition of low or high levels of GFAP, and the timing of any event wherein said subject may have sustained an injury to the head.

11. The method of any one of claims 1-10, wherein said method is done using a volume of less than 20 microliters of said biological sample.

12. The method of any one of claims 1-11, wherein (a) said method has a lower end limit of detection (LoD) of about 10 pg/mL or (b) has a lower end limit of detection (LoD) of about 20 pg/mL; and/or (c) said method provides an expanded window of detection.

13. The method of any one of claims 1-12, wherein the GFAP is detected by an immunoassay or a single-molecule detection assay.

14. The method of any one of claims 1-13, wherein the biological sample does not require dilution.

FIG. 1

FIG. 2

FIG. 3

FIG. 4

FIG. 5

**GFAP Linearity**

$y = 1.0287x + 1.3581$
$R^2 = 0.9978$

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62403293
- US 62455269
- US 6914128 B1
- WO 2001058956 A
- WO 0202773 A
- WO 2007024715 A
- US 7612181 B
- US 5593896 A
- US 5573904 A
- US 5496925 A
- US 5359093 A
- US 5352803 A
- US 5468646 A
- US 5543524 A
- US 5783699 A
- US 5241070 A
- US 69783507
- US 20060134713 A
- WO 2016161402 A
- WO 2016161400 A
- US 4554101 A
- US 4704692 A
- US 5723323 A
- US 5763192 A
- US 5814476 A
- US 5817483 A
- US 5824514 A
- US 5976862 A
- US 5627052 A
- US 5827690 A
- US 5849992 A
- US 4873316 A
- US 5994616 A
- US 5565362 A
- US 5304489 A
- EP 404097 A
- WO 9311161 A
- US 6632926 B, Chen
- WO 0037504 A
- WO 9202551 A
- WO 9729131 A
- WO 0056772 A
- US 5916771 A
- US 5939598 A
- US 5985615 A
- US 5998209 A
- US 6075181 A
- US 6091001 A
- US 6114598 A
- US 6130364 A
- WO 9110741 A
- WO 9402602 A
- WO 9634096 A
- WO 9633735 A
- WO 9816654 A
- WO 9824893 A
- WO 9850433 A
- WO 9945031 A
- WO 9953049 A
- WO 0009560 A
- US 5223409 A, Ladner
- WO 9218619 A, Kang
- WO 9117271 A, Dower
- WO 9220791 A, Winter
- WO 9215679 A, Markland
- WO 9301288 A, Breitling
- WO 9201047 A, McCafferty
- WO 9209690 A, Garrard
- US 20030186374
- WO 9002809 A
- WO 9110737 A
- WO 9311236 A
- WO 9515982 A
- WO 9520401 A
- US 5698426 A
- US 5403484 A
- US 5580717 A
- US 5427908 A
- US 5750753 A
- US 5821047 A
- US 5571698 A
- US 5516637 A
- US 5780225 A
- US 5658727 A
- US 5733743 A
- US 5969108 A
- WO 9222324 A
- US 4946778 A
- US 5258498 A
- WO 9831700 A, Szostak and Roberts
- US 6699658 B, Wittrup
- US 4816567 A
- US 5766886 A
- US 5714352 A
- US 6204023 B
- US 6180370 B
- US 5693762 A
- US 5530101 A

- US 5585089 A
- US 5225539 A
- US 5770429 A
- US 5833985 A
- US 5837243 A
- US 5922845 A
- US 6017517 A
- US 6096311 A
- US 6111166 A
- US 6270765 A
- US 6303755 A
- US 6365116 A
- US 6410690 A
- US 6682928 A
- US 6984720 A
- US 6143576 A
- US 6113855 A
- US 6019944 A
- US 5985579 A

- US 5947124 A
- US 5939272 A
- US 5922615 A
- US 5885527 A
- US 5851776 A
- US 5824799 A
- US 5679526 A
- US 5525524 A
- US 5480792 A
- US 5063081 A
- US 20030170881
- US 20040018577 A
- US 20050054078 A
- US 20060160164 A
- US 5089424 A
- US 5006309 A
- US 5294404 A
- US 61142048

**Non-patent literature cited in the description**

- **MARKS et al.** *BioTechnology*, 1992, vol. 10, 779-783
- **BARBAS et al.** *Proc. Nat. Acad. Sci. USA*, 1994, vol. 91, 3809-3813
- **SCHIER et al.** *Gene*, 1995, vol. 169, 147-155
- **YELTON et al.** *J. Immunol*, 1995, vol. 155, 1994-2004
- **JACKSON et al.** *J. Immunol.*, 1995, vol. 154 (7), 3310-3319
- **HAWKINS et al.** *J. Mol. Biol.*, 1992, vol. 226, 889-896
- **WU, C. et al.** *Nature Biotechnology*, 2007, vol. 25 (11), 1290-1297
- **HOLT et al.** *Trends in Biotechnology*, 2014, vol. 21, 484-490
- **MILSTEIN et al.** *Nature*, 1983, vol. 305 (5934), 537-540
- **STAERZ et al.** *Nature*, 1985, vol. 314 (6012), 628-631
- **HOLLIGER et al.** *Proc. Natl. Acad. Sci. USA*, 1993, vol. 90 (14), 6444-6448
- **KABAT et al.** Sequences of Proteins of Immunological Interest. National Institutes of Health, 1987
- **CHOTHIA ; LESK.** *J. Mol. Biol.*, 1987, vol. 196, 901-917
- **CHOTHIA et al.** *Nature*, 1989, vol. 342, 877-883
- **PADLAN.** *FASEB J.*, 1995, vol. 9, 133-139
- **MACCALLUM.** *J. Mol. Biol.*, 1996, vol. 262 (5), 732-745
- **WU et al.** *Nature Biotech.*, 2007, vol. 25, 1290-1297

- **WINNAKER.** From Genes to Clones. Verlagsgesellschaft, 1987
- **POLAK ; VAN NOORDEN.** Introduction to Immunocytochemistry,. Springer Verlag, 1997
- **HAUGLAND.** Handbook of Fluorescent Probes and Research Chemicals. Molecular Probes, Inc., 1996
- **ADAMCZYK et al.** *Bioorg. Med. Chem. Lett*, 2006, vol. 16, 1324-1328
- **ADAMCZYK et al.** *Bioorg. Med. Chem. Lett.*, 2004, vol. 4, 2313-2317
- **ADAMCZYK et al.** *Biorg. Med. Chem. Lett.*, 2004, vol. 14, 3917-3921
- **ADAMCZYK et al.** *Org. Lett.*, 2003, vol. 5, 3779-3782
- **MATTINGLY.** *J. Biolumin. Chemilumin.*, 1991, vol. 6, 107-114
- **ADAMCZYK et al.** *J. Org. Chem.*, 1998, vol. 63, 5636-5639
- **ADAMCZYK et al.** *Tetrahedron*, 1999, vol. 55, 10899-10914
- **ADAMCZYK et al.** *Org. Lett.*, 1999, vol. 1, 779-781
- **ADAMCZYK et al.** *Bioconjugate Chem.*, 2000, vol. 11, 714-724
- **MATTINGLY et al.** In Luminescence Biotechnology: Instruments and Applications. CRC Press, 2002, 77-105
- **MCCAPRA et al.** *Photochem. Photobiol.*, 1965, vol. 4, 1111-21
- **RAZAVI et al.** *Luminescence*, 2000, vol. 15, 245-249
- **RAZAVI et al.** *Luminescence*, 2000, vol. 15, 239-244

- **WELLS**. High Throughput Bioanalytical Sample Preparation. Methods and Automation Strategies. Elsevier, 2003
- Protocols for Determination of Limits of Detection and Limits of Quantitation;. Approved Guideline
- **JAMES F. PIERSON-PERRY et al.** *Clinical and Laboratory Standards Institute*, 01 June 2012
- **BIRD et al.** *Science*, 1988, vol. 242, 423-426
- **HUSTON et al.** *PNAS USA*, 1988, vol. 85, 5879-5883
- **MCCAFFERTY et al.** *Nature*, 1990, vol. 348, 552-554
- **WU, C. et al.** *Nature Biotechnology*, 2007, vol. 25, 1290-1297
- **KYTE et al.** *J. Mol. Biol.*, 1982, vol. 157, 105-132
- **FUNARO et al.** *BMC Biotechnology*, vol. 2008 (8), 85
- **URLAUB ; CHASIN.** *Proc. Natl. Acad. Sci. USA*, 1980, vol. 77, 4216-4220
- **KAUFMAN ; SHARP.** *J. Mol. Biol.*, 1982, vol. 159, 601-621
- **NGUYEN et al.** *Microbiol. Immunol*, 1997, vol. 41, 901-907
- **SANDHU et al.** *Crit. Rev. Biotechnol.*, 1996, vol. 16, 95-118
- **EREN et al.** *Immunol.*, 1998, vol. 93, 154-161
- **HANES et al.** *Proc. Natl. Acad. Sci. USA*, 1997, vol. 94, 4937-4942
- **HANES et al.** *Proc. Natl. Acad. Sci. USA*, 1998, vol. 95, 14130-14135
- **WEN et al.** *J. Immunol.*, 1987, vol. 17, 887-892
- **BABCOOK et al.** *Proc. Natl. Acad. Sci. USA*, 1996, 7843-7848
- **POWELL et al.** *Biotechnol.*, 1990, vol. 8, 333-337
- **GRAY et al.** *J. Imm. Meth.*, 1995, vol. 182, 155-163
- **KENNY et al.** *Bio/Technol.*, 1995, vol. 13, 787-790
- **STEENBAKKERS et al.** *Molec. Biol. Reports*, 1994, vol. 19, 125-134
- **YELTON et al.** *J. Immunol.*, 1995, vol. 155, 1994-2004
- **CRAMER et al.** *Curr. Top. Microbiol. Immunol.*, 1999, vol. 240, 95-118
- **HOOD et al.** *Adv. Exp. Med. Biol.*, 1999, vol. 464, 127-147
- **CONRAD et al.** *Plant Mol. Biol*, 1998, vol. 38, 101-109
- **HOLLINGER et al.** *Proc. Natl. Acad. Sci. USA*, 1993, vol. 90, 6444-6448
- **ZAPATA et al.** *Protein Eng*, 1995, vol. 8 (10), 1057-1062
- **HARLOW et al.** Antibodies: A Laboratory Manual. Cold Spring Harbor Laboratory Press, 1988
- **HAMMERLING et al.** In Monoclonal Antibodies and T-Cell Hybridomas,. Elsevier, 1981
- **BABCOOK et al.** *Proc. Natl. Acad. Sci. USA*, 1996, vol. 93, 7843-7848
- **GREEN et al.** *Nature Genetics*, 1994, vol. 7, 13-21
- **MENDEZ et al.** *Nature Genetics*, 1997, vol. 15, 146-156
- **GREEN ; JAKOBOVITS.** *J. Exp. Med.*, 1998, vol. 188, 483-495
- **FUCHS et al.** *Bio/Technology*, 1991, vol. 9, 1369-1372
- **HAY et al.** *Hum. Antibod. Hybridomas*, 1992, vol. 3, 81-85
- **HUSE et al.** *Science*, 1989, vol. 246, 1275-1281
- **GRIFFITHS et al.** *EMBO J.*, 1993, vol. 12, 725-734
- **CLACKSON et al.** *Nature*, 1991, vol. 352, 624-628
- **GRAM et al.** *Proc. Natl. Acad. Sci. USA*, 1992, vol. 89, 3576-3580
- **GARRARD et al.** *Bio/Technology*, 1991, vol. 9, 1373-1377
- **HOOGENBOOM et al.** *Nucl. Acids Res.*, 1991, vol. 19, 4133-4137
- **BARBAS et al.** *Proc. Natl. Acad. Sci. USA*, 1991, vol. 88, 7978-7982
- **BRINKMANN et al.** *J. Immunol. Methods*, 1995, vol. 182, 41-50
- **AMES et al.** *J. Immunol. Methods*, 1995, vol. 184, 177-186
- **KETTLEBOROUGH et al.** *Eur. J. Immunol.*, 1994, vol. 24, 952-958
- **PERSIC et al.** *Gene*, 1997, vol. 187, 9-18
- **BURTON et al.** *Advances in Immunology*, 1994, vol. 57, 191-280
- **MULLINAX et al.** *BioTechniques*, 1992, vol. 12 (6), 864-869
- **SAWAI et al.** *Am. J. Reprod. Immunol.*, 1995, vol. 34, 26-34
- **BETTER et al.** *Science*, 1988, vol. 240, 1041-1043
- **HUSTON et al.** *Methods in Enzymology*, 1991, vol. 203, 46-88
- **SHU et al.** *Proc. Natl. Acad. Sci. USA*, 1993, vol. 90, 7995-7999
- **SKERRA et al.** *Science*, 1988, vol. 240, 1038-1041
- **ROBERTS ; SZOSTAK.** *Proc. Natl. Acad. Sci. USA*, 1997, vol. 94, 12297-12302
- **ADAMCZYK et al.** *Anal. Chim. Acta*, 2006, vol. 579 (1), 61-67
- Evaluation of Precision Performance of Quantitative Measurement Methods;. Approved Guideline. NCCLS, 2004
- User Verification of Performance for Precision and Trueness;. Approved Guideline. Clinical and Laboratory Standards Institute, 2005

- Protocols for Determination of Limits of Detection and Limits of Quantitation;. **JAMES F. PIERSON-PERRY et al.** Approved Guideline. Clinical and Laboratory Standards Institute, 01 June 2012, 80

- Interference Testing in Clinical Chemistry;. Approved Guideline. Clinical and Laboratory Standards Institute, 2005